(19) Europäisches Patentamt
European Patent Office
Office européen des brevets

(11) Publication number: **0 068 876**
**B1**

(12) # EUROPEAN PATENT SPECIFICATION

(45) Date of publication of patent specification: **03.04.85**

(21) Application number: **82303381.6**

(22) Date of filing: **28.06.82**

(51) Int. Cl.$^4$: **C 09 B 57/00,** C 07 D 471/04
// G03C1/72, G03C5/16

(54) **Oxoindolizine and oxoindolizinium dyes and processes for their preparation.**

(30) Priority: **29.06.81 US 278022**

(43) Date of publication of application:
**05.01.83 Bulletin 83/01**

(45) Publication of the grant of the patent:
**03.04.85 Bulletin 85/14**

(84) Designated Contracting States:
**BE DE FR GB IT NL**

(58) References cited:

**None**

(73) Proprietor: **EASTMAN KODAK COMPANY**
**343 State Street**
**Rochester New York 14650 (US)**

(72) Inventor: **Fletcher, Jr., George Leland**
**786 Mile Square Road**
**Pittsford New York 14534 (US)**
Inventor: **Bender, Steven Lee**
**330 Waldo Avenue, Apt. 2**
**Pasedena California 91101 (US)**
Inventor: **Wadsworth, Donald Harols**
**210 Nob Hill**
**Rochester New York 14617 (US)**

(74) Representative: **Pepper, John Herbert et al**
**KODAK LIMITED Patent Department P.O. Box**
**114 190 High Holborn**
**London WC1V 7EA (GB)**

**EP 0 068 876 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to new oxoindolizine and oxoindolizinium dyes and to their preparation.

Dyes useful in imaging materials are well known in the photographic art. However, of the various types of dyes available or described in the prior art, no class is known which offers the combined advantages of wide absorption ranges and ease of preparation without the need for complex multistep reactions.

Attempts have been made to react cyclopropenones with heteroaromatic nitrogen compounds as described in, for example, "Reaction of Cyclopropenones With Hetero aromatic Nitrogen Compounds" by J. W. Lown and K. Matsumoto, *Canadian Journal of Chemistry*, Vol. 49, 1971, pages 1165—1175. However, such attempts did not produce oxoindolizine or oxoindolizinium dyes.

In accordance with the present invention there is provided a method of preparing an oxoindolizine or an oxoindolizinium dye compound which comprises reacting (A) a pyridine compound having hydrogen atoms on the carbon atoms ortho to the heterocyclic nitrogen atom with (B) a disubstituted cyclopropenone compound, and, if substituents in the pyridine compound are not such that the reaction product is the desired dye compound, reacting the said reaction product with a color-forming compound to give the desired dye compound.

None of the known classes of dyes involve preparation by means of a simple reaction of a cyclopropenone with a pyridine compound nor do they involve reactions of (1) color-forming couplers with (2) products derived from reaction of photosensitive cyclopropenones with pyridine compounds. This invention provides dyes which are easily synthesized and which have a variety of uses in imaging technology.

The new oxoindolizine and oxoindolizinium dyes provided by this invention are useful in laser recording and reading applications. Some of these dyes are also useful as image dyes in photothermography and thermography.

Oxoindolizine and oxoindolizinium dyes described herein include methyleneoxoindolizine, (4-oxoarylene)oxoindolizine, bis-oxoindolizine, bis(oxoindolizinyl) ethylene, (2- and 4-amino-arylene)-oxoindolizine and pyridiniumoxoindolizine dyes. These dyes may be in their keto or enol form, but are also provided in their various isomeric and tautomeric forms.

Oxoindolizine dyes according to this invention, in their keto form, have the following structure:

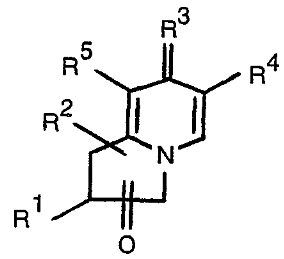

(I)

wherein

$R^1$ and $R^2$ are individually straight or branched chain alkyl containing 1 to 18, preferably 1 to 10 carbon atoms; substituted or unsubstituted aryl containing 6 to 20 carbon atoms; or polystyryl having appended indolizine or indolizinium groups, or combinations thereof;

$R^3$ is a divalent group which, with the oxoindolizinone nucleus, completes an organic chromophore;

$R^4$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine; and

$R^5$ is hydrogen; chlorine; bromine or alkyl containing 1 to 18 carbon atoms.

Oxoindolizinium dyes according to this invention, in their keto form, have the following structure:

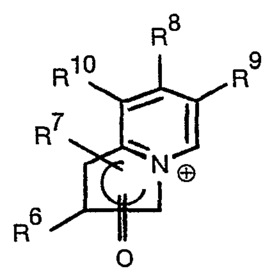

$X^{\ominus}$

(II)

wherein

$X^{\ominus}$ is an anion, preferably an acid anion;

$R^6$ and $R^7$ are individually straight or branched chain alkyl containing 1 to 18, preferably 1 to 10 carbon atoms; substituted or unsubstituted aryl containing 6 to 20 carbon atoms; or polystyryl having appended indolizine or indolizinium groups or combinations thereof;

$R^8$ is a monovalent group which, with the oxoindolizinium nucleus, completes an organic chromophore;

$R^9$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms, bromine or chlorine; and

$R^{10}$ is hydrogen; chlorine, bromine or alkyl containing 1 to 18 carbon atoms, Alkyl groups which are suitable for use as $R^1$, $R^2$, $R^4$, $R^5$, $R^6$, $R^7$, $R^9$ or $R^{10}$ substituents include, for example, methyl, ethyl and straight or branched chain propyl, butyl, amyl, decyl, dodecyl or lauryl.

Aryl groups which are suitable for use as $R^1$, $R^2$, $R^6$ or $R^7$ substituents include, for example, unsubstituted or substituted phenyl tolyl, xylyl, methoxyphenyl, 4-t-butylphenyl, anisyl, naphthyl or methoxynaphthyl.

Examples of acyl groups which are suitable for use as $R^4$ and $R^9$ substituents include acetyl, propionyl, 2-ethylhexanoyl and stearoyl.

Examples of acyloxy groups which are suitable for use as $R^4$ and $R^9$ substituents include acetoxy, propionoxy, butyroxy and lauroyloxy.

Examples of carboalkoxy and aminocarbonyl groups which are suitable for use as $R^4$ and $R^9$ substituents include, respectively, carbomethoxy, carboethoxy and carbobutoxy, and unsubstituted aminocarbonyl or methylaminocarbonyl, dimethylaminocarbonyl and ethylaminocarbonyl.

Examples of X anions are methanesulfonate, trifluoromethanesulfonate, paratoluenesulfonate, bromide, chloride, iodide and sulfinate.

The $R^5$ and $R^{10}$ substituents, as defined above, are such that they have no adverse affect upon the desired dye properties of the described oxoindolizine and oxoindolizinium compounds.

Useful $R^3$ and $R^8$ groups are, for example

a) substituted or unsubstituted heterocyclyl or heterocyclylidene groups optionally appended through methine and polymethine groups, such as i) indolizine and indolizinium groups illustrated by structures (I) and (II) appended directly as the respective $R^3$ and $R^8$ groups or appended through a substituted or unsubstituted methine or polymethine chain, such as containing 1 to 6 methine groups, ii) pyridylidene, iii) pyranyl, iv) pyranylidene, v) thiopyranyl, vi) thiopyranylidene, and vii) julolidyl; including the onium salts of such heterocyclyl and heterocyclylidene groups, such as the immonium, oxonium and sulfonium salts; and the acid addition salt derivatives of such heterocyclyl and heterocyclylidene groups;

b) substituted and unsubstituted aminoarylmethine and hydroxyarylmethine, including their tautomers, such as represented by the formula: (Z)(A)(D) wherein

Z is a methine or polymethine group, such as containing 1 to 6 methine groups;

A is a substituted or unsubstituted aromatic group, such as arylene containing 6 to 20 carbon atoms, for example, phenylene, phenylidene, naphthylene, and naphthylidene; and

D is $-OR^{129}$, $-NR^{130}R^{131}$, $=O$, or $=NR^{132}$ wherein $R^{129}$ is a monovalent cation, preferably hydrogen, $R^{130}$ and $R^{131}$ are independently selected from hydrogen, substituted or unsubstituted alkyl, such as alkyl containing 1 to 20 carbon atoms, alkenyl, such as alkenyl containing 2 to 20 carbon atoms, and aryl, such as aryl containing 6 to 20 carbon atoms, including phenyl and tolyl; or, $R^{130}$ and $R^{131}$ taken together with (A) form a polycyclic heterocyclic group, such as a 9-julolidyl group;

$R^{132}$ is alkyl, such as alkyl containing 1 to 20 carbon atoms or aryl such as aryl containing 6 to 20 carbon atoms;

c) a methylene group substituted with at least one, preferably two electronegative groups, such as acyl, cyano, aryl, alkoxycarbonyl, and aminocarbonyl groups; and

d) a formyl group.

$X^{\ominus}$ is an anion, for example, methanesulfonate, trifluoromethanesulfonate, para-toluenesulfonate, bromide, chloride, iodide, and sulfinate.

The term "enol" herein means an enol from the keto form of the dye as well as an enol produced by a protonation reaction or other reaction. For example, typical enols are represented by the formula:

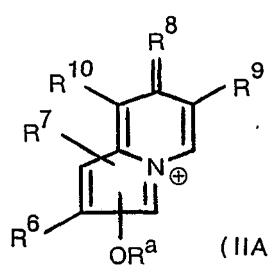 $X^{\ominus}$ (IIA) or 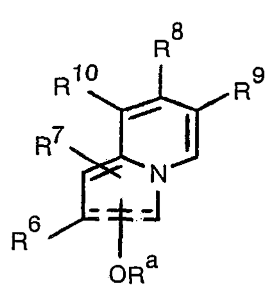 (IIB)

wherein $X^{\ominus}$, $R^3$, $R^6$, $R^7$, $R^8$, $R^9$ and $R^{10}$ are as defined above and $R^a$ is hydrogen or acyl.

The term "acyl" herein means alkylcarbonyl containing 2 to 20 carbon atoms and arylcarbonyl, such as arylcarbonyl containing 7 to 20 carbon atoms.

The term "aryl" means unsubstituted or substituted aryl containing 6 to 20 carbon atoms, such as phenyl, tolyl, xylyl, naphthyl, and methoxyphenyl.

As noted above, the preparations of oxoindolizine and oxoindolizinium dyes of the invention do not involve complicated reaction steps as do the preparations of prior art dyes.

The oxoindolizine and oxoindolizinium dyes of this invention are prepared by

1) reaction of a cyclopropenone compound with a pyridine compound, or
2) reaction of a cyclopropenone compound with a pyridine compound and then with a color-forming compound, or
3) a condensation reaction. The term "condensation reaction" herein means a dehydration involving, for example, an active methylene and a carbonyl group.

A useful pyridine compound does not contain a substituent in the 2- and 6-position on the pyridine ring. Pyridine compounds do not form oxoindolizine or oxoindolizinium dyes when substituted in the 2- or 6-position, that is in the positions next to the ring nitrogen atom.

The oxoindolizine and oxoindolizinium dyes herein are alternatively named as indolizinone compounds.

Many pyridine compounds are useful in forming a dye compound of this invention. Examples of useful pyridine compounds are represented by the formula:

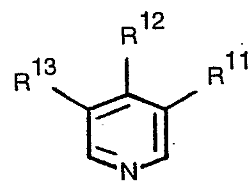

(III)

wherein

$R^{11}$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine;

$R^{12}$ is hydrogen; alkyl containing 1 to 18 carbon atoms; cyano; acyl containing to to 20 carbon atoms; benzyl or pyridyl; and

$R^{13}$ is hydrogen; chlorine; bromine or alkyl contfaining 1 to 18 carbon atoms.

Alkyl groups which are suitable for use as $R^{11}$, $R^{12}$ and $R^{13}$ substituents include, for example, methyl, ethyl, decyl and dodecyl.

Acyl groups which are suitable for use as $R^{11}$ and $R^{12}$ substituents include acetyl, propionyl, 2-ethylhexanoyl, stearoyl and lauroyl.

Examples of carboalkoxy and aminocarbonyl groups which are useful as $R^{11}$ substituents include, respectively, carbomethoxy, carboethoxy and carbobutoxy, and unsubstituted aminocarbonyl or methylaminocarbonyl, dimethylaminocarbonyl and ethylaminocarbonyl.

Acyloxy groups which are suitable for use as $R^{11}$ substituents include acetoxy, propionoxy, butyroxy and lauroyloxy.

Examples of useful pyridine compounds for preparation of dyes according to the invention are:

P–1 4,4′-Dipyridylethylene:

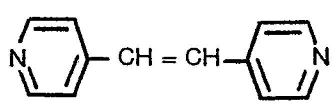

P–2 1-Methyl-4-(4-pyridyl)pyridinium-p-toluenesulfonate:

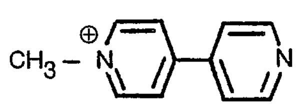

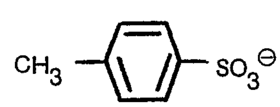

P–3 Pyridine:

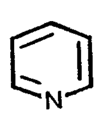

P–4 4-Picoline:

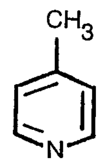

P–5 4-Formylpyridine (also known as 4-pyridinecarboxaldehyde):

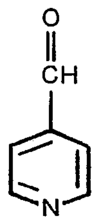

P–6 4-(4-Azastyryl)-1-methylpyridinium *p*-toluene sulfonate:

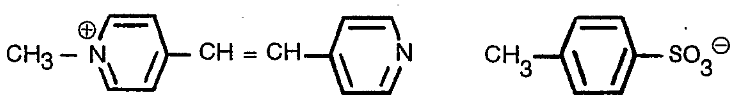

P–7 4-Acetylpyridine:

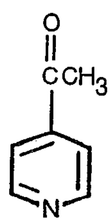

P–8 3-Acetylpyridine:

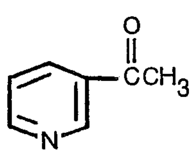

P–9 3-Benzylpyridine:

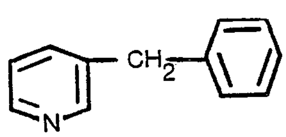

P–10 4-Benzylpyridine:

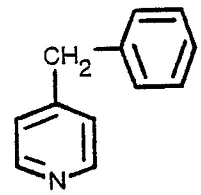

P–11 3-Bromopyridine:

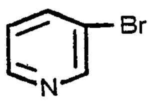

P–12 4-(*p*-chlorobenzyl)pyridine:

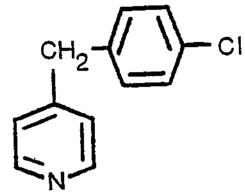

P–13 3-Chloropyridine:

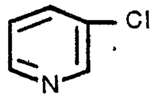

P–14 3-Cyanopyridine:

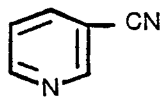

P–15 3,5-Dichloropyridine:

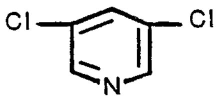

P–16 N,N-diethylnicotinamide:

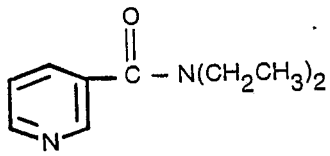

P–17 3-Ethylpyridine:

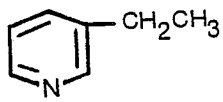

P–18 4-Ethylpyridine:

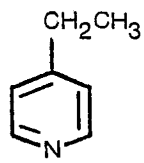

P–19 Ethyl-3-pyridylacetate:

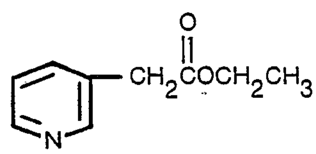

P–20 3,4-Lutidine:

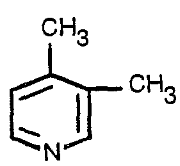

P–21 3,5-Lutidine:

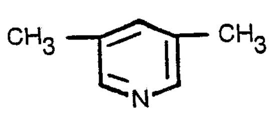

P–22 2-Methyl-1,2-di-3-pyridyl-1-oxo-propane:

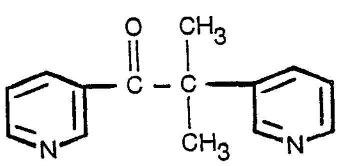

P–23 N-methylnicotinamide:

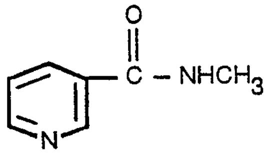

P–24 Methyl nicotinate:

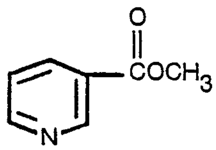

P–25 3-Picoline:

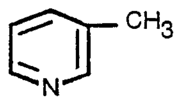

P–26 3-Formylpyridine (also known as 3-Pyridinecarboxaldehyde):

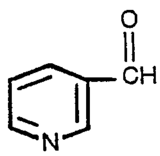

P–27 3-Cyanomethylpyridine (also known as 3-Pyridylacetonitrile):

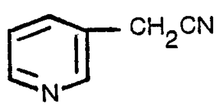

P–28 3-(3-pyridyl)-1-propanol:

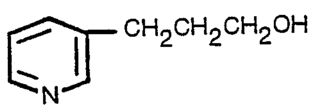

P–29 Trans-1-(3-pyridyl)-2-(4-pyridyl)ethylene:

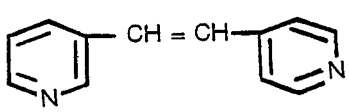

P–30 4-Cyanopyridine:

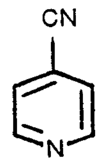

P–31 1-Benzyl-4-(4-pyridyl)pyridinium bromide:

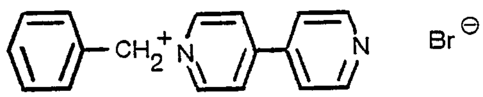

Many cyclopropenone compounds are useful for preparing dyes according to the invention. Examples of useful cyclopropenones are represented by the formula:
(IV)

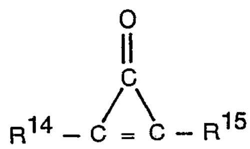

(IV)

wherein:

$R^{14}$ and $R^{15}$ are individually aryl containing 6 to 20 carbon atoms; aralkenyl containing 6 to 20 carbon atoms; alkyl containing 1 to 18, preferably 1 to 10 carbon atoms; or $R^{14}$ and $R^{15}$ together represent the carbon atoms necessary to complete a 7- or 8-member cyclic structure.

Aryl groups which are suitable for use as $R^{14}$ and $R^{15}$ substituents include, for example, unsubstituted and substituted phenyl, naphthyl or anthryl, such as methoxyphenyl and methoxynaphthyl.

Aralkenyl groups which are suitable for use as $R^{14}$ and $R^{15}$ substituents include for example, 2,2-diphenylvinyl, 2-phenylvinyl, 2-naphthylvinyl and 2-methyl-(2-phenylvinyl).

Alkyl groups which are suitable for use as $R^{14}$ and $R^{15}$ substituents include methyl, ethyl, propyl, decyl and lauryl.

An example of an $R^{14}$ and $R^{15}$ cyclic structure is 2,3-pentamethylene.

The aryl group of $R^{14}$ and $R^{15}$ is unsubstituted or substituted by one or more groups such as:

1) alkyl or alkoxy containing 1 to 5 carbon atoms, for example, methyl, ethyl, propyl, isopropyl, butyl, methoxy, ethoxy, propoxy and butoxy;
2) nitro;
3) aryloxy containing 6 to 10 carbon atoms, such as phenoxy and naphthoxy;
4) halogen, for example, chlorine, fluorine, iodine and bromine;
5) a homopolymer or copolymer to which the aryl group is attached as a pendant moiety with the polymer having at least one repeating unit represented by the formula:

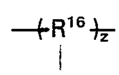

wherein:

$R^{16}$ is a lower alkylene group containing from 1 to 5 carbon atoms; and

z is at least a portion of the number of repeating units in a polymer chain, such as 10 to 1000.

Examples of useful cyclopropenone compounds, some of which are described in U.S. Patent No. 4,128,422, are:

2,3-diphenylcyclopropenone
2-(2-methoxynaphthyl)-3-phenylcyclopropenone
2-(2-methoxynaphthyl)-3-(4-methoxyphenyl)cyclopropenone
2,3-bis(2-methoxynaphthyl)cyclopropenone
2,3-bis(2,4-dimethylphenyl)cyclopropenone
2,3-bis(4-n-butoxyphenyl)cyclopropenone
2,3-bis(4-methoxyphenyl)cyclopropenone
poly[styrene-co-4-(2-phenylcyclopropenonyl)styrene]
2,3-bis(4-phenoxyphenyl)cyclopropenone

2-(4-n-butoxyphenyl)-3-phenylcyclopropenone
2-(2,5-dimethylphenyl)-3-phenylcyclopropenone
2-(4-methoxyphenyl)-3-phenylcyclopropenone
2-(2,4-dimethoxyphenyl)-3-phenylcyclopropenone
2,3-bis(2,4-dimethoxyphenyl)cyclopropenone
2,3-bis(2-methyl-5-isopropylphenyl)cyclopropenone
2,3-bis(3-nitrophenyl)cyclopropenone
2,3-bis(2,5-dimethylphenyl)cyclopropenone
2,3-bis(4-methylphenyl)cyclopropenone
2,3-di-*n*-propylcyclopropenone
2,3-pentamethylenecyclopropenone
2-(2,4-dimethoxyphenyl)-3-(2,4-dimethylphenyl)-cyclopropenone
2,3-bis(2,5-dimethoxyphenyl)cyclopropenone
2-(2,4,6-trimethylphenyl)-3-phenylcyclopropenone
2-phenyl-3-(2,5-dimethoxyphenyl)cyclopropenone
2-phenyl-3-(2,4-dimethylphenyl)cyclopropenone
2,3-bis(2,2-diphenylvinyl)cyclopropenone
2,3-bis(2-methyl-2-phenylvinyl)cyclopropenone

The described cyclopropenones are prepared by processes known in the organic synthesis art.

The cyclopropenone compounds may be spectrally sensitized using procedures and compounds known in the photographic art, such as described in U.S. Patent No. 4,128,422.

The color-forming compound may, for example, be a photographic color-forming coupler as used in silver halide color photography.

Especially useful phenolic, aniline and active methylene couplers for forming dyes according to this invention are those which are known to be useful in the photographic art for producing dye images.

The term "phenolic coupler" herein means a phenolic or naphtholic compound which forms a dye by reaction with a described oxoindolizine or oxoindolizinium compound.

Examples of useful phenolic couplers are represented by the formula:

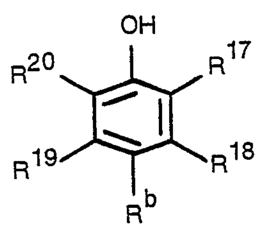

(V)

wherein:

$R^b$, $R^{17}$, $R^{18}$, $R^{19}$ and $R^{20}$ individually represent substituents which do not adversely affect the desired indolizinone and indolizinium dyes, such as by altering the solubility or desired dye hue, and individually represent substituents that are useful in phenolic couplers in the photographic art, such as described in, for example, U.S. Patent No. 3,620,747. In Structure V at least one of $R^{17}$, $R^{20}$ and $R^b$ is hydrogen. For example, $R^b$, $R^{17}$ and $R^{18}$ are individually hydrogen; hydroxyl; alkyl containing 1 to 22 carbon atoms, such as methyl, ethyl, propyl and decyl; aryl containing 6 to 20 carbon atoms, such as phenyl and tolyl; amino; carboxamido; sulfonamido; sulfamyl; carbamyl; halogen; such as chlorine, fluorine, bromine and iodine; and alkoxy containing 1 to 18 carbon atoms, such as methoxy, ethoxy and propoxy;

$R^{19}$ and $R^{20}$ are individually hydrogen, alkyl containing 1 to 22 carbon atoms, such as methyl, ethyl, propyl and decyl; aryl containing 6 to 20 carbon atoms, such as phenyl and tolyl; amino; carboxamido; sulfonamido, sulfamyl; carbamyl; halogen, such as chlorine, fluorine, bromine and iodine; and alkoxy containing 1 to 18 carbon atoms, such as methoxy, ethoxy and propoxy; or $R^{19}$ and $R^{20}$ taken together represent the atoms necessary to complete a benzo group which is unsubstituted or substituted by at least one of the groups given for $R^{17}$;

Examples of useful phenolic couplers are:

C–1 2-Acetylamino-5-methylphenol

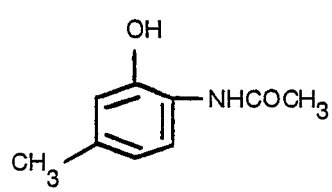

C–2 2-[α-(4′-tert.-amylphenoxy)butyrylamino]-5-methyl-1-phenol

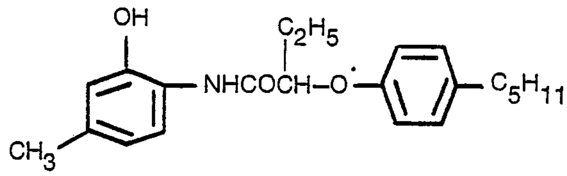

C–3 2-cyanoacetamidophenol

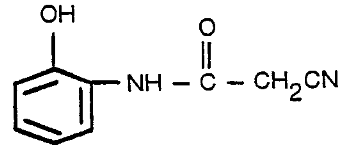

C–4 2-(2-stearoyloxyethyl)iminomethylphenol

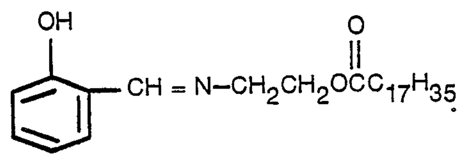

C–5 2-octadecyloxyphenol

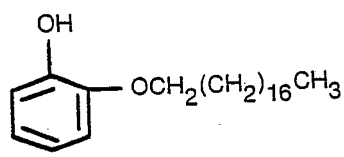

C–6 2-perfluorobutyramido-5-propionamidophenol

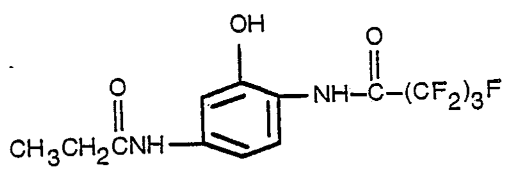

C–7 2-octadecyl aminocarbonyl-1-naphthol

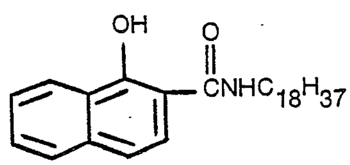

C–8 2-(2-sulfonoxy-4-stearoylamino anilinocarbonyl)-1-naphthol

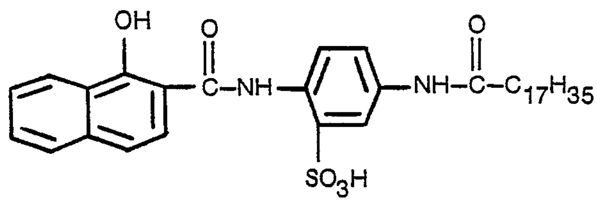

C–9 2-(propylaminocarbonyl)-1-naphthol

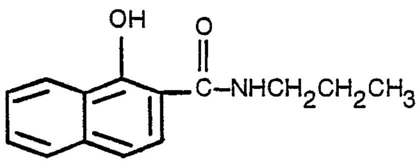

C–10 2-[α-(4-tert-amylphenoxy)butyryl amino] phenol

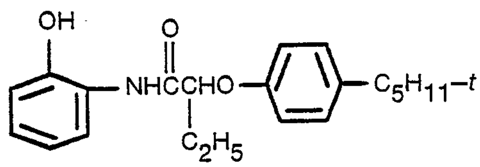

C–11 2-(N-methylanilinocarbonyl)-1-naphthol

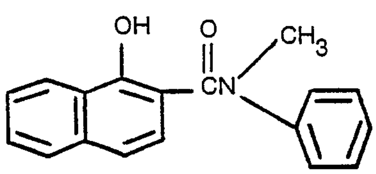

C–12 2-[2-(2-acetamidophenyl)ethyl aminocarbonyl]-1-naphthol

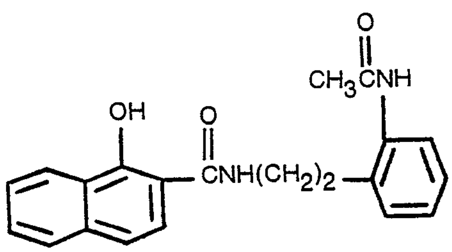

C–13 2-(4-tert-butylbenzamido resorcinol

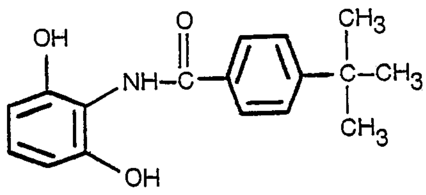

C–14 resorcinol

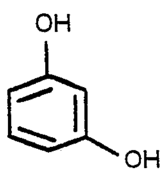

C–15 2-(2-amyloxybenzamido)resorcinol

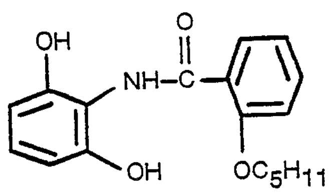

C–16 bis-4,4′-resorcinyl sulfide

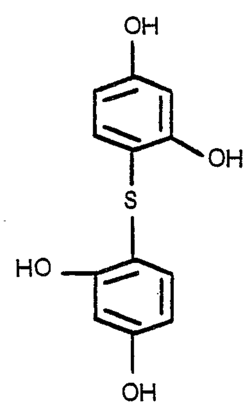

C–17 2-propinoamidoresorcinol

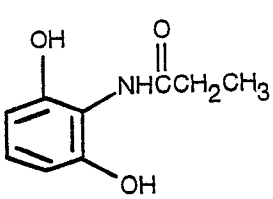

C—18 2-benzamidoresorcinol

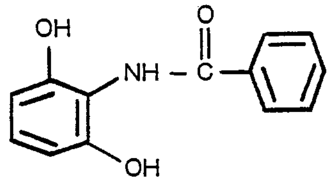

C—19 2,6-di-tert-butylphenol

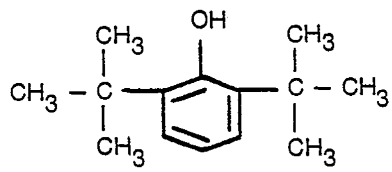

The term "aniline coupler" herein means an aniline compound or related derivative which forms a dye by reaction with a described oxoindolizine or oxoindolizinium compound.

Examples of useful aniline couplers and derivatives thereof useful in forming oxoindolizine and oxoindolizinium dyes according to the invention are represented by the formulas:

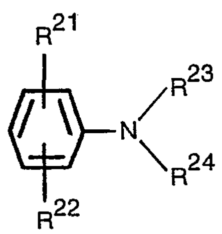

(VI)

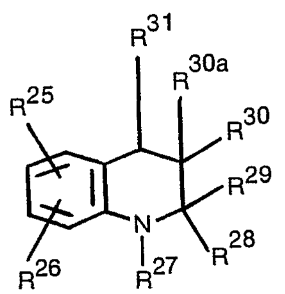

(VII)

and

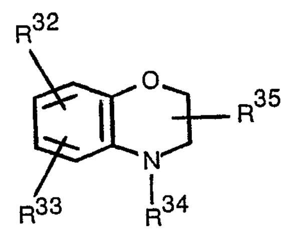

(VIII)

wherein

$R^{21}$, $R^{22}$, $R^{25}$, $R^{26}$, $R^{32}$ and $R^{33}$ are individually hydrogen; fluorine; chlorine; bromine; alkyl containing 1 to 6 carbon atoms; cycloalkyl containing 3 to 10 carbon atoms; alkoxy containing 1 to 4 carbon atoms; phenoxy; alkylthio, such as alkylthio containing 1 to 4 carbon atoms; arylthio, such as arylthio containing 6 to 20 carbon atoms; and groups represented by the formula —NH—X—$R^{36}$ in which X is —CO—, —COO or —$SO_2$—;

$R^{23}$, $R^{24}$, $R^{27}$ and $R^{34}$ are individually selected from hydrogen; cycloalkyl, such as cycloalkyl containing 6 to 20 carbon atoms; straight or branched alkenyl containing 2 to 10 carbon atoms; alkyl containing 1 to 18 carbon atoms, or $R^{23}$ and $R^{24}$ together represent the atoms necessary to complete a 5- or 6-member heterocyclic ring with the nitrogen atom to which they are bonded, such as atoms completing a pentamethylene, ethyleneoxyethylene or ethylenesulfonylethylene group which forms a ring or a julolidyl group; or

$R^{23}$ and $R^{24}$ individually can be —S—$R^{37}$; wherein

$R^{37}$ is alkyl containing 1 to 6 carbon atoms, phenyl, phenyl substituted with halogen, alkoxy containing 1 to 6 carbon atoms, alkanoylamino containing 1 to 6 carbon atoms, cyano or lower alkoxycarbonyl, pyridyl, pyrimidinyl, benzoxazolyl, benzimidazolyl, benzothiazolyl, triazolyl; $SO_2R^{39}$; —$COOR^{40}$; —$OXR^{41}$; —NH—X—$R^{42}$; —X—$R^{43}$; —OCO—$R^{44}$; —$CONR^{45}R^{46}$; —$SO_2NHR^{47}$; —$SO_2NR^{48}R^{49}$; $R^{28}$, $R^{29}$, $R^{30}$, $R^{30a}$, $R^{31}$ and $R^{35}$ are individually selected from hydrogen and alkyl containing 1 to 6 carbon atoms;

$R^{36}$ is alkyl containing 1 to 6 carbon atoms or alkyl substituted by a group that does not adversely affect the desired indolizinone or indolizinium dye, such as halogen, hydroxy, phenoxy, aryl, such as aryl containing 6 to 20 carbon atoms, cyano, cycloalkyl, such as cycloalkyl containing 6 to 12 carbon atoms, alkylsulfonyl containing 1 to 6 carbon atoms, alkylthio containing 1 to 6 carbon atoms, alkanoyloxy containing 1 to 6 carbon atoms and alkoxy containing 1 to 6 carbon atoms; when X is —CO—, then $R^{36}$ is also selected from hydrogen, amino, alkenyl containing 2 to 6 carbon atoms, alkylamino containing 1 to 6 carbon atoms, alkylcarbamoyl containing 1 to 6 carbon atoms, dialkylamino containing 2 to 12 carbon atoms, arylamino containing 6 to 12 carbon atoms, aryl containing 6 to 20 carbon atoms and furyl.

When $R^{23}$, $R^{24}$, $R^{27}$ or $R^{34}$ are alkyl, the alkyl is unsubstituted or substituted by, for example, hydroxy, halogen, cyano, alkoxy containing 1 to 6 carbon atoms, alkoxyalkoxy containing 2 to 8 carbon atoms, hydroxyalkoxy containing 1 to 4 carbon atoms, succinimido, glutarimido, phenylcarbamoyloxy, phthalimido, phthalimidino, 2-pyrrolidono, cyclohexyl, phenoxy, phenyl or phenyl substituted by alkyl containing 1 to 6 carbon atoms, alkoxy containing 1 to 6 carbon atoms, halogen, alkanoylamino containing 1 to 6 carbon atoms, alkoxycarbonyl containing 2 to 6 carbon atoms; sulfamoyl; alkylsulfamoyl containing 1 to 6 carbon atoms; vinylsulfonyl; acrylamido; alkylsulfonamido, such as alkylsulfonamido containing 1 to 6 carbon atoms; phenylsulfonamido; alkoxycarbonylamino containing 1 to 6 carbon atoms; alkylcarbamoyloxy containing 1 to 6 carbon atoms; alkoxycarbonyloxy containing 1 to 6 carbon atoms; alkenylcarbonylamino containing 3 to 6 carbon atoms; groups represented by the formula:

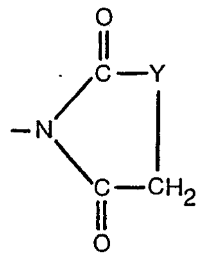

wherein

Y is —NH—, —N-alkyl

containing 1 to 6 carbon atoms, —O—, —S—, or —$CH_2O$—;

$R^{39}$, $R^{40}$, $R^{41}$, $R^{42}$, $R^{43}$ and $R^{44}$ are individually selected from unsubstituted alkyl containing 1 to 6 carbon atoms and alkyl containing 1 to 6 carbon atoms substituted by at least one group that does not adversely affect the desired oxoindolizine or oxoindolizinium dye, such as halogen, hydroxy, phenoxy, aryl containing 6 to 20 carbon atoms, cyano, cycloalkyl containing 6 to 12 carbon atoms, alkylsulfonyl containing 1 to 6 carbon atoms, alkylthio containing 1 to 6 carbon atoms, alkanoyloxy containing 1 to 6 carbon atoms; and alkoxy containing 1 to 6 carbon atoms, and when X is —CO—, then $R^{41}$, $R^{42}$ and $R^{43}$ are also individually selected from hydrogen, amino, alkenyl containing 2 to 6 carbon atoms, alkylamino containing 1 to 6 carbon atoms, alkyl carbamoyl containing 2 to 6 carbon atoms, dialkylamino containing 2 to 6 carbon atoms, arylamino containing 6 to 20 carbon atoms, aryl containing 6 to 20 carbon atoms or furyl;

$R^{45}$, $R^{46}$, $R^{47}$, $R^{48}$ and $R^{49}$ are individually selected from hydrogen, unsubstituted alkyl containing 1 to 6 carbon atoms and alkyl containing 1 to 6 carbon atoms substituted by at least one group that does not adversely affect the desired oxoindolizine or oxoindolizinium dye, such as halogen, hydroxy, phenoxy, aryl containing 6 to 20 carbon atoms, cyano, cycloalkyl containing 6 to 12 carbon atoms, alkylsulfonyl containing 1 to 6 carbon atoms, alkylthio containing 1 to 6 carbon atoms, alkanoyloxy containing 1 to 6 carbon atoms and alkoxy containing 1 to 6 carbon atoms, cyano, alkanoyloxy containing 1 to 6 carbon atoms, phenoxy, phenoxy substituted by at least one of alkyl containing 1 to 6 carbon atoms, alkoxy containing 1 to 6 carbon atoms, and halogen.

The term "cycloalkyl" herein means an unsubstituted cycloalkyl group or a cycloalkyl group containing substituents that do not adversely affect an oxoindolizine or oxoindolizinium dye according to the invention. The cycloalkyl group, for example, contains 3 to 7 carbon atoms and is unsubstituted or substituted by one or two groups selected from alkyl containing 1 to 4 carbon atoms, hydroxyl, alkoxy containing 1 to 4 carbon atoms, phenyl or phenyl containing an alkyl group containing 1 to 4 carbon atoms, alkoxy containing 1 to 4 carbon atoms, halogen, alkanoylamino, cyano and alkoxycarbonyl, such as alkoxycarbonyl containing 1 to 4 carbon atoms.

Examples of useful aniline couplers are as follows:

AN—1 N,N-dimethylaniline

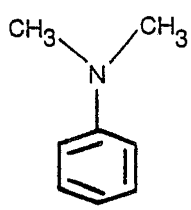

AN–2 julolidine

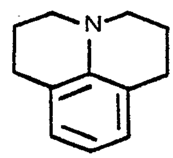

AN–3 N,N-diethylaniline

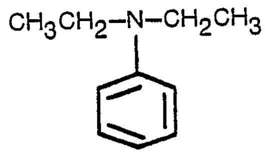

AN–4 N-phenylpiperidine

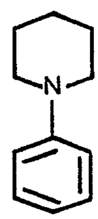

Examples of useful active methylene couplers for forming dyes according to the invention are represented by the formula:

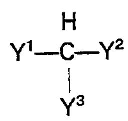

(IX)

wherein:

$Y^1$ and $Y^2$ are the same or different electronegative groups, such as aryl containing 6 to 20 carbon atoms, such as phenyl and naphthyl; cyano; acyl containing 2 to 18 carbon atoms, such as acetyl, propionyl and butyryl; carboalkoxy containing 1 to 18 carbon atoms, such as carbomethoxy, carboethoxy, carbobutoxy and carboamyloxy; aminocarbonyl containing 1 to 18 carbon atoms, such as unsubstituted aminocarbonyl, methylaminocarbonyl, dimethylaminocarbonyl and ethylaminocarbonyl; or oxo-, thio- or selenopyrylium; or oxoindolizinium; or $Y^2$ is hydrogen; and

$Y^3$ is hydrogen or halogen, such as chlorine, bromine and iodine.

Preferred active methylene couplers are ketomethylene couplers. Other useful active methylene couplers include those known to be useful in the photographic art, such as pyrazalinone and coumarin couplers.

Examples of preferred ketomethylene couplers are represented by the formula:

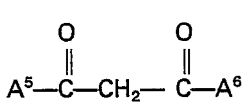

(X)

wherein:

$A^5$ and $A^6$ are individually alkyl containing 1 to 18 carbon atoms, such as methyl, ethyl, propyl and amyl; aryl containing 6 to 14 carbon atoms, such as phenyl, naphthyl and anthryl; hydroxy; alkoxy, such as alkoxy containing 1 to 6 carbon atoms; amino; substituted amino; or thiol.

Ketocarboxamides are examples of especially useful ketomethylene couplers for forming dyes according to the invention. Examples of useful ketocarboxamides are represented by the formula:

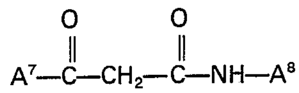

(XI)

wherein:

$A^7$ and $A^8$ are individually alkyl containing 1 to 18 carbon atoms, such as methyl, ethyl, propyl, butyl, amyl, decyl and stearyl; and aryl containing 6 to 14 carbon atoms, such as phenyl, naphthyl, and anthryl; carbonyl; amino or vinyl.

Other particularly useful active methylene couplers are alkyl flavylium salts and alkyl pyrylium salts, such as described in U.S. Patents 3,141,770 and 3,250,615.

Examples of useful methylene couplers include the following:

M–1 2,6-Diphenyl-4-methylpyrylium perchlorate

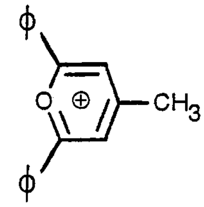

$ClO_4^{\ominus}$

M–2 2,4-Diphenyl-6-methylpyrylium perchlorate

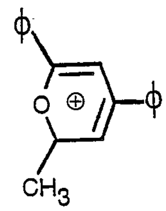

$ClO_4^{\ominus}$

M–3 2,6-Diphenyl-4-methylthiopyrylium perchlorate

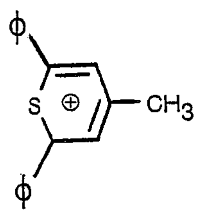

$ClO_4^{\ominus}$

M–4 4-Methyl-2-phenylflavylium perchlorate

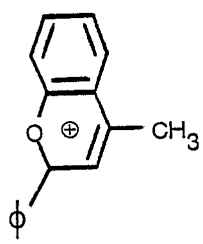

$ClO_4^{\ominus}$

M–5 2-Methyl-4-phenylflavylium perchlorate

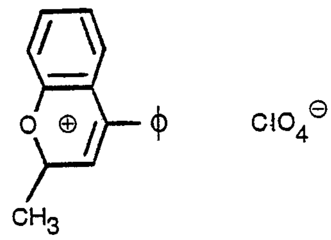

M–6 4-Methyl-2-phenylthioflavylium perchlorate

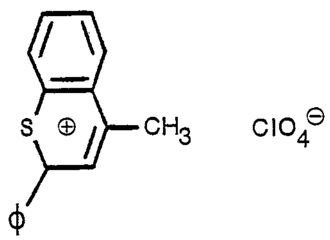

M–7 2,6-di-(2-thiopheneyl)-4-methylpyrylium fluoborate

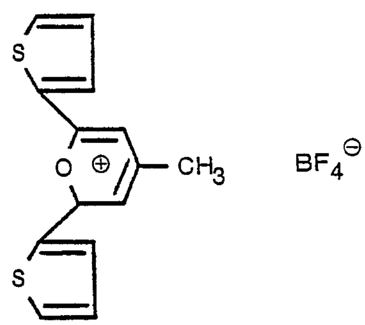

M–8 2-(4-methoxyphenyl)-4-methylthioflavylium perchlorate

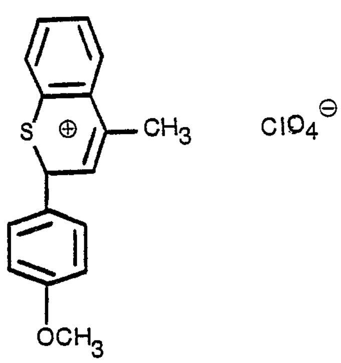

M–9 2,4-pentanedione

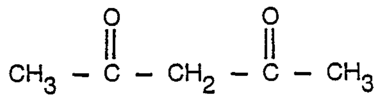

M–10 dibenzoylmethane

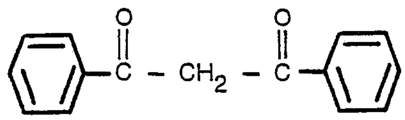

M–11 1-anilino-3-phenyl-1,3-propanedione

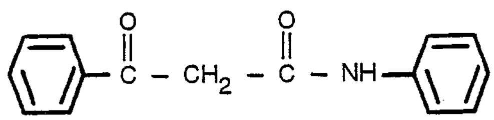

M–12

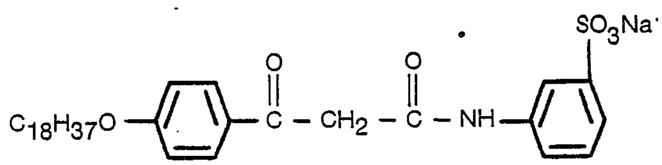

M–13 1-tert-butyl-3-(4-methoxy anilino)-1,3-propanedione

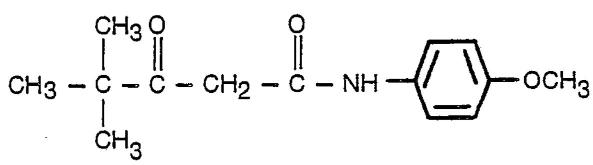

M–14 malononitrile

$CH_2(CN)_2$

M–15 phenylacetonitrile

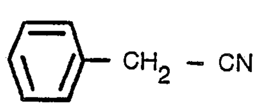

M–16 phenylacetamide

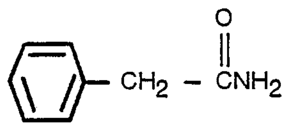

M–17 N-phenyl acetylacetamide

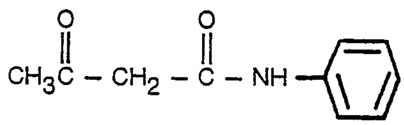

M–18 bis-nitrophenylmethane

$CH_2(C_6H_4NO_2)_2$

M–19 methyl cyanoacetate

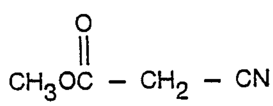

M–20 2,2-dimethyl-m-dioxane-4,6-dione

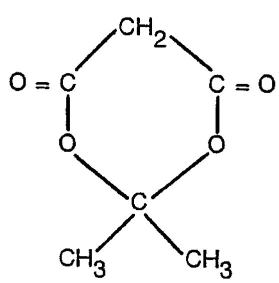

M–21 cyanoacetamide

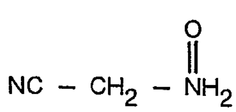

The designation ϕ herein means a phenyl group.

Other particularly useful active methylene couplers are alkyl indolizinonium salts represented by the formula:

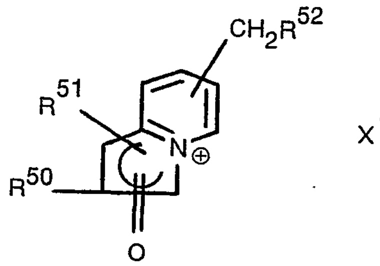

(XII)

wherein

$R^{50}$ and $R^{51}$ are individually aryl containing 6 to 14 carbon atoms, such as phenyl, naphthyl, anthryl, methoxyphenyl and methoxynaphthyl; aralkenyl containing 6 to 14 carbon atoms, such as 2,2-diphenylvinyl, 2-phenylvinyl, 2-naphthylvinyl and 2-methyl(2-phenylvinyl); alkyl containing 1 to 20 carbon atoms, such as methyl, ethyl, propyl, decyl and lauryl; or $R^{50}$ and $R^{51}$ together represent the carbon atoms necessary to complete a 7- or 8-member cyclic structure; and

$R^{52}$ is a substituent which does not interfere with the coupling action of the indolizinium salt and does not adversely affect the desired properties of a resulting oxoindolizinium or oxoindolizine dye, such as hydrogen; carboxyl; alkyl containing 1 to 18 carbon atoms, for example, methyl, ethyl, propyl and dodecyl; cyano; and, aryl containing 6 to 20 carbon atoms, such as phenyl and xylyl;

$X^{\ominus}$ is an anion as defined above, such as $CF_3SO_3^{\ominus}$, $Br^{\ominus}$ and $BF_4^{\ominus}$.

Another method of preparation of oxoindolizine and oxoindolizinium dyes within Structures I and II comprises condensation of suitable indolizinols, indolizinones or indolizinonium ions with active —CH= compounds which complete an organic chromophore. such indolizinols (IA), indolizinones (IB) and indolizinonium (IC) ions are represented by the formulas:

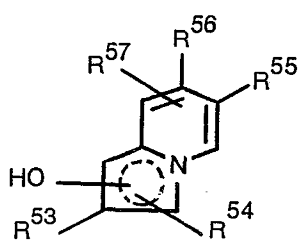

(IA)

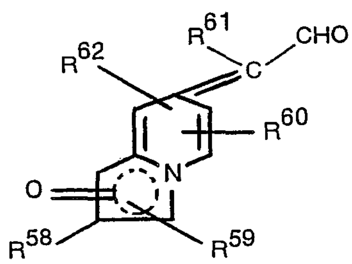

(IB)

and

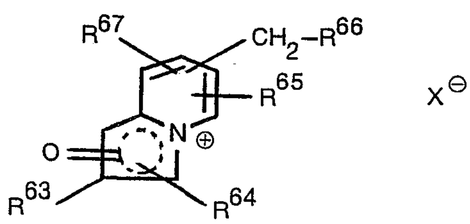

(IC)

wherein:

$X^{\ominus}$ is an anion as defined above;

$R^{53}$, $R^{54}$, $R^{58}$, $R^{59}$, $R^{63}$ and $R^{64}$ are individually aryl containing 6 to 14 carbon atoms, such as phenyl, naphthyl, anthryl, methoxyphenyl and methoxynaphthyl; aralkenyl containing 6 to 14 carbon atoms, such as 2,2-diphenylvinyl, 2-phenylvinyl, 2-naphthylvinyl and 2-methyl-(2-phenylvinyl), alkyl containing 1 to 18 carbon atoms, such as methyl, ethyl, propyl, decyl and octadecyl; or $R^{53}$ and $R^{54}$, $R^{58}$ and $R^{59}$, and $R^{63}$ and $R^{64}$ together represent the carbon atoms necessary to complete a 7- or 8-member cyclic structure;

$R^{55}$, $R^{60}$ and $R^{65}$ are individually hydrogen, alkyl containing 1 to 18 carbon atoms, such as methyl, ethyl, and dodecyl; cyano; acyl containing 2 to 18 carbon atoms, such as acetyl, propionyl, 2-ethylhexanoyl and stearoyl; carboalkoxy containing 1 to 18 carbon atoms, such as carbomethoxy, carboethoxy and carbobutoxy; aminocarbonyl, such as unsubstituted aminocarbonyl, methylaminocarbonyl, dimethylaminocarbonyl and ethylaminocarbonyl; acyloxy containing 2 to 18 carbon atoms, such as acetoxy, propionoxy, butyroxy and lauroyloxy; bromine and chlorine;

$R^{56}$ is hydrogen; alkyl containing 1 to 18 carbon atoms, such as methyl, ethyl, propyl and dodecyl; acyl containing 2 to 18 carbon atoms, such as acetyl, propionyl, butyryl and lauryl; benzyl or pyridyl;

$R^{57}$, $R^{62}$ and $R^{67}$ are individually hydrogen; chlorine; bromine; or, alkyl containing 1 to 18 carbon atoms, such as methyl, ethyl, propyl and dodecyl;

$R^{61}$ is alkyl containing 1 to 18 carbon atoms, such as methyl, ethyl, propyl, butyl and decyl; and

$R^{66}$ is alkyl containing 1 to 18 carbon atoms; hydrogen; carbonyl; alkoxycarbonyl, such as methoxycarbonyl, ethoxycarbonyl and propoxycarbonyl; cyano; and carboxamido.

Such indolizinols (IA), indolizinones (IB) and indolizinonium (IC) ions are prepared by reacting a cyclopropenone with a pyridine compound as described.

The term "active —CH= compounds" herein means aldehyde and ketone compounds which are capable of condensing with the active methylene of the indolizinonium ion (IC) and which have electropositive or electronegative substituents which complete a chromophore with the indolizinonium ion (IC). The term "active —CH= compounds" includes active methylene compounds that are capable of condensing with indolizinols (IA) or indolizinones (IB) to complete a chromophore. Examples of useful "active —CH= compounds" are as follows:

1,2-diphenyl-7-formyl-3-indolizinol

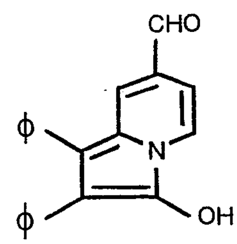

7-formylmethylidene-2,3-diphenyl-1(7H)-indolizinone

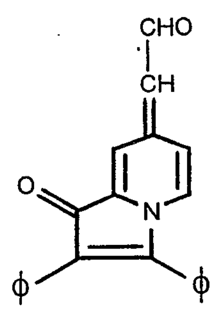

1,2-diphenyl-7-methyl-3-indolizinonium trifluoromethane sulfonate

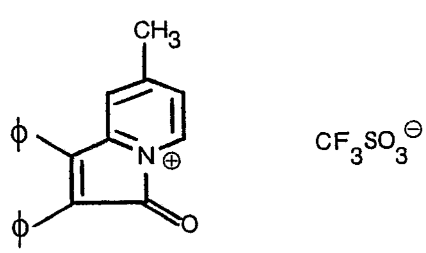

7-cyanomethyl-2,3-diphenyl-1-indolizinonium trifluoromethane sulfonate

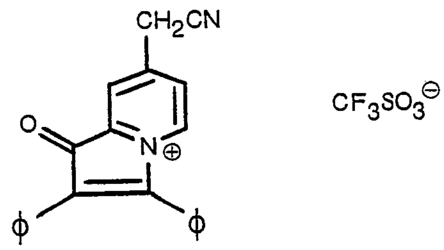

2,3-diphenyl-6-methyl-1-indolizinonium trifluoromethane sulfonate

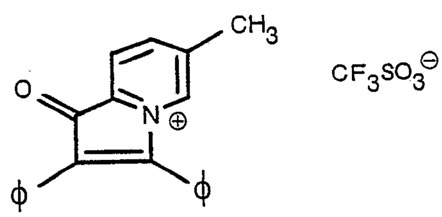

*p*-dimethylaminocinnamaldehyde

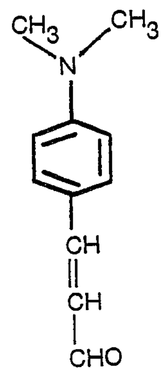

*p*-hydroxybenzaldehyde

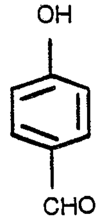

*p*-hydroxycinnamaldehyde

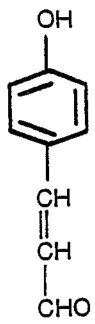

1-dimethylamino-4-formyl naphthalene

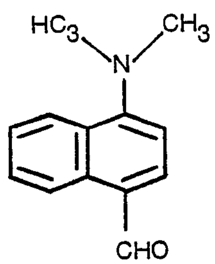

*p*-nitrobenzaldehyde

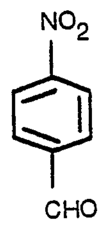

4-dimethylaminobenzaldehyde

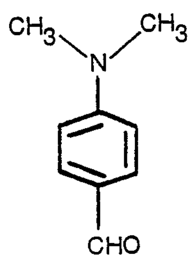

1,2-dimethyl-6-formyl-1,2,3-tetrahydroquinoline

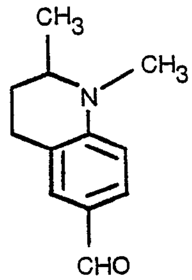

2,6-diphenyl-4-formylmethylidene(4H)pyran

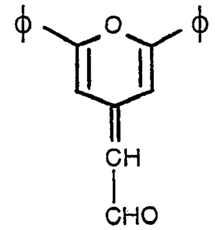

9-formyljulolidine

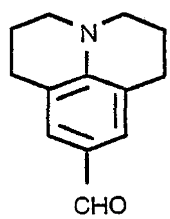

1-chloroethyl-6-formyl-2,2,4,7-tetramethyl-1,2,3,4-tetrahydroquinoline

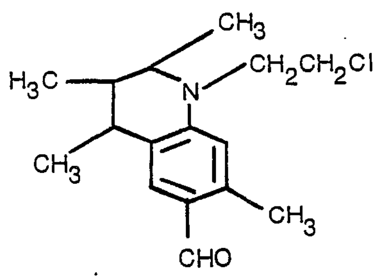

Many oxoindolizine dyes according to the invention are formed by the reaction of a phenolic coupler with an appropriate oxoindolizine. Examples of useful oxoindolizine dyes that are formed by reaction of phenolic couplers with a suitable oxoindolizine are represented by the formulas:

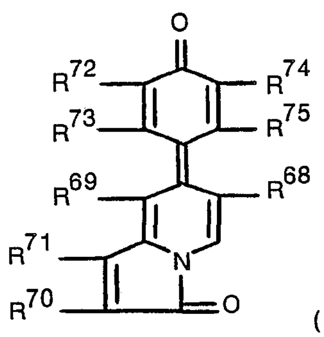

(XIII)

and

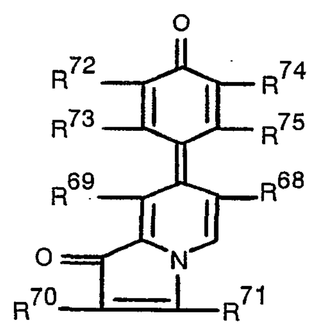

(XIV)

wherein:

$R^{68}$ is hydrogen or a substituent that does not adversely affect desired dye properties, such as alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine;

$R^{69}$ is hydrogen or a substituent that does not adversely affect desired dye properties, such as chlorine, bromine or alkyl containing 1 to 18 carbon atoms;

$R^{70}$ and $R^{71}$ are individually alkyl, containing 1 to 18, preferably 1 to 10 carbon atoms, or aryl containing 6 to 20 carbon atoms;

$R^{72}$ and $R^{73}$ are individually hydrogen; alkyl containing 1 to 22 carbon atoms; aryl containing 6 to 20 carbon atoms; amino; carboxamido; sulfonamido; sulfamyl; carbamyl; halogen, including chlorine, fluorine, bromine and iodine; alkoxy containing 1 to 18 carbon atoms, or $R^{72}$ and $R^{73}$ together represent the atoms necessary to complete a benzo group which is unsubstituted or substituted by at least one of the groups given above for $R^{17}$; and

$R^{74}$ and $R^{75}$ are individually hydrogen; hydroxy; alkyl containing 1 to 22 carbon atoms; aryl containing 6 to 20 carbon atoms; amino; carboxamido; sulfonamido; sulfamyl; carbamyl; halogen, including chlorine, fluorine, bromine and iodine; or alkoxy containing 1 to 18 carbon atoms.

Examples of alkyl groups which are suitable for use as $R^{68}$ to and including $R^{75}$ substituents include, where conforming to the above carbon length descriptions, methyl, ethyl straight or branched chain propyl, butyl, decyl, dodecyl and eicosyl.

Acyl groups which are suitable for use as an $R^{68}$ substituent include acetyl, propionyl, 2-ethylhexanoyl and stearoyl.

Examples of carboalkoxy and aminocarbonyl groups which are suitable for use as an $R^{68}$ substituent include, respectively, carbomethoxy, carboethoxy and carbobutoxy, and unsubstituted aminocarbonyl or methylaminocarbonyl, dimethylaminocarbonyl, and ethylaminocarbonyl.

Acyloxy groups which are suitable for use as an $R^{68}$ substituent include acetoxy, propionoxy, butyroxy and lauroyloxy.

Examples of aryl groups which are suitable for use as $R^{70}$ to and including $R^{75}$ substituents are unsubstituted and substituted groups such as phenyl, tolyl, xylyl, methoxyphenyl, 4-t-butylphenyl, anisyl, naphthyl and methoxynaphthyl.

Examples of alkoxy groups which are suitable for use as $R^{72}$ to and including $R^{75}$ substituents are methoxy, ethoxy and propoxy.

An example of a useful class of oxoindolizine dyes prepared from phenolic couplers are those derived from resorcinolic couplers. Resorcinolic couplers form compounds wherein $R^{75}$ is hydroxy.

Examples of oxoindolizine dyes prepared from phenolic couplers are as follows:

1,2-diphenyl-7-(4-oxo-2-hydroxy-1-phenylidene)-3-(7H)-indolizinone

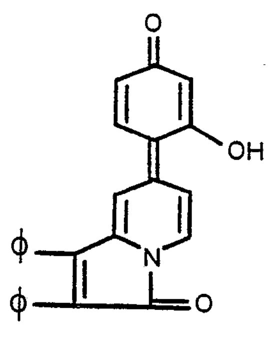

1,2-diphenyl-7-(4-oxo-1-naphthylidene)-3(7H)-indolizinone

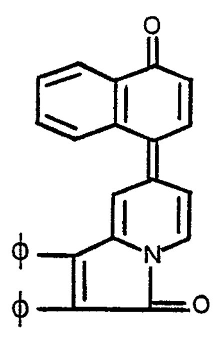

1,2-diphenyl-6-methyl-7-(4-oxo-1-phenylidene)-3(7H)-indolizinone

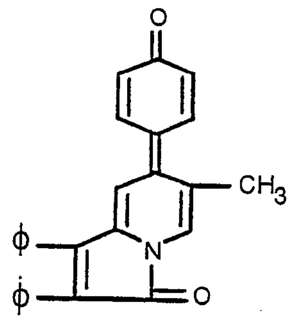

2,3-diphenyl-6-formyl-7-(4-oxo-1-phenylidene)-1-(7H)-indolizinone

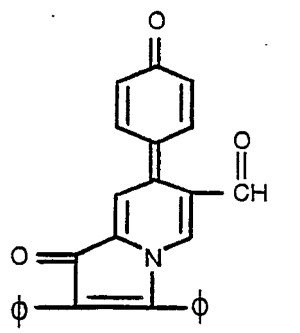

6-diethylaminocarbonyl-2,3-diphenyl-(4-oxo-1-phenylidene)-1(7H)-indolizinone

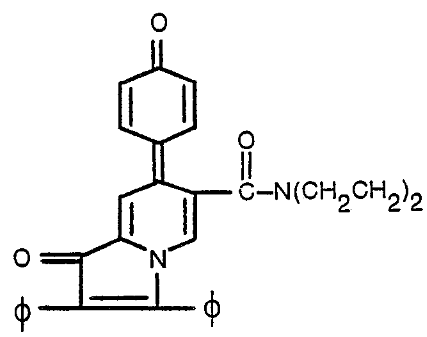

1,2-diphenyl-6-ethyl-7-(4-oxo-1-phenylidene)-3(7H)-indolizinone

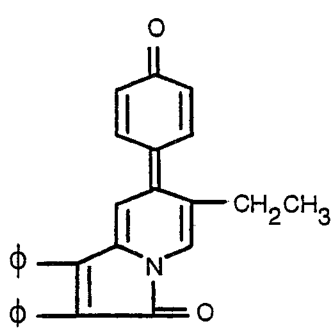

6-cyanomethyl-1,2-diphenyl-7-(4-oxo-1-phenylidene)-
3(7H)-indolizinone

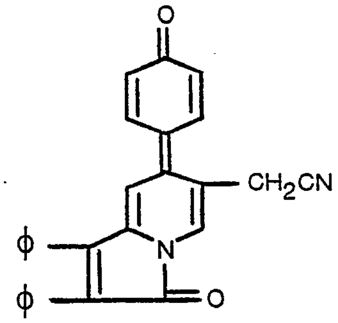

1,2-diphenyl-6-(3-hydroxypropyl)-7-(4-oxo-1-phenylidene)-
3(7H)-indolizinone

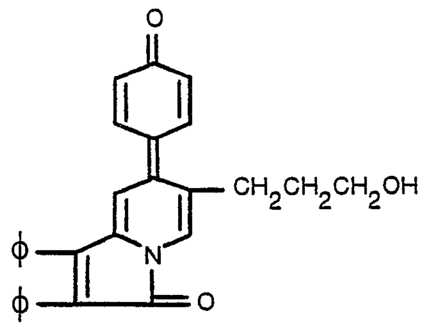

1,2-diphenyl-6-ethoxycarbonylmethyl-6-(4-oxo-1-phenylidene)-
3(7H)-indolizinone

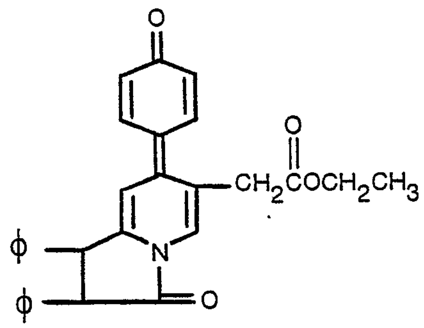

6,8-dimethyl-1,2-diphenyl-7-(4-oxo-1-phenylidene)-
3(7H)-indolizinone

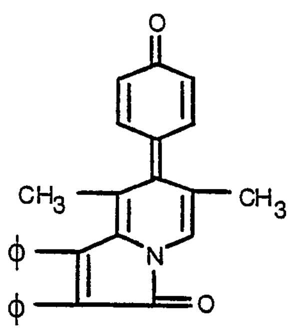

2,3-diphenyl-6-methylaminocarbonyl-7-(4-oxo-1-phenylidene)-1(7H)-indolizinone

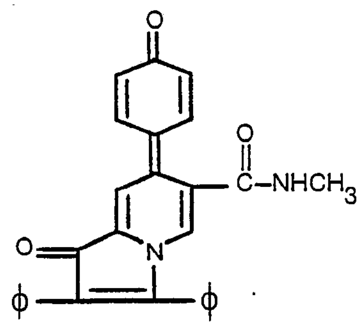

2,3-diphenyl-6-methoxycarbonyl-7-(4-oxo-1-phenylidene)-1(7H)-indolizinone

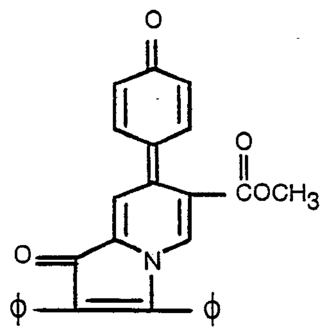

2,3-diphenyl-6-[2-methyl-2-(3-pyridyl)-propionyl-7-(4-oxo-1-phenylidene)]-1(7H)-indolizinone

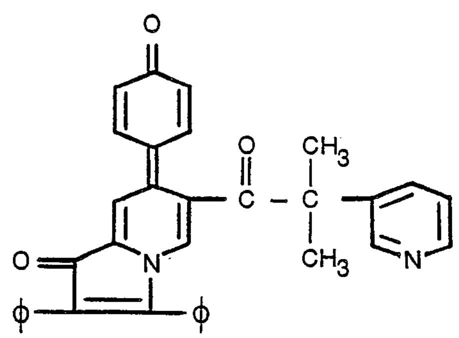

1,2-bis{6,6'-[2,3-diphenyl-7-(4-oxo-1-phenylidene)-1(7H)-indolizinonyl]}-2-methyl-1-oxopropane

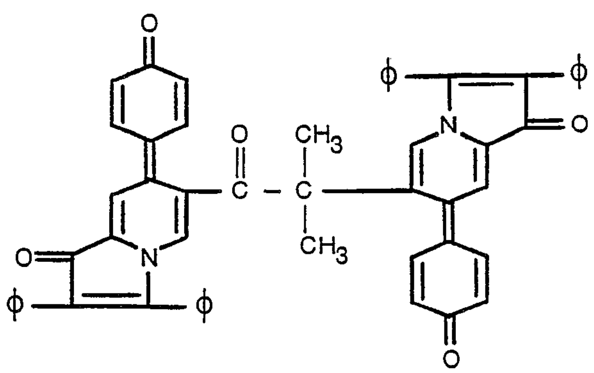

6-acetyl-2,3-diphenyl-7-(4-oxo-phenylidene)-
1(7H)-indolizinone

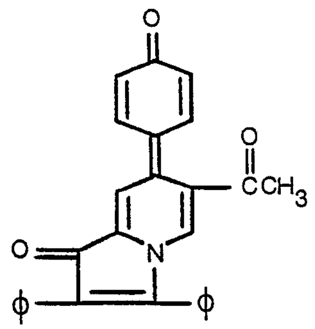

6-benzyl-1,2-diphenyl-7-(4-oxo-1-phenylidene)-
3(7H)-indolizinone

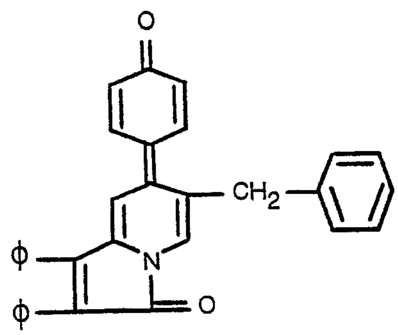

6-chloro-1,2-diphenyl-7-(4-oxo-1-phenylidene)-
3(7H)-indolizinone

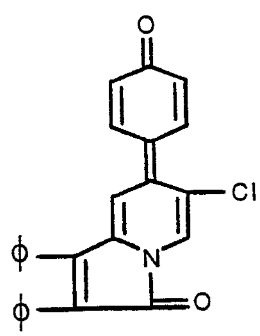

6-cyano-2,3-diphenyl-7-(4-oxo-1-phenylidene)-
1(7H)-indolizinone

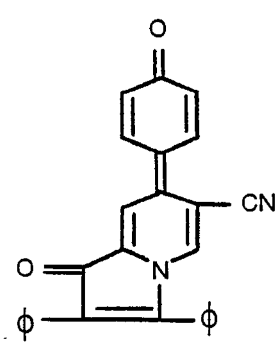

6-(4-azastyryl)-1,2-diphenyl-7-(4-oxo-1-phenylidene)-3(7H)-indolizinone

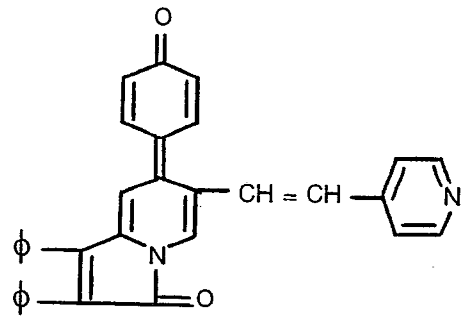

2,3-diphenyl-7-(2-hydroxy-4-oxo-3-pivalamido-1-phenylidene)-1(7H)-indolizinone

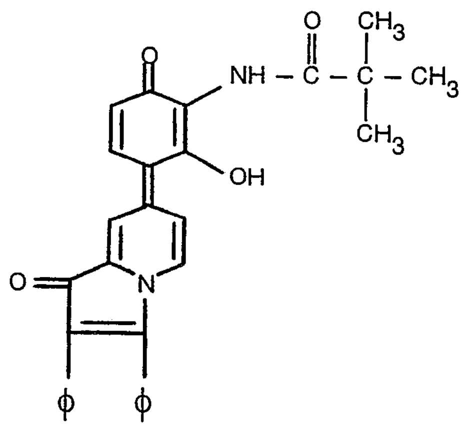

6-(4-azastyryl)-7-[3-(4-tert-butylbenzamido)-2-hydroxy-4-oxo-1-phenylidene]-1,2-diphenyl-3(7H)-indolizinone

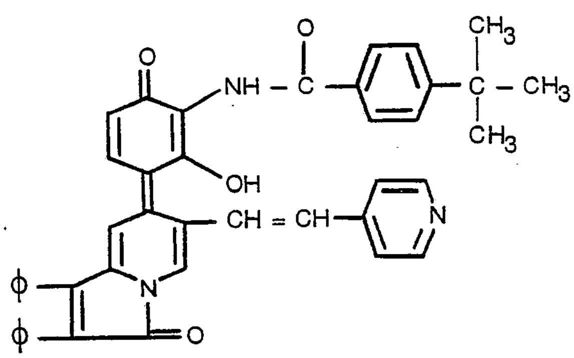

7-[3-(4-tert-butylbenzamido)-2-hydfoxy-4-oxo-1-phenylidene)]-1,2-diphenyl-6-(3-hydroxypropyl)-3-(7H)-indolizinone

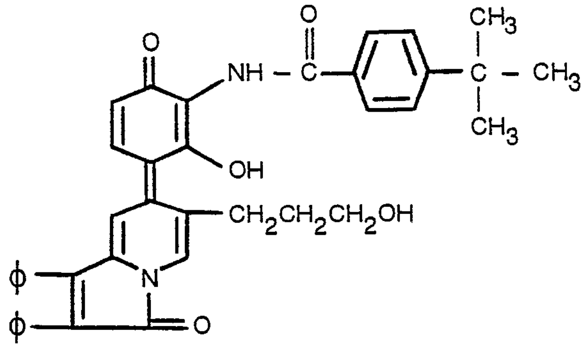

7-[3-(4-tert-butylbenzamido)-2-hydroxy-4-oxo-1-phenylidene]-6-carbomethoxy-2,3-diphenyl-1(7H)-indolizinone

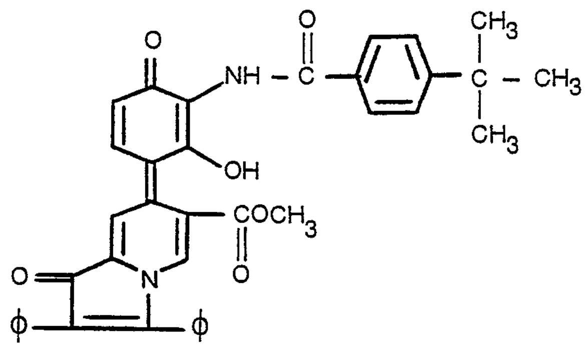

7-[3-(4-tert-butylbenzamido)-2-hydroxy-4-oxo-1-phenylidene]-2,3-diphenyl-6-methylcarbamyl-1(7H)-indolizinone

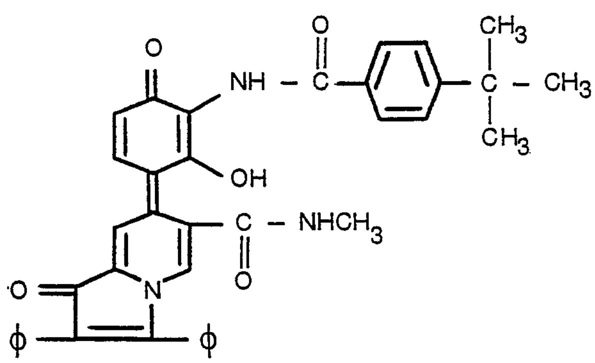

7-[3-(4-tert-butylbenzamido)-2-hydroxy-4-oxo-1-phenylidene]-
1,2-diphenyl-6-methyl-3(7H)-indolizinone

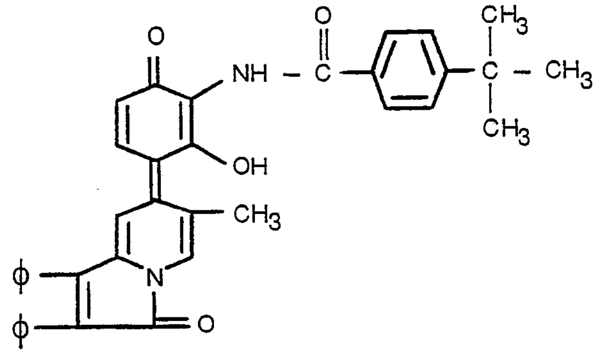

7-[3-(4-tert-butylbenzamido)-2-hydroxy-4-oxo-1-phenylidene]-
6,8-dimethyl-1,2-diphenyl-3(7H)-indolizinone

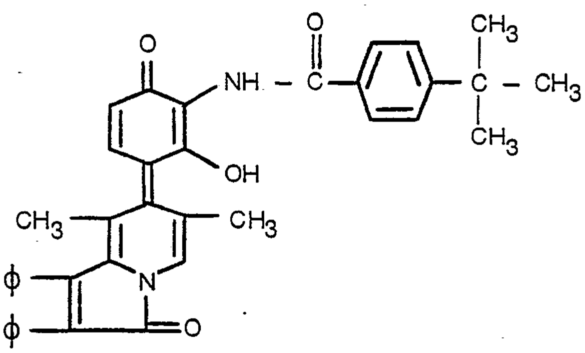

7-[3-(4-tert-butylbenzamido)-2-hydroxy-4-oxo-1-phenylidene]-
6-diethylcarbamyl-2,3-diphenyl-1(7H)-indolizinone

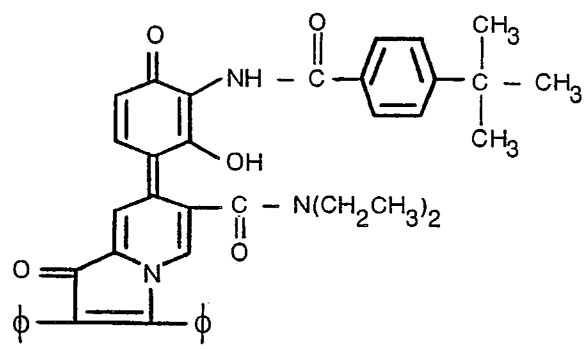

6-benzyl-7-[3-(4-tert-butylbenzamido)-2-hydroxy-4-oxo-1-phenylidene]-
1,2-diphenyl-3(7H)-indolizinone

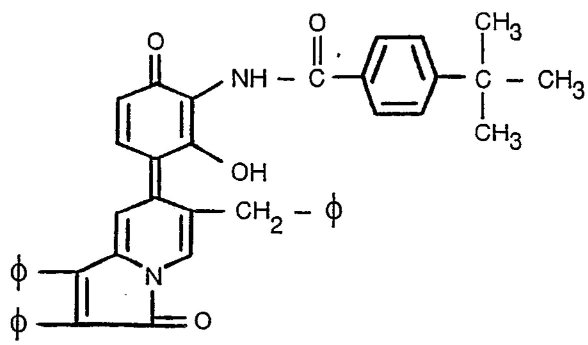

1,2-bis-{6,6′-{7-[3-(4-tert-butylbenzamido)-2-hydroxy-4-oxo-1-phenylidene]}-2,3-diphenyl-1(7H)-indolizinonyl-}2-methyl-1-oxo-propane

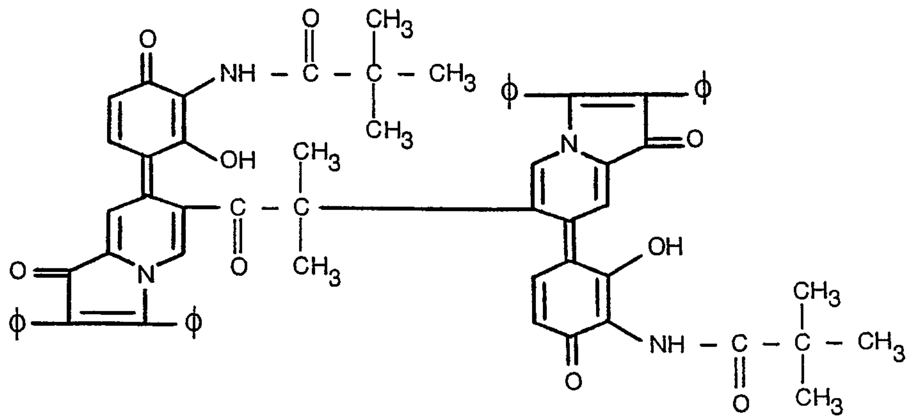

2,3-diphenyl-7-[3-(4-tert-butylbrnzamido)-2-hydroxy-4-oxo-1-phenylidene]-1(7H)-indolizinone

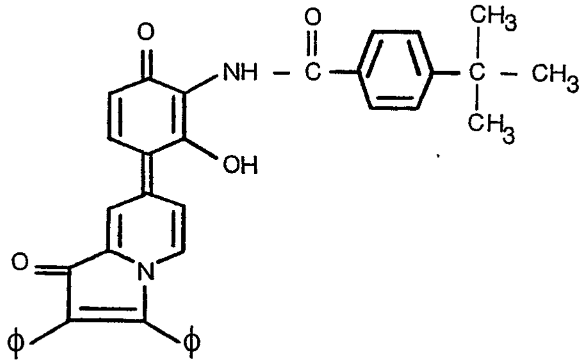

7-[3,5-di-tert-butyl-4-oxo-1-phenylidene]-1,2-di-(4-methoxyphenyl)-3(7H)-indolizinone

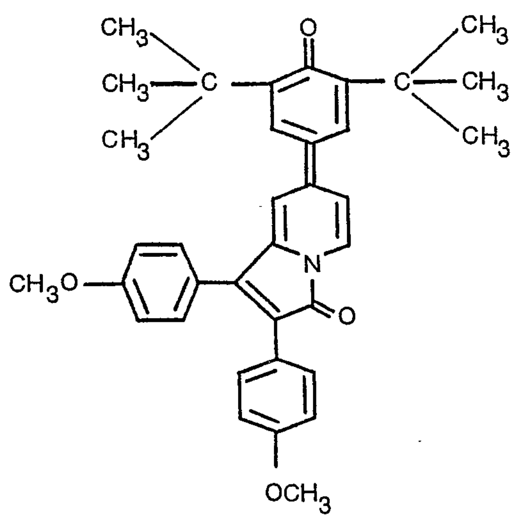

7-[3,5-di-tert-butyl-4-oxo-1-phenylidene]-2,3-di-n-propyl-1(7H)-indolizinone

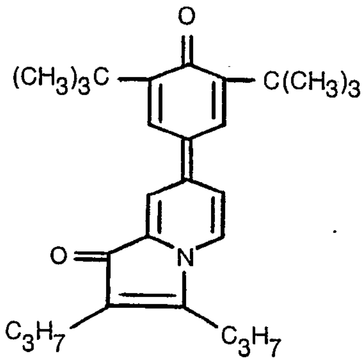

Further examples of oxoindolizine dyes prepared from phenolic couplers are listed below. Where available, λmax values, in nanometers (nm), are reported in parentheses:

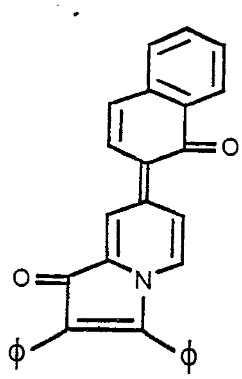

(750)

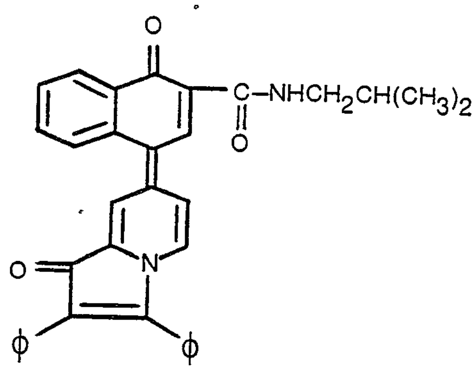

(750)

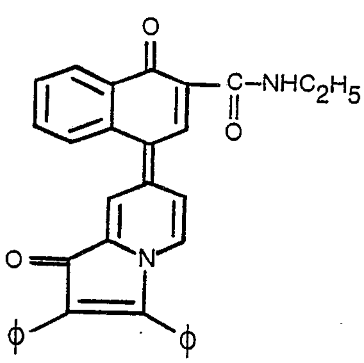

(750)

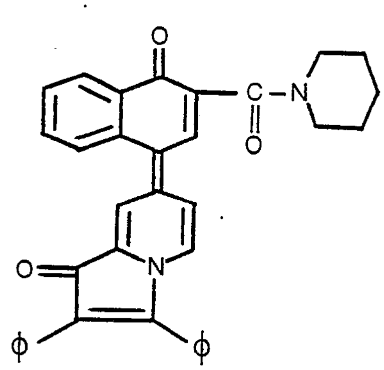

(725)

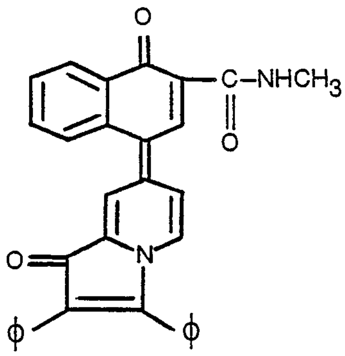

(750)

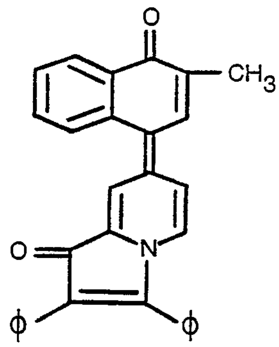

(703)

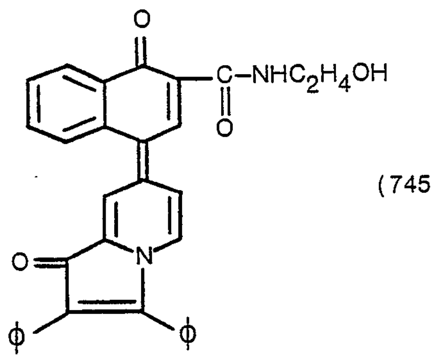

(745)

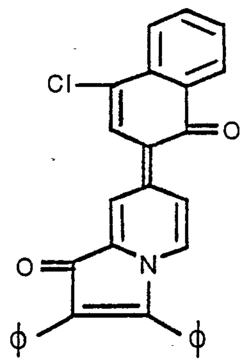

(745)

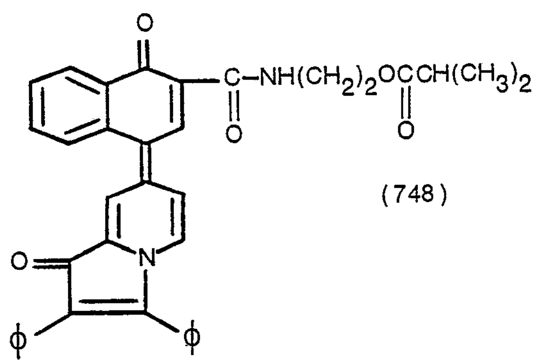

(748)

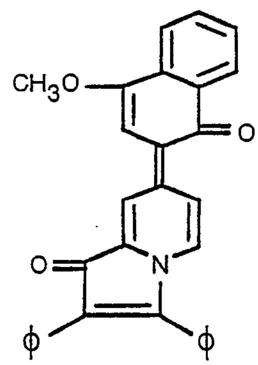

(750)

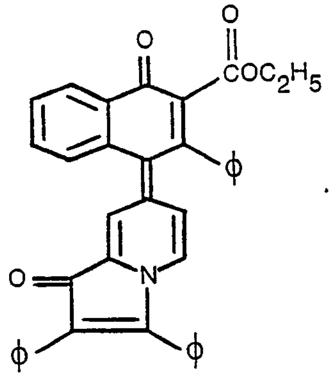

(755)

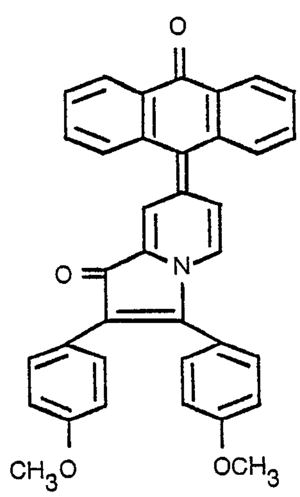

(640)

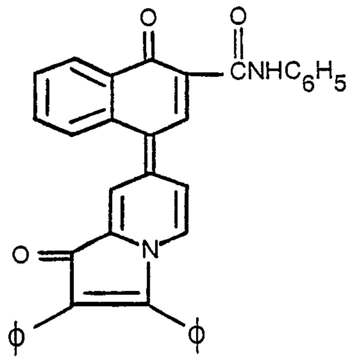

(768)

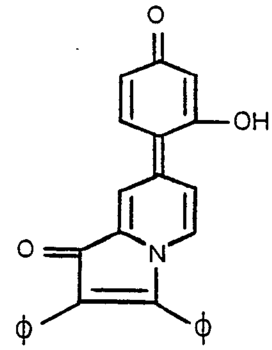

(685)

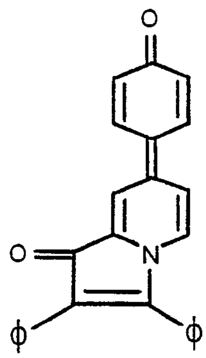

(676)

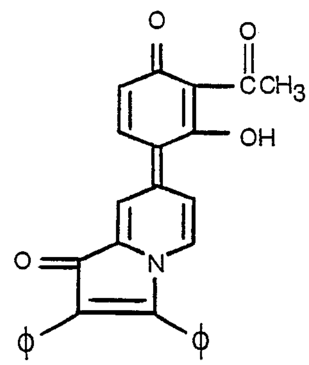

(700)

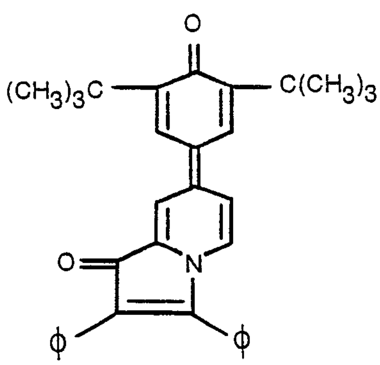

(668)

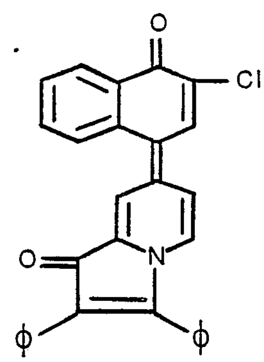

(730)

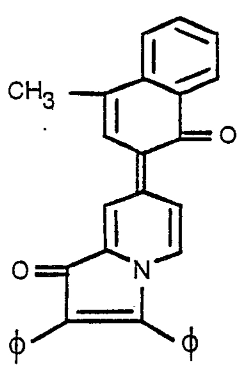

(740)

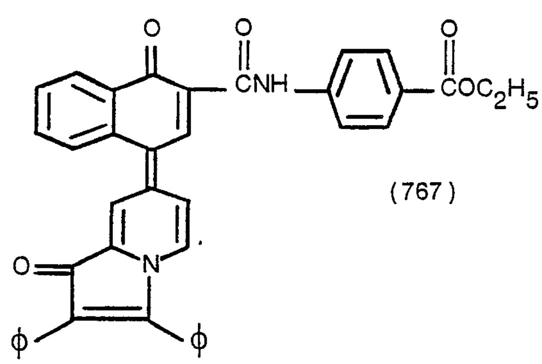

(767)

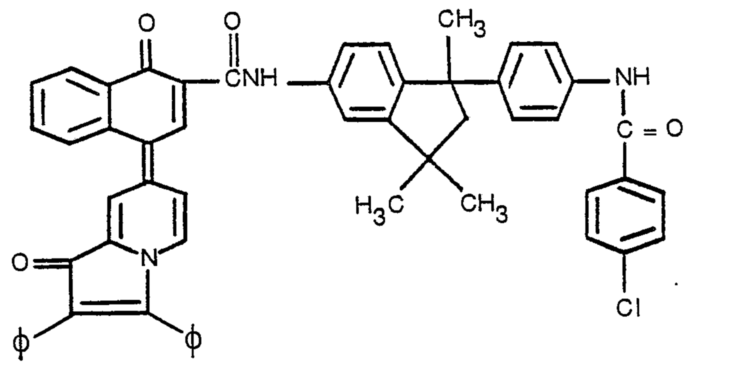

(760)

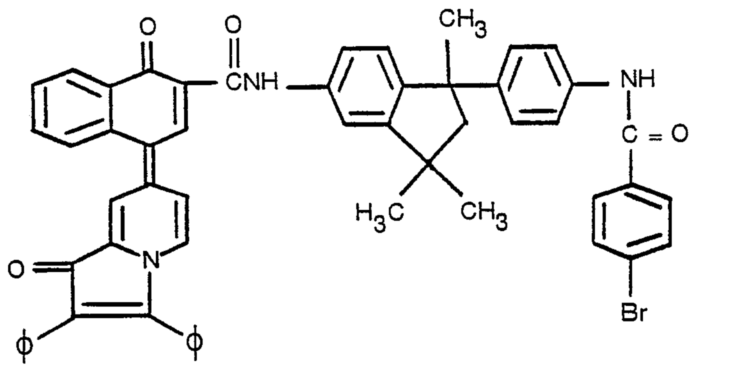

(760)

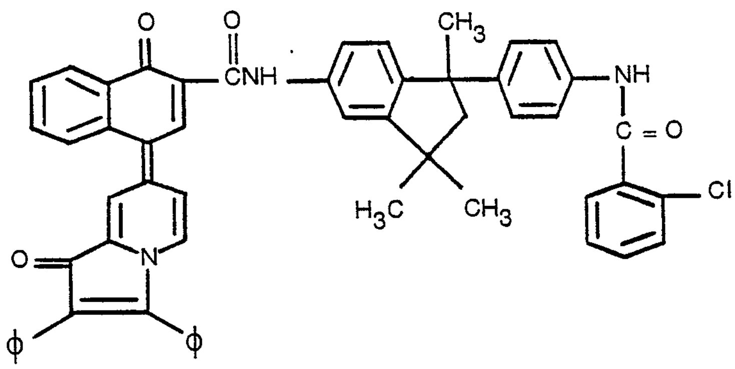

(760)

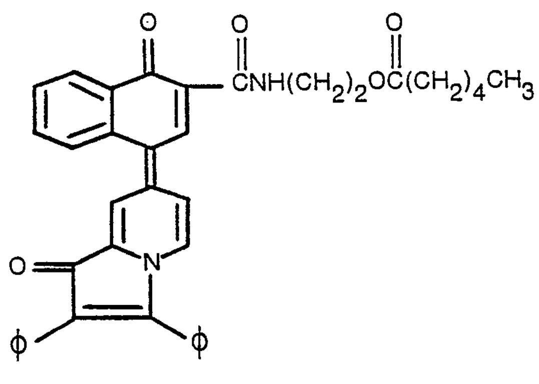

(750)

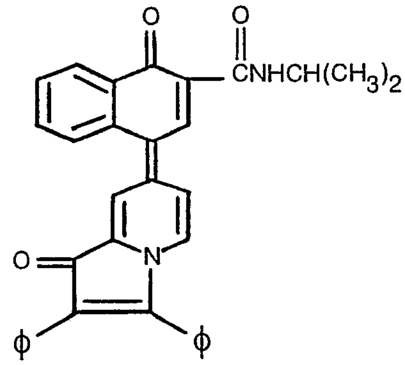

(750)

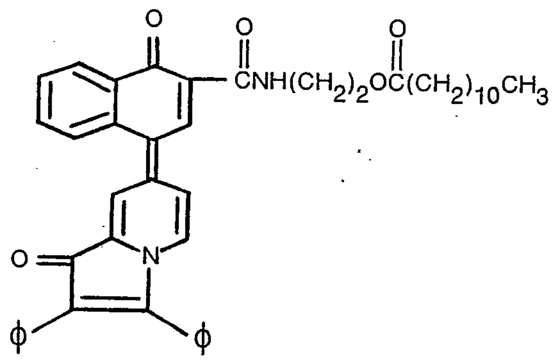

(750)

Oxoindolizinium dyes according to the invention are also formed from reaction of an aniline coupler with an oxoindolizine compound. Such dyes are represented by the structural formulae:

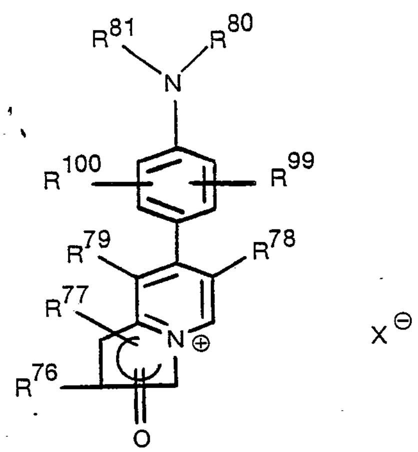

(XV)

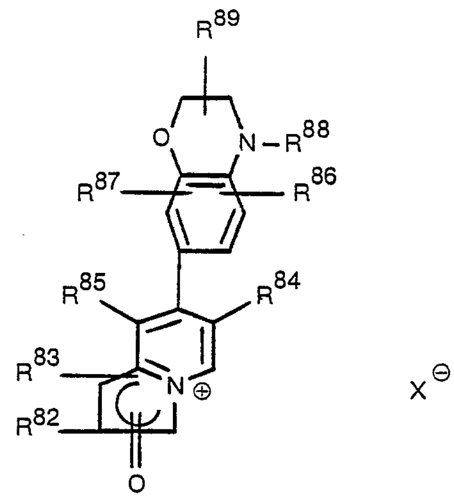

(XVI)

and

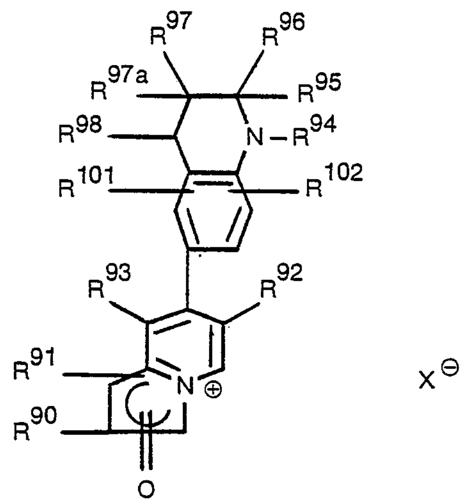

(XVII)

wherein

$R^{76}$, $R^{77}$, $R^{82}$, $R^{83}$, $R^{90}$ and $R^{91}$ are individually aryl containing 6 to 14 carbon atoms, such as phenyl, naphthyl, anthryl, methoxyphenyl and methoxynaphthyl, aralkenyl containing 6 to 14 carbon atoms, such as 2,2-diphenylvinyl, 2-phenylvinyl, 2-naphthylvinyl and 2-methyl-(2-phenylvinyl); alkyl containing 1 to 18 carbon atoms, such as methyl, ethyl, propyl, decyl and eicosyl; or $R^{76}$ and $R^{77}$, $R^{82}$ and $R^{83}$, $R^{90}$ and $R^{91}$ together represent the carbon atoms necessary to complete a 7- or 8-member cyclic structure,

$R^{78}$, $R^{84}$ and $R^{92}$ are individually hydrogen, alkyl containing 1 to 18 carbon atoms, such as methyl, ethyl, and dodecyl; cyano; acyl containing 2 to 18 carbon atoms, such as acetyl, propionyl, 2-ethylhexanoyl and stearoyl; carboalkoxy containing 2 to 18 carbon atoms such as carbomethoxy, carboethoxy and carbobutoxy; aminocarbonyl, methylaminocarbonyl, dimethylaminocarbonyl and ethylaminocarbonyl; acyloxy containing 2 to 18 carbon atoms, such as acetoxy, propionoxy, butyroxy and lauroyloxy; bromine and chlorine;

$R^{79}$, $R^{85}$ and $R^{93}$ are individually hydrogen; chlorine; bromine; or, alkyl containing 1 to 18 carbon atoms, such as methyl, ethyl, propyl and dodecyl;

$R^{80}$, $R^{81}$, $R^{88}$ and $R^{94}$ are individually hydrogen or substituents that do not adversely affect the desired indolizinium dye, such as alkyl containing 1 to 18 carbon atoms, such as methyl, ethyl, propyl, decyl, and lauryl; cycloalkyl, such as cycloalkyl containing 6 to 20 carbon atoms; straight or branched chain alkenyl containing 2 to 10 carbon atoms; or $R^{80}$ and $R^{81}$ together represent the atoms necessary to complete a 5- or 6-member heterocyclic ring with the nitrogen atom to which they are bonded, such as atoms completing a pentamethylene, ethyleneoxyethylene or ethylenesulfonylethylene group which forms a ring, or a julolidyl group;

$R^{99}$, $R^{100}$, $R^{86}$, $R^{87}$, $R^{101}$ and $R^{102}$ are individually hydrogen; fluorine; chlorine; bromine; alkyl containing 1 to 6 carbon atoms; cycloalkyl containing 5 to 12 carbon atoms; alkoxy containing 1 to 4 carbon atoms; phenoxy; alkylthio, such as alkylthio containing 1 to 4 carbon atoms; arylthio, such as arylthio containing 6 to 20 carbon atoms; and groups represented by the formula —NH—$XR^{36}$ in which X is —CO—, —COO— or —$SO_2$—, wherein $R^{36}$ is as defined above; and

$R^{89}$, $R^{95}$, $R^{96}$, $R^{97}$, $R^{97a}$ and $R^{98}$ are individually hydrogen and alkyl containing 1 to 6 carbon atoms; and

$X^{\ominus}$ is an anion as defined above, such as $CF_3SO_3^{\ominus}$, $BF_4^{\ominus}$ and $Br^{\ominus}$.

Examples of related oxoindolizinium and oxoindolizine dyes are:

7-(2-N,N-diethylamino-1-ethenyl)-2,3-di-(4-methoxyphenyl)-1-oxoindolizinium fluoborate

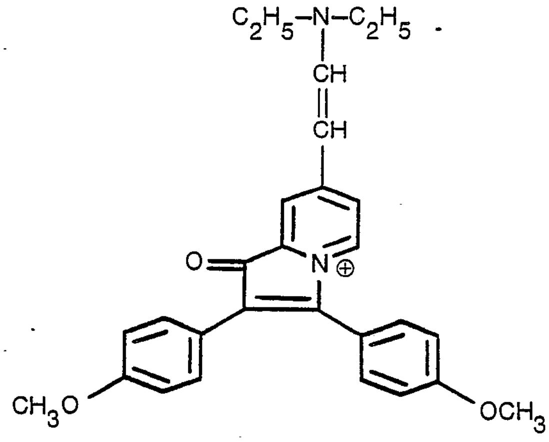

$BF_4^{\ominus}$

(610)

7-(2-N,N-diethylamino-1-ethenyl)-1,2-diphenyl-3-oxoindolizinium iodide

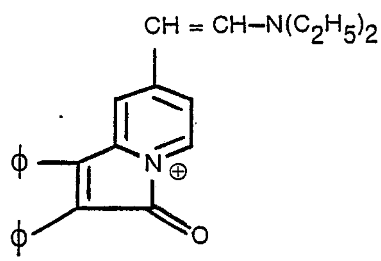

$I^{\ominus}$ , and

2,3-di-(4-methoxyphenyl)-7-dimethylamino-1-oxoindolizinium iodide

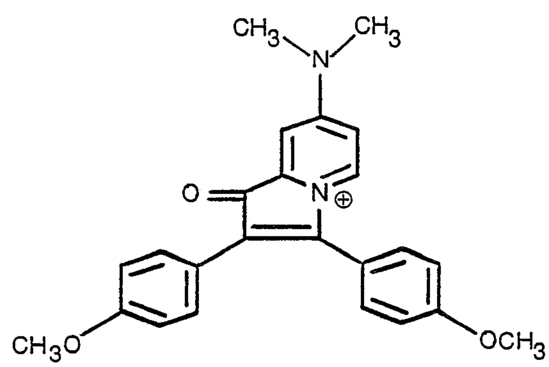

$I^{\ominus}$

Further examples of oxoindolizinium dyes are listed below. Where available, λmax values, in nanometers (nm), are reported in parentheses. In instances where two λmax values are reported, both value intensities are approximately equal.

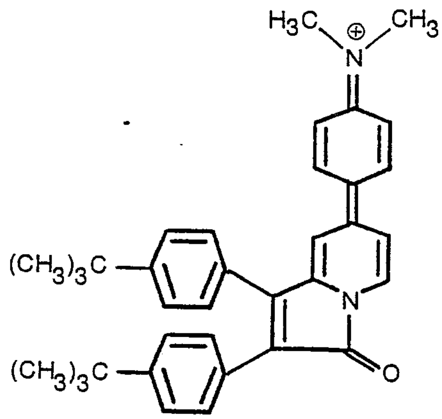

(755)

$CF_3SO_3^{\ominus}$

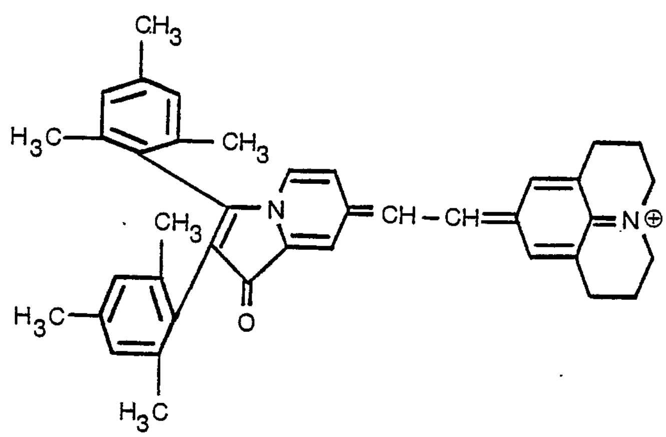

(840)

$CF_3SO_3^{\ominus}$

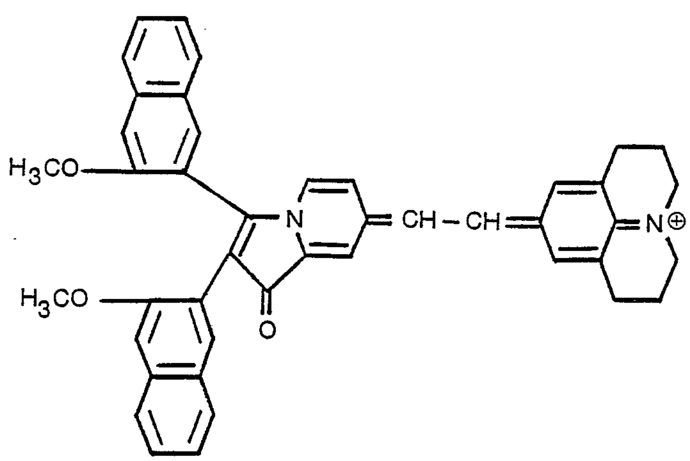

(830)

$CF_3SO_3^{\ominus}$

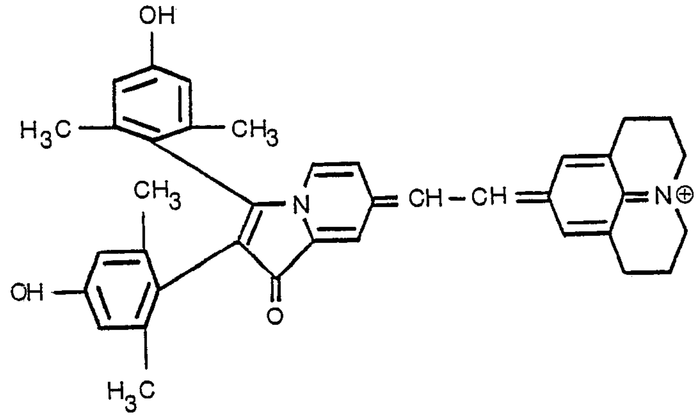

(835)

$CF_3SO_3^{\ominus}$

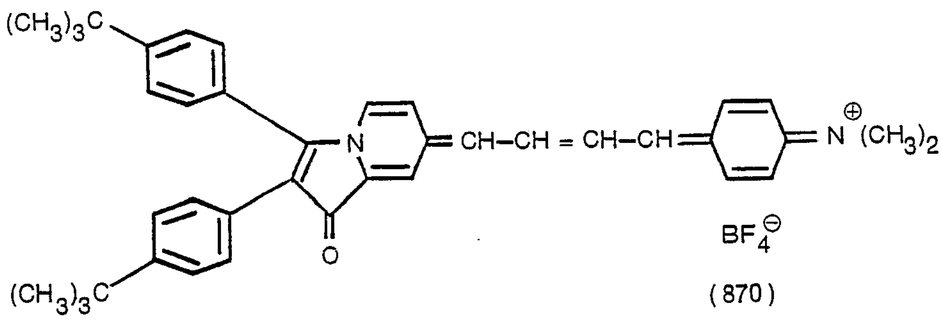

$BF_4^{\ominus}$

(870)

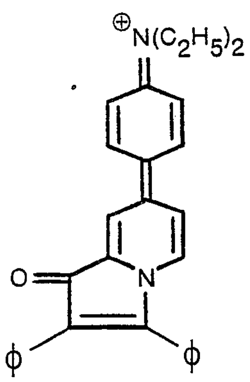

$I^{\ominus}$

(680)

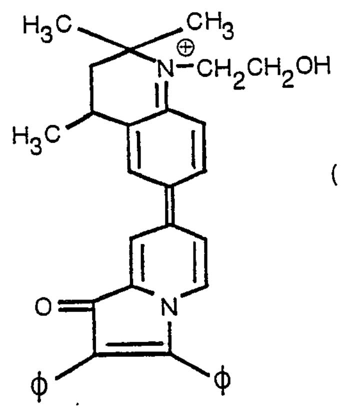

$I^{\ominus}$

(750)

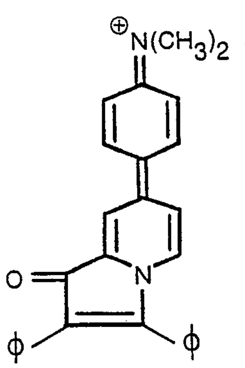

$I^{\ominus}$

(680)

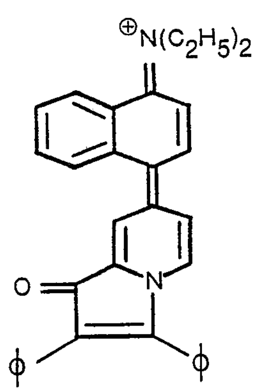

$I^{\ominus}$

(660)

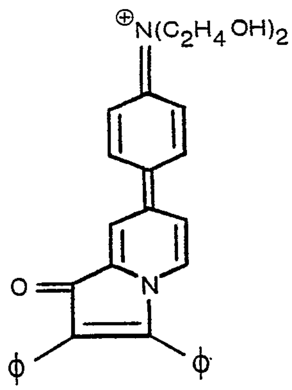

$I^{\ominus}$

(735)

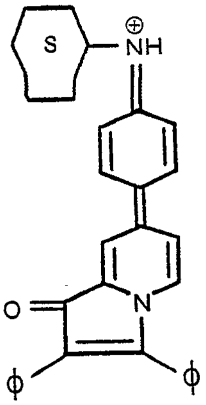

$I^{\ominus}$

(720)

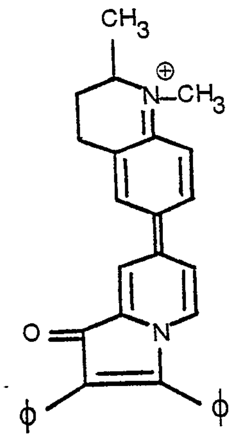

$I^{\ominus}$

(710)

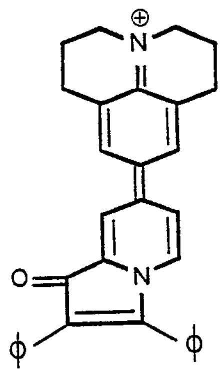

$I^{\ominus}$

(730, 790)

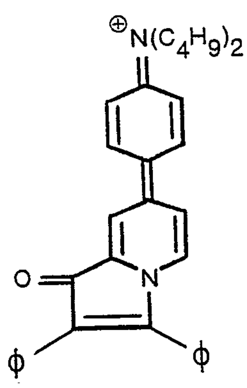

$I^{\ominus}$

(700)

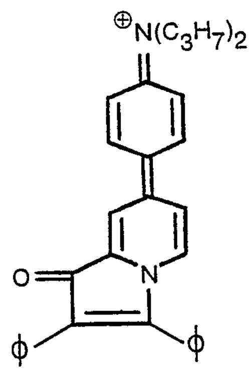

$I^{\ominus}$

(690)

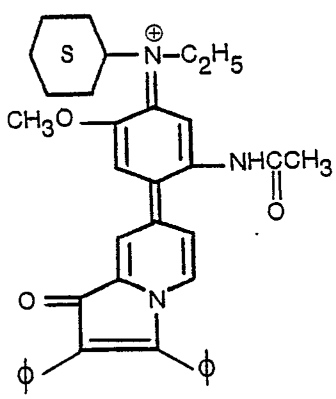

$I^{\ominus}$

(640)

Many useful oxoindolizine dyes according to the invention are formed from the reaction of an active methylene coupler with a suitable oxoindolizine compound. Especially useful oxoindolizines are dyes formed from the reaction of ketomethylene couplers, methylpyrylium couplers and methylindolizinium couplers with appropriate oxoindolizine compounds. Examples of useful indolizinone dyes formed from active methylene couplers are represented by the formula:

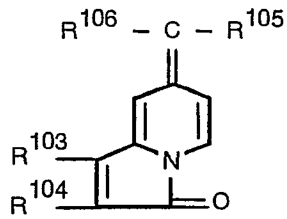

(XVIII)

and

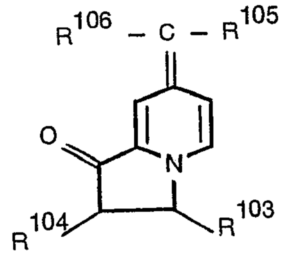

(XVIIIA)

wherein:

$R^{103}$ and $R^{104}$ are individually aryl containing 6 to 20 carbon atoms, such as phenyl, naphthyl, anthryl, methoxyphenyl and methoxynaphthyl; aralkenyl containing 6 to 20 carbon atoms, such as 2,2-diphenylvinyl, 2-phenylvinyl, 2-naphthylvinyl and 2-methyl-(2-phenylvinyl); alkyl containing 1 to 18 carbon atoms, such as methyl, ethyl, propyl, decyl and lauryl; or $R^{103}$ and $R^{104}$ together represent the carbon atoms necessary to complete a 7- or 8-member cyclic structure;

$R^{105}$ and $R^{106}$ are individually electronegative groups, such as aryl containing 6 to 20 carbon atoms, such as phenyl and naphthyl; cyano; acyl containing 2 to 18 carbon atoms, such as acetyl, propionyl and butyryl; carboalkoxy containing 2 to 18 carbon atoms, such as carbomethoxy, carboamyloxy and carbobutoxy; aminocarbonyl containing 2 to 18 carbon atoms such as unsubstituted aminocarbonyl, methylaminocarbonyl, dimethylaminocarbonyl and ethylaminocarbonyl; and $R^{105}$ is alternatively hydrogen.

Examples of oxoindolizine dyes formed from active methylene couplers are shown below. Where available, λmax values, in nanometers (nm), are reported in parentheses:

7-(diacetylmethylidene)-1,2-diphenyl-3-(7H)-indolizinone

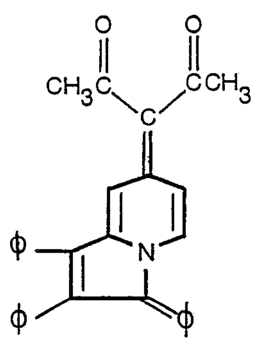

(410, 480)

7-(dibenzoylmethylidene)-2,3-diphenyl-1-(7H)-indolizinone

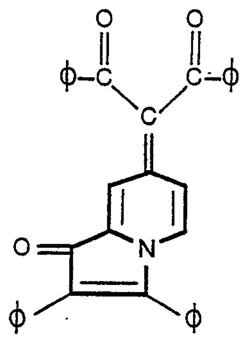

(610)

7-(anilinocarbonyl benzoylmethylidene)-2,3-diphenyl-1(7H)-indolizinone

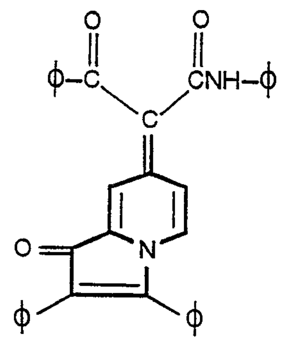

6-cyano-7-(diacetylmethylidene)-2,3-diphenyl)-1(7H)-indolizinone

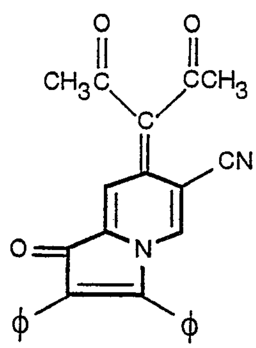

(530)

7-(dicyanomethylidene)-2,3-diphenyl-
1(7H)-indolizinone

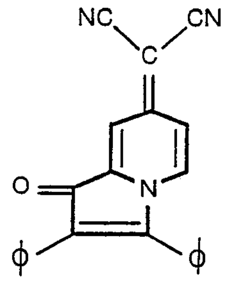

(555, 590)

7-(1-cyano-1-phenylmethylidene)-1,2-diphenyl-
3(7H)-indolizinone

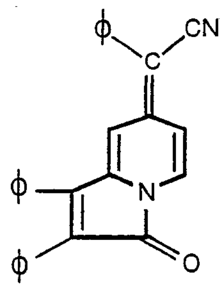

7-(1-aminocarbonyl-1-phenylmethylidene)-2,3-diphenyl-
1(7H)-indolizinone

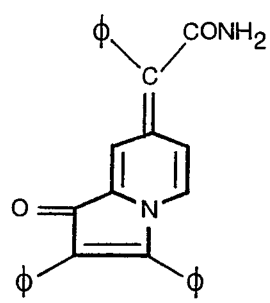

7-(dicarboethoxymethylidene)-2,3-diphenyl-
1(7H)-indolizinone

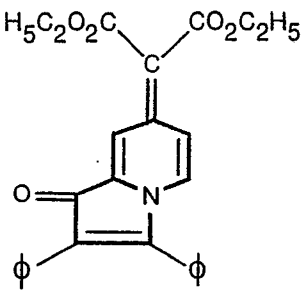

2,3-diphenyl-7-(2,2-dimethyl-4,6-dioxo-1,3-dioxanylidene)-
1(7H)-indolizinone

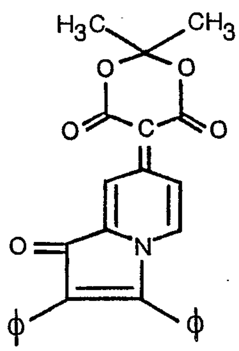

(560, 580)

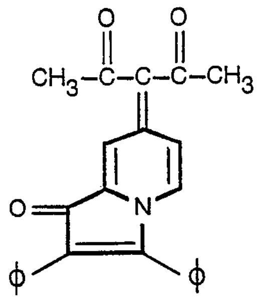

(599)

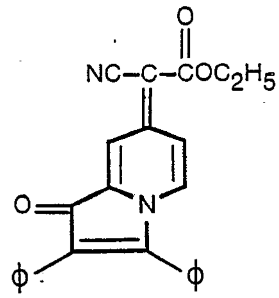

(553, 590)

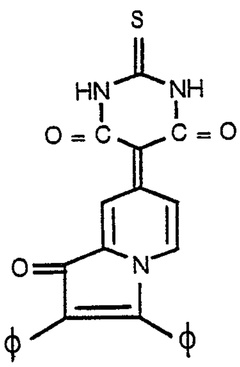

(580)

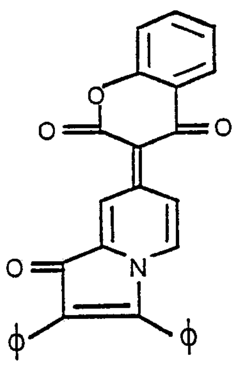

(590)

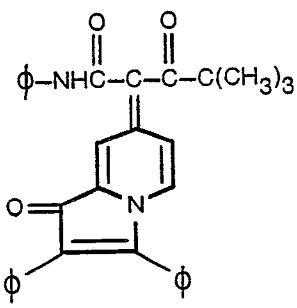

(570)

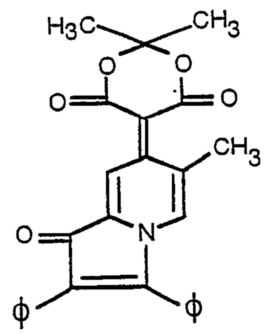

(600)

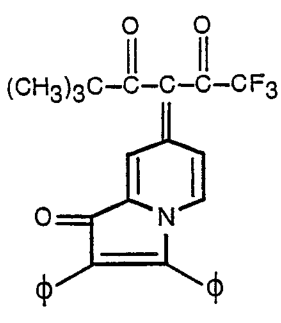

(570, 603)

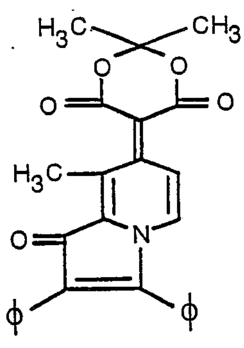

(600)

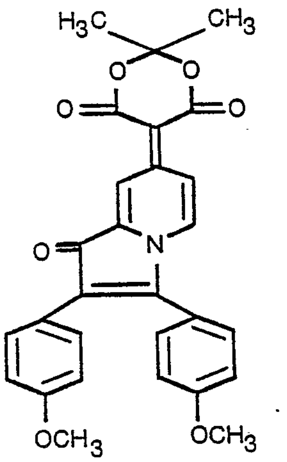

(570)

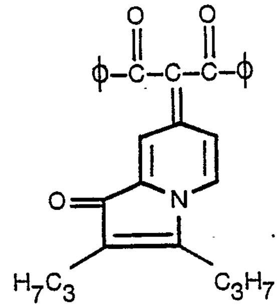

(553, 590)

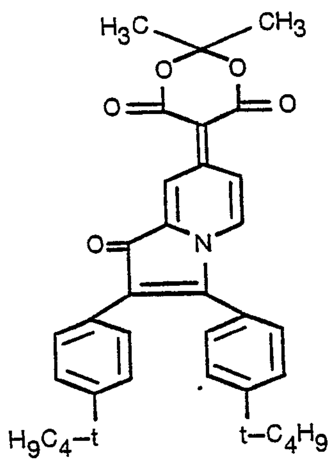

(563)

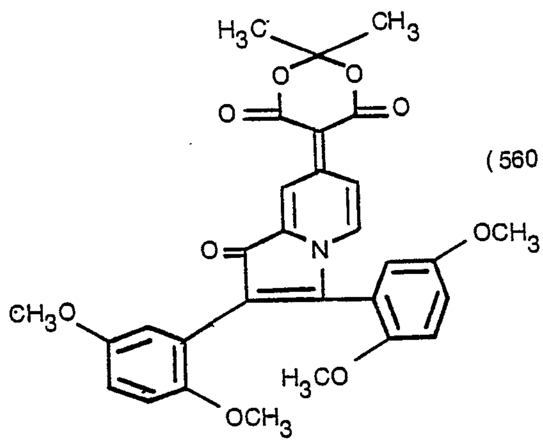

(560)

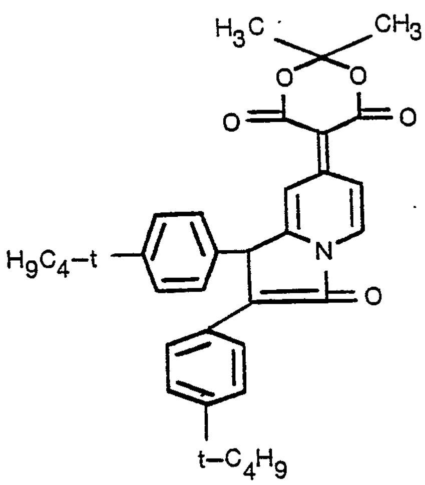

(460)

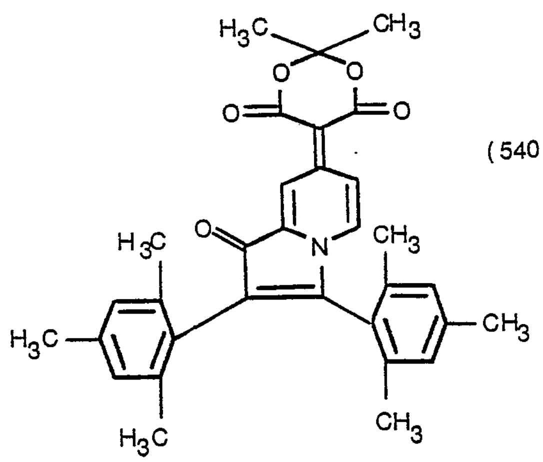

(540)

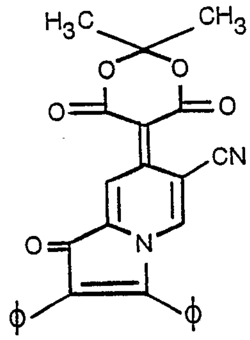

(540)

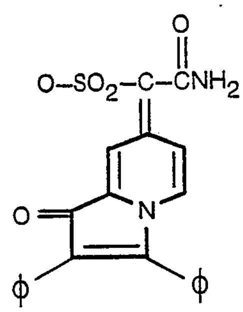

(570)

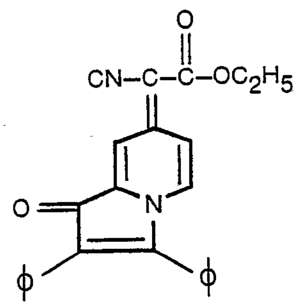

(560)

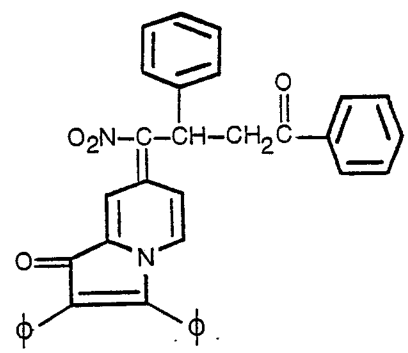

(600)

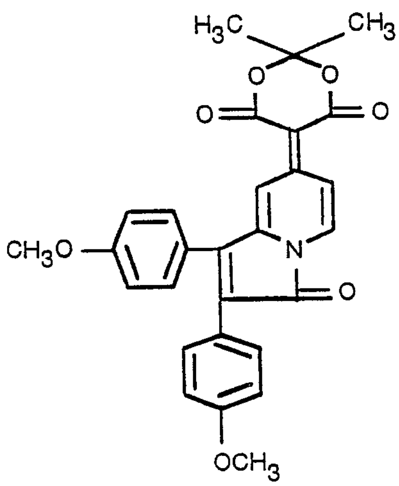

(440)

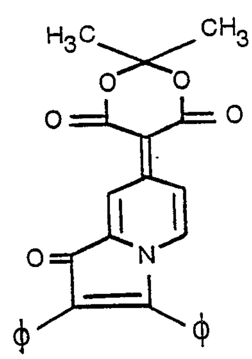

(615)

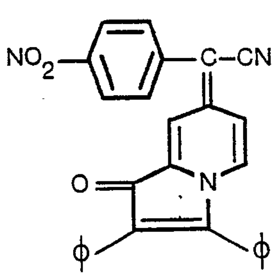

(640)

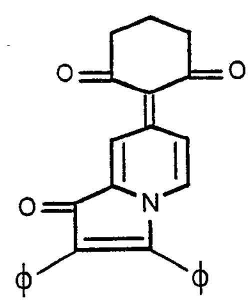

(615)

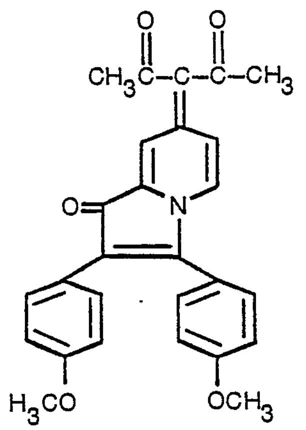

(600)

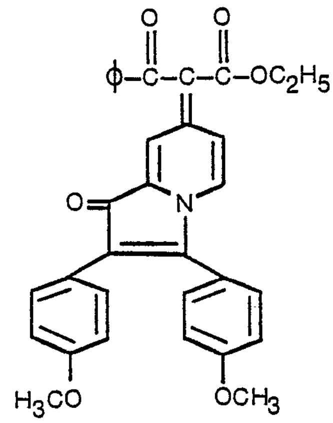

(590)

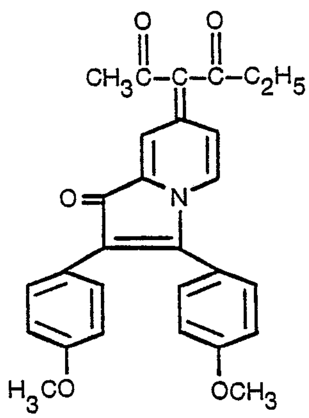

(590)

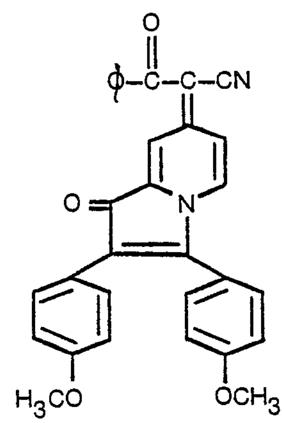

(600)

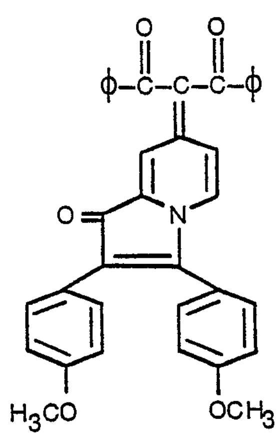

(610)

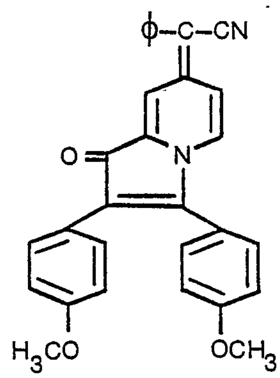

(610)

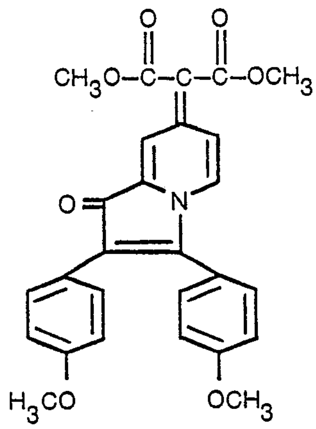

(580)

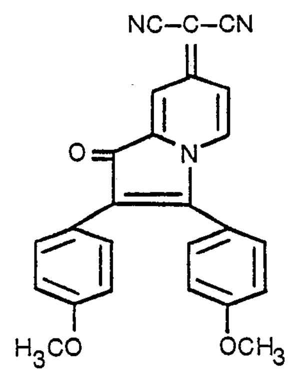

(585)

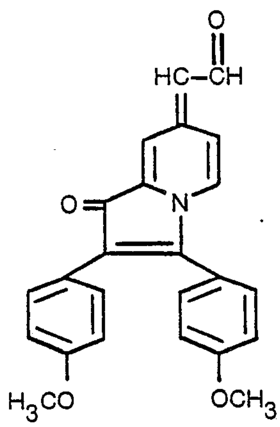

(595)

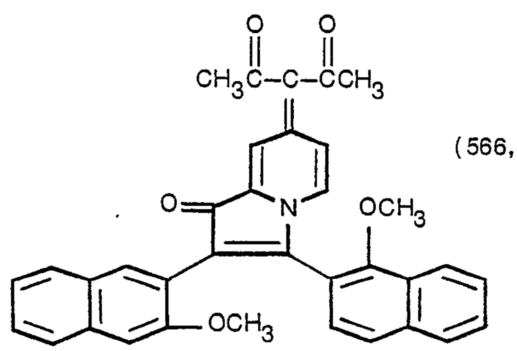

(566, 600)

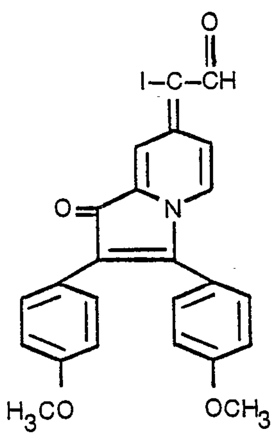

(610)

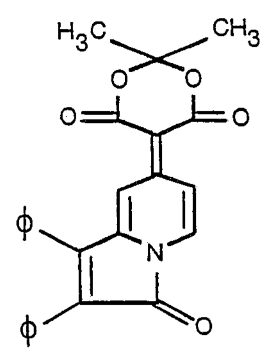

(445)

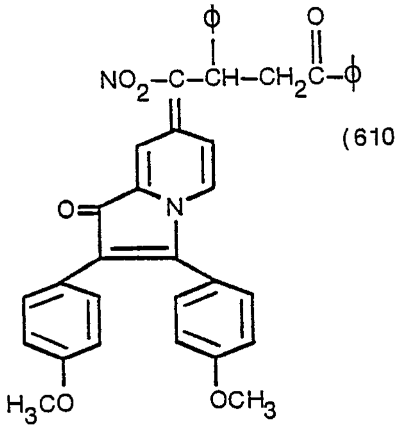

(610)

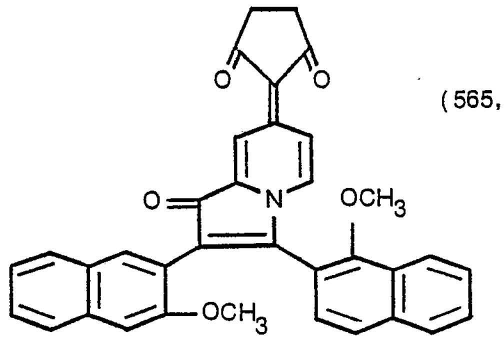

(565, 605)

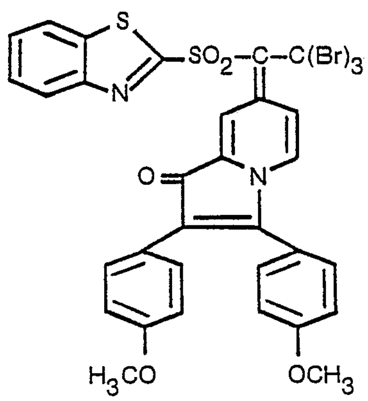

(605)

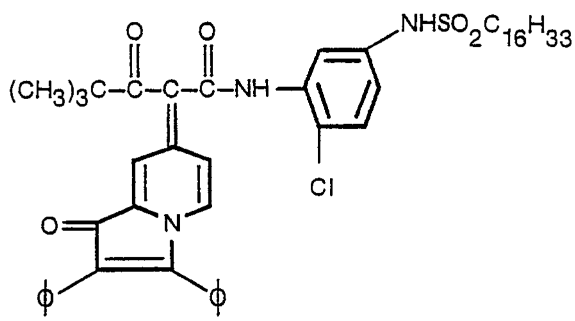

(580)

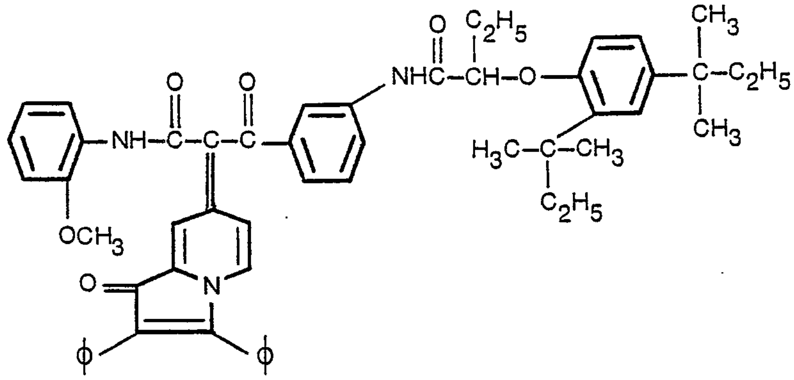

(610)

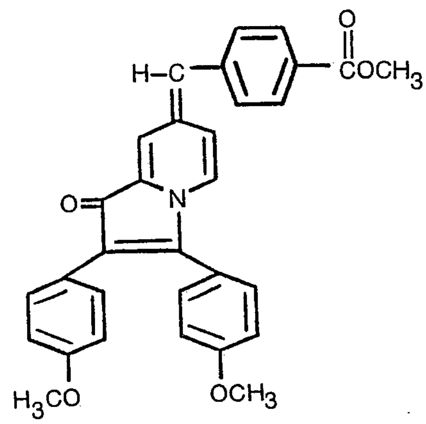

(615)

Examples of oxoindolizinium dyes formed from active methylene couplers are represented by the formula:

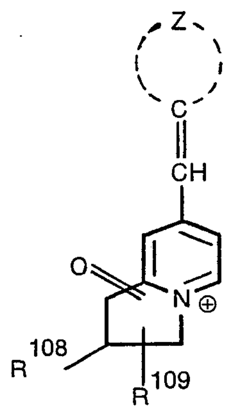

$X^{\ominus}$

(XIX)

wherein

$X^{\ominus}$ is an anion as defined above;

$R^{108}$ and $R^{109}$ are individually the same as $R^{103}$ and $R^{104}$; and

Z represents the atoms necessary to complete a chromophore, such as the carbon, hydrogen, oxygen and nitrogen atoms necessary to complete a heterocyclic group, such as a pyranylidene, indolizinylidene, thiopyranylidene, selenopyranylidene, coumarinylidene, or pyrazolinonylidene group.

Examples of oxoindolizinium dyes formed from such active methylene couplers are as follows:

2,3-diphenyl-7-[(2,6-diphenyl-4-pyranylidene)methyl]-1-oxoindolizinium perchlorate

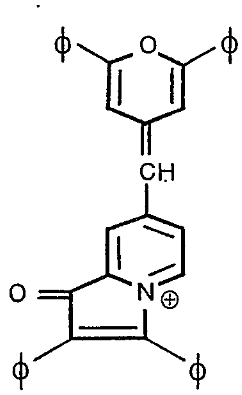

$ClO_4^{\ominus}$

(695)

2,3-diphenyl-7-[(2,3-diphenyl-7-1(7H)-indolizinonylidene)methyl]-
1-indolizinonium trifluoromethane sulfonate

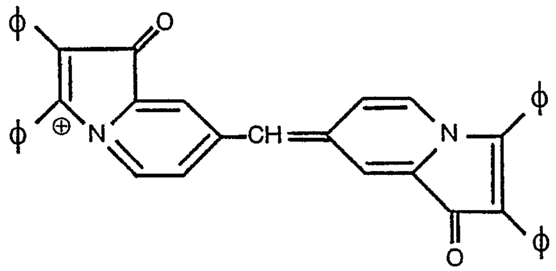

$CF_3SO_3^{\ominus}$

(780)

2,3-diphenyl-7-[(2,6-diphenyl-4-thiopyranylidene)methyl]-
1-indolizinonium trifluoromethane sulfonate

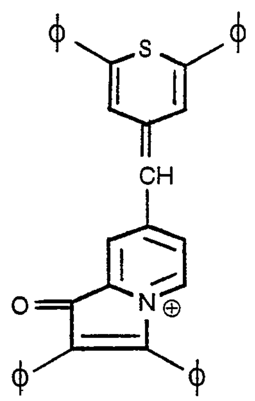

$CF_3SO_3^{\ominus}$

(730)

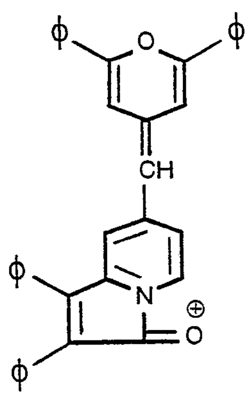

$BF_4^{\ominus}$

(640)

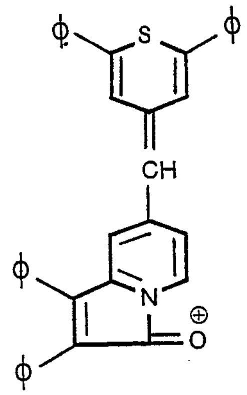

$ClO_4^{\ominus}$

(675)

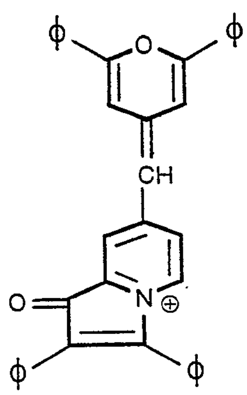

$BF_4^{\ominus}$

(690)

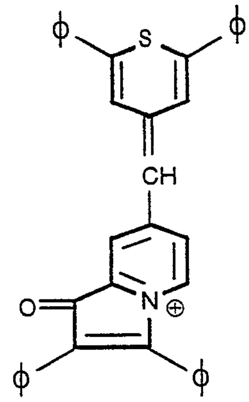

$ClO_4^{\ominus}$

(725)

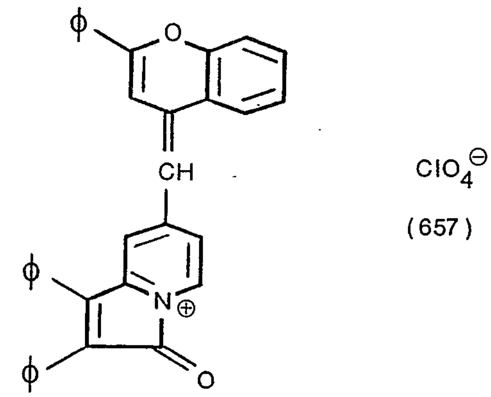

(657)

Another class of oxoindolizine dyes according to the invention is represented by the formula:

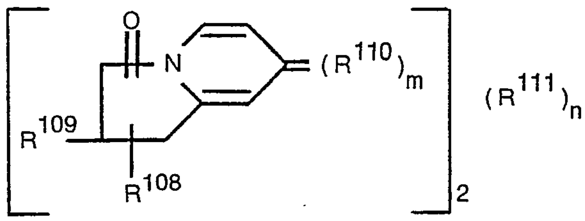

(XX)

wherein:

$R^{108}$ and $R^{109}$ are individually aryl containing 6 to 14 carbon atoms; or, alkyl containing 1 to 20 carbon atoms;

$R^{110}$ is CH, phenylene or naphthylene;

$R^{111}$ is phenylene or naphthylene; and

n and m are individually 0 or 1.

Examples of aryl groups which are suitable for use as $R^{108}$ or $R^{109}$ substituents include unsubstituted or substituted phenyl, naphthyl and anthryl.

Examples of alkyl groups which are suitable for use as $R^{108}$ or $R^{109}$ substituents include methyl, ethyl, propyl, t-butyl, decyl, lauryl and eicosyl.

In oxoindolizine dyes according to the formula containing $R^{110}$ and $R^{108}$, the oxoindolizine moiety represents a group completing an organic chromophore to produce the desired dye. Examples of such compounds are:

1,2-bis[7-(1,2-diphenyl-3(7H)-indolizinonylidene)] ethane

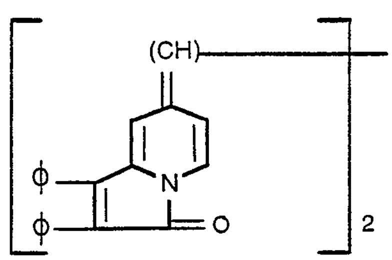

(685)

1,4-bis[7-(1,2-diphenyl-3(7H)-indolizinonylidene]-2,5-cyclohexadiene

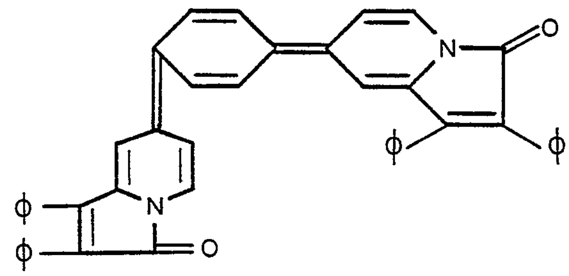

1,4-bis-[7-(1,2-diphenyl-3(7H)-indolizinonylidene)]-2,3-benzo-2,5-cyclohexadiene

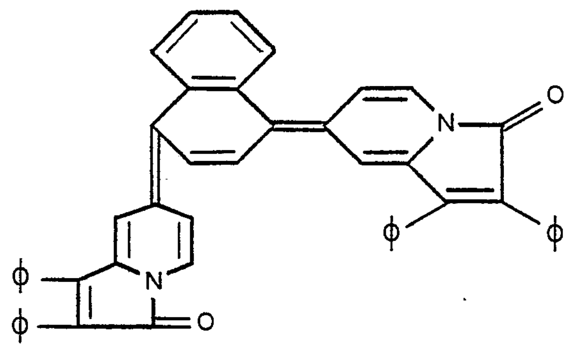

7,7′-bis[1,2-di-n-propyl-3(7H)-indolizonylidene]

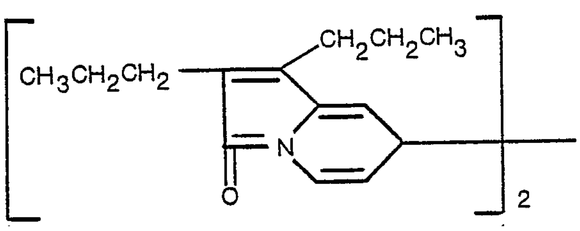

7,7′-bis-[1,2-pentamethylene-3(7H)-indolizonylidene]

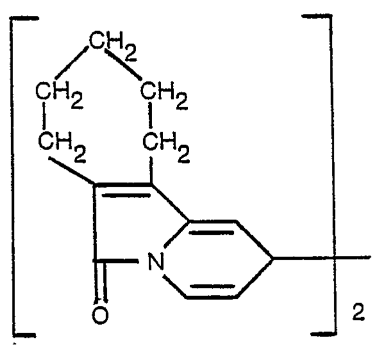

1,2-bis-[2,3-di-(4-methoxyphenyl)-1(7H)-indolizinonylidene] ethane

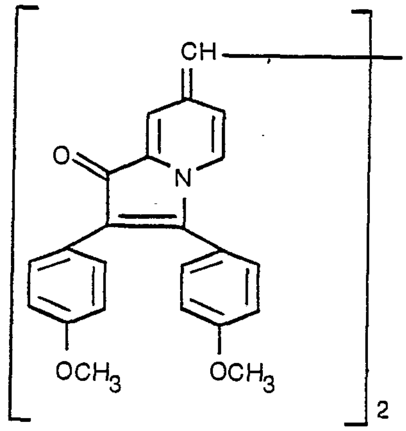

(790)

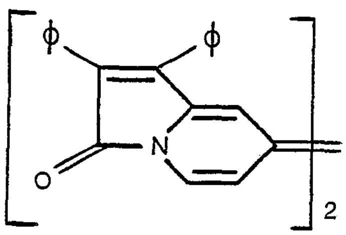

(690)

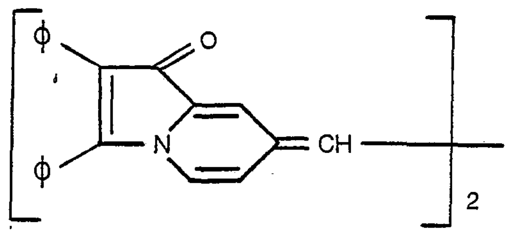

(780)

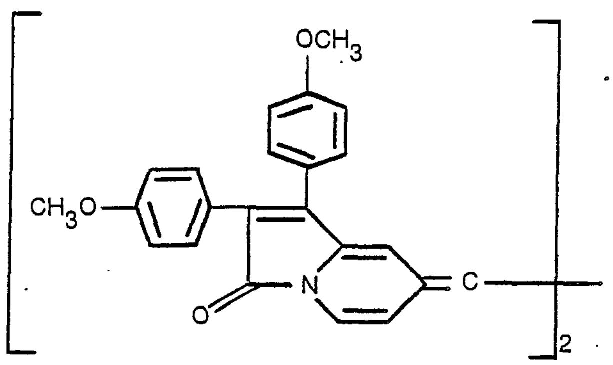

(684)

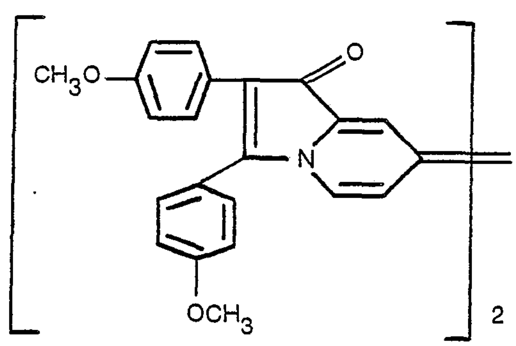

(805)

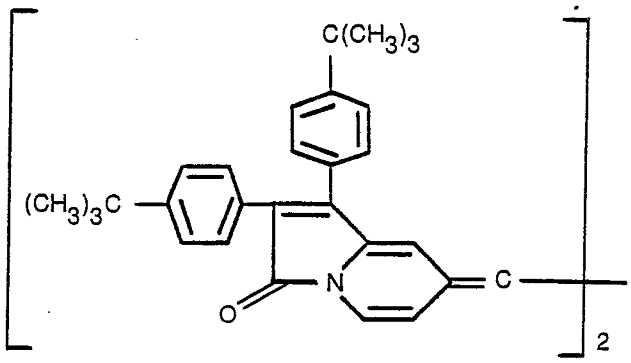

(688)

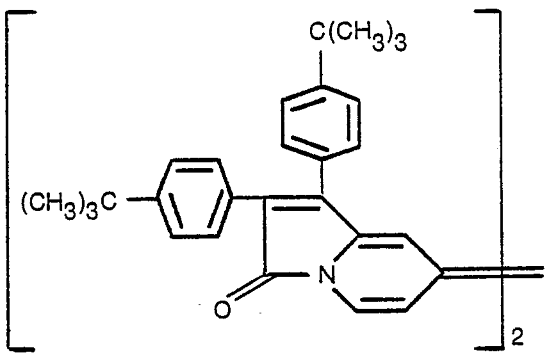

(685)

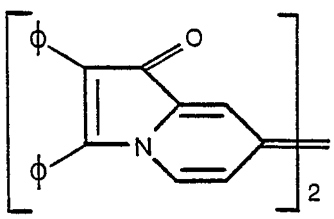

(800)

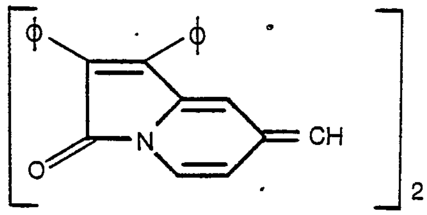

(740)

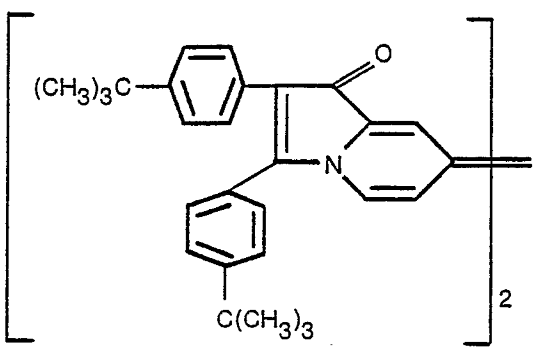

(803)

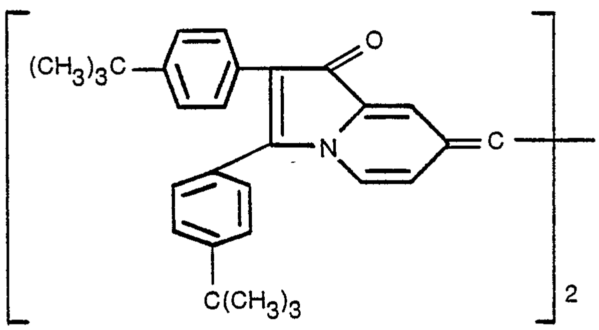

(790)

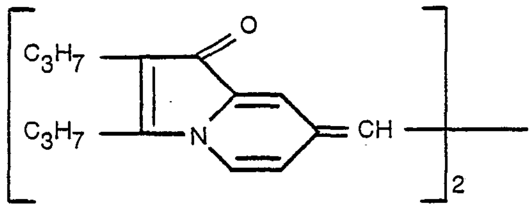

(725)

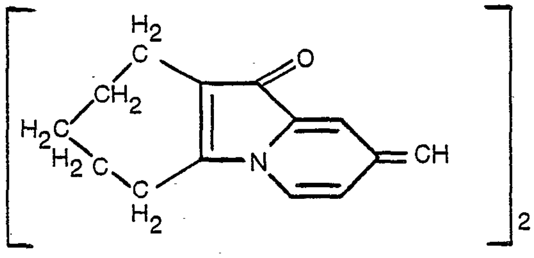

(725)

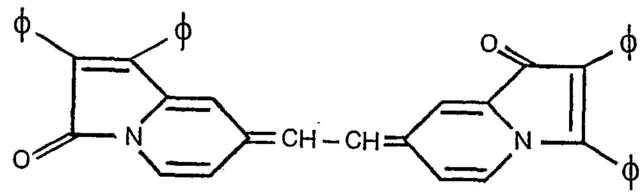

(740)

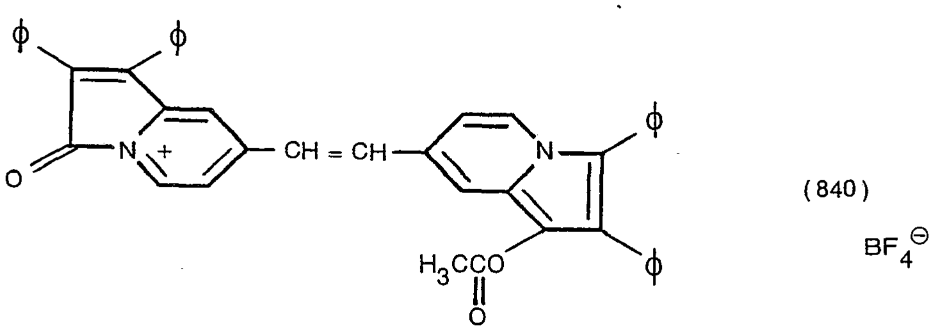

(840)

Examples of other dyes within the above structures (I) and (II) are as follows:

N-benzyl-4-{7-[2,3-di(4-methoxyphenyl)-3-indolizinolyl]
pyridinium bromide

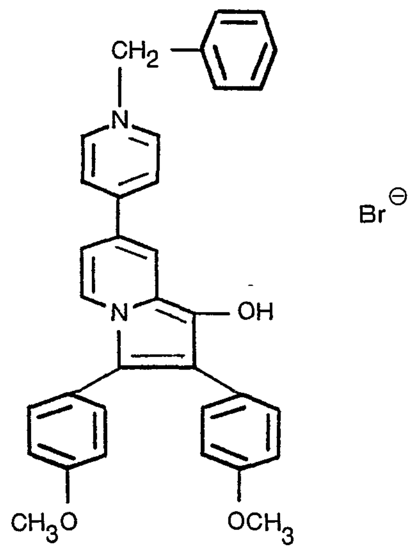

(590)

7-[4-(N-benzylpyridylidene)]-2,3-diphenyl-1-hydroxy
indolizinium chloride

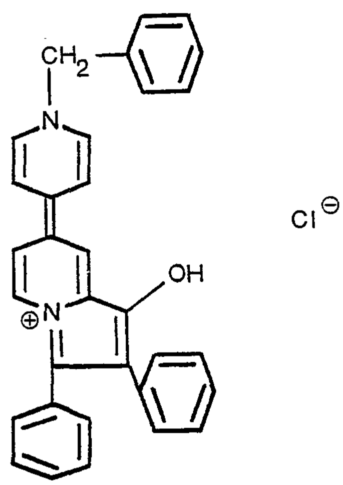

7-[4-(N-benzylpyridylidene)]-2,3-diphenyl-1-indolizinone

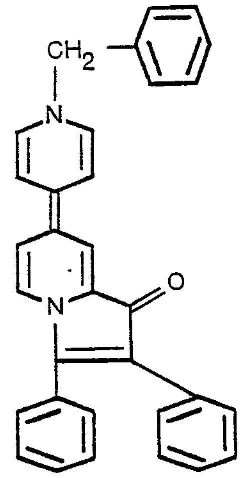

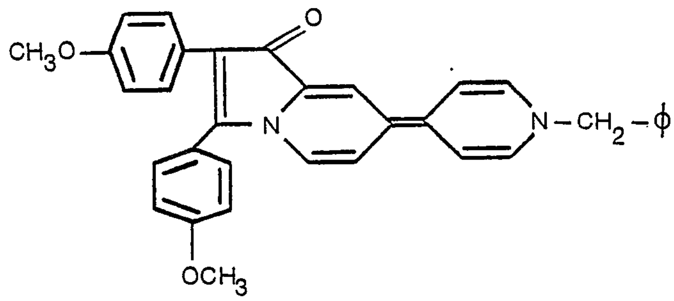

(590)

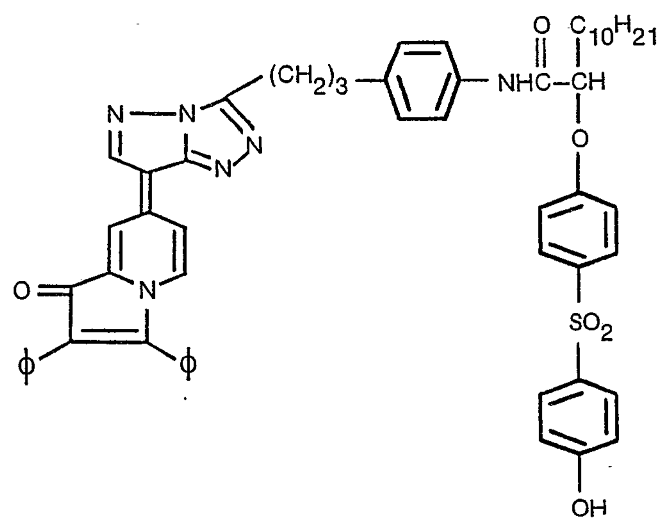

(630, 690)

Another class of dyes according to the invention is represented by the formula:

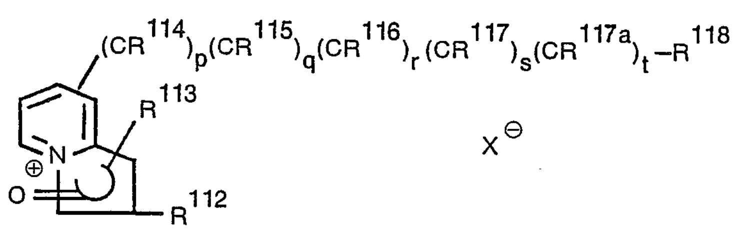

(XXI)

wherein

$X^{\ominus}$ is an anion as defined above, preferably an acid anion such as methanesulfonate, trifluoromethanesulfonate, para-toluenesulfonate $BF_4^{\ominus}$ bromide, chloride, iodide and sulfinate;

$R^{112}$ and $R^{113}$ are individually aryl containing 6 to 20 carbon atoms; aralkenyl containing 6 to 20 carbon atoms, and alkyl containing 1 to 20 carbon atoms; or $R^{112}$ and $R^{113}$ together represent the carbon atoms necessary to complete a 7- or 8-member cyclic structure;

$R^{114}$, $R^{115}$, $R^{116}$, $R^{117}$ and $R^{117a}$ are individually hydrogen; alkyl containing 1 to 18 carbon atoms; phenyl; cyano; carboxy; carboxamide; and carboalkoxy, containing 2 to 18 carbon atoms; at least one of $R^{114}$, $R^{115}$, $R^{116}$, $R^{117}$ and $R^{117a}$ is hydrogen;

$R^{118}$ is an electropositive or an electronegative group necessary to complete a chromophore, such as amino, anilino, nitrophenyl, quino, pyranyl, pyridyl, indolizinyl, julolidyl and thiopyranyl;

p, q, r, s and t are individually 0 or 1; any free bonds being satisfied by hydrogen or unsaturated bonding as required.

Aryl groups which are suitable for use as $R^{112}$ and $R^{113}$ substituents include unsubstituted or substituted phenyl, naphthyl, anthryl, methoxyphenyl and methoxynaphthyl.

Examples of aralkenyl groups which are suitable for use as $R^{112}$ and $R^{113}$ substituents include 2,2-diphenylvinyl, 2-phenylvinyl, 2-naphthylvinyl and 2-methyl-(2-phenylvinyl).

Alkyl groups which are suitable for use as $R^{112}$ to and including $R^{117a}$ include methyl, ethyl, propyl, t-butyl, decyl and lauryl.

Examples of compounds within this class are as follows:

7-[2-(4-N,N-dimethylaminophenyl-1-ethenyl] -2,3-diphenyl-1-indolizinonium fluoroborate

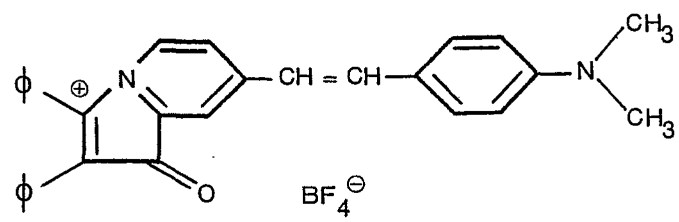

(780)

2,3-diphenyl-7-[2-(9-julolidyl)-1-ethenyl]-1-indolizinonium trifluoromethane sulfonate

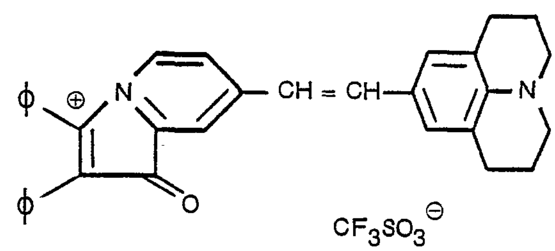

(837)

2,3-diphenyl-7-[3-(2,3-diphenyl-4(4H)-pyranylidene-1-propenyl]-1-indolizinonium perchlorate

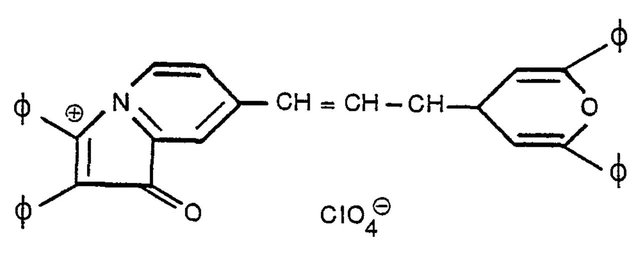

(840)

7-[2-(4-N,N-dimethylaminonaphthyl)-1-ethenyl]-2,3-diphenyl-1-indolizinonium fluoroborate

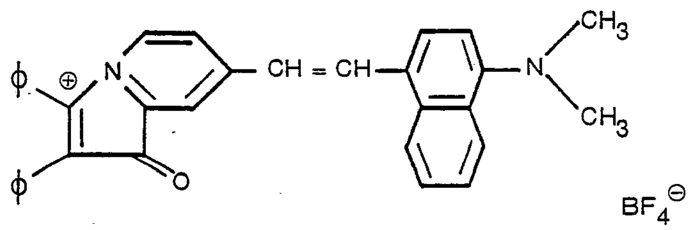

7-[4-(4-dimethylaminophenyl)-1-butadienyl]-1,2-diphenyl-3-indolizinonium trifluoromethane sulfonate

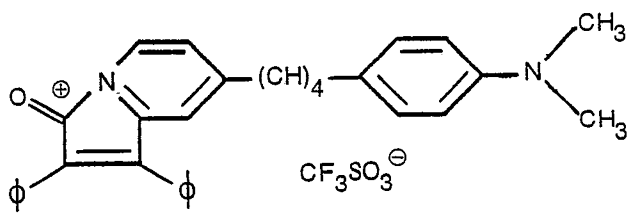

(885)

1-(3,5-di-tert-butyl-4-oxo-1-phenylidene)-2-[7-(2,3-diphenyl-1-(7H)-indolizinonylidene)]ethane

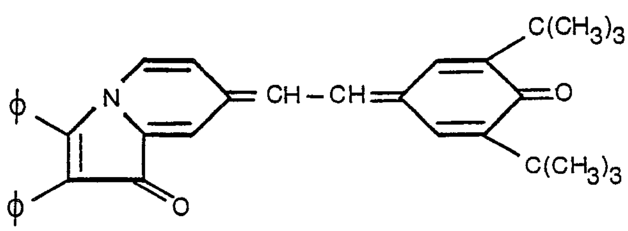

(690)

2,3-diphenyl-7-[2-(4-nitrophenyl)-1-ethenyl]-1-indolizinol sodium salt

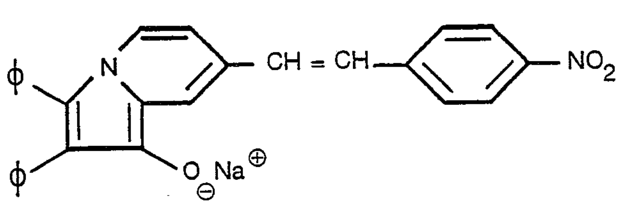

2,3-diphenyl-7-[2-(2,6-diphenyl-4-(4H)-pyranylidene)-1-ethylidene]-
1-hydroxy-(7H)-indolizinium perchlorate

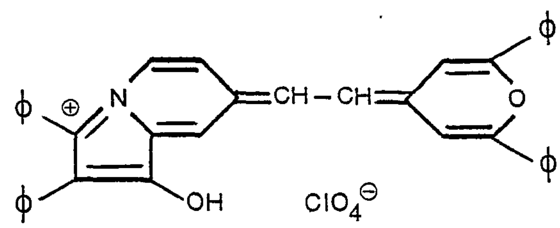

2,3-diphenyl-7-[2-(2,6-diphenyl-4-(4H)-pyranylidene)-1-ethylidene]-
1-acetoxy-(7H)-indolizinium perchlorate

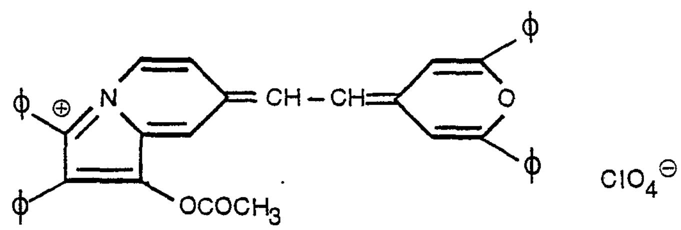

(710)

7-(2,2-diacetyl-1-ethenyl)-2,3-diphenyl-1-indolizinol
sodium salt

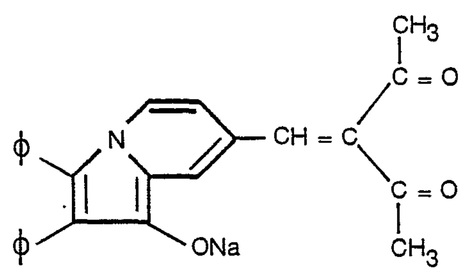

2,3-diphenyl-6-[2-(4-nitrophenyl)-1-ethenyl]-
1-indolizinol

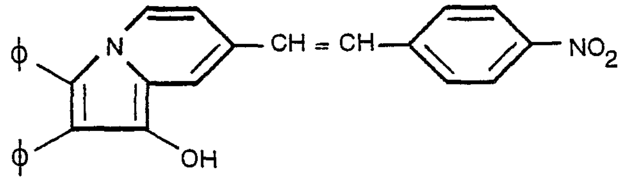

1-[7-(2,3-diphenyl-1-(7H)-indolizinonylidene)]-2-[4-(2,6-diphenyl-4(4H)-pyranylidene)]-ethane

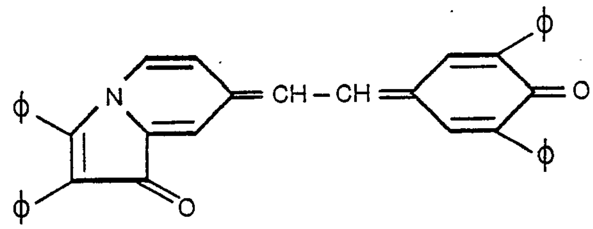

7-(3,3-diacetyl-1-propenylidene)-2,3-diphenyl-1-(7H)-indolizinone

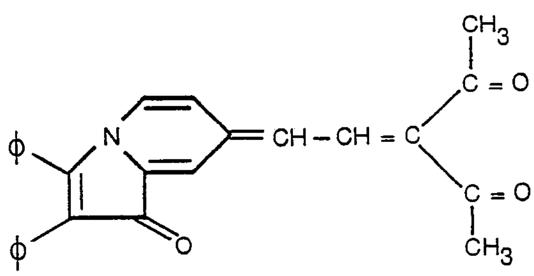

7-[1-cyano-2-(4-dimethylaminophenyl)-1-ethenyl]-1,2-diphenyl-3-indolizinonium trifluoromethane sulfonate

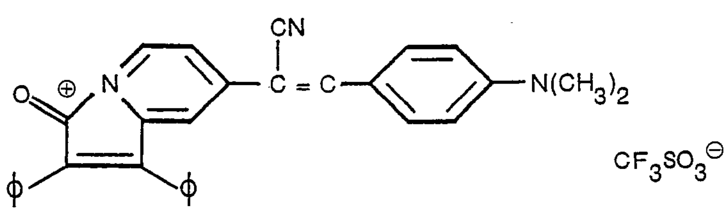

1,2-di-tert-butylphenyl-7-[4-(4-dimethylaminophenyl)-1-(1,3-butadienyl)]-3-indolizonium trifluoromethane sulfonate

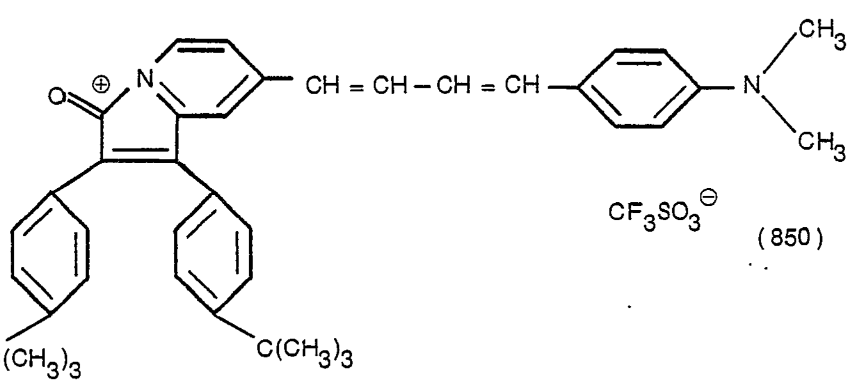

(850)

2,3-diphenyl-7-4-(2,6-diphenyl-4(4H)-pyranylidene)-2-(2-butenyl)-1-indolizinonium trifluoromethane sulfonate

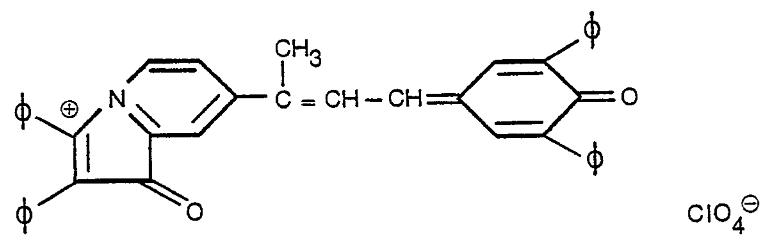

Additional compounds of this class are shown below in Tables I and II:

TABLE I

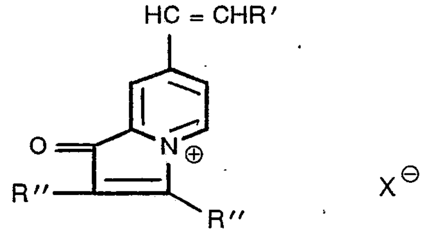

| Compound | R′ | R″ | $X^{\theta}$ | λmax (nm) |
|---|---|---|---|---|
| 1–1 | 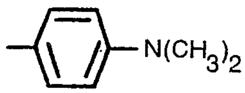 | 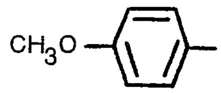 | $BF_4$ | 780 |
| 1–2 | 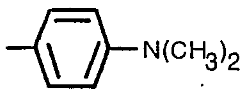 | 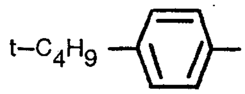 | $BF_4$ | 780 |
| 1–3 | 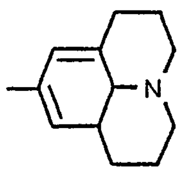 | 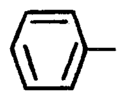 | $BF_4$ | 837 |
| 1–4 | 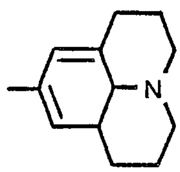 | 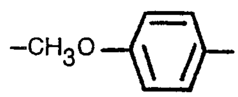 | $BF_4$ | 838 |
| 1–5 | 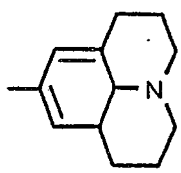 | 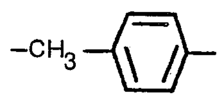 | $CF_3SO_3$ | 838 |
| 1–6 | 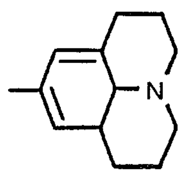 | 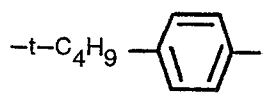 | $BF_4$ | 840 |
| 1–7 | 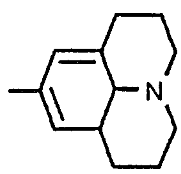 | 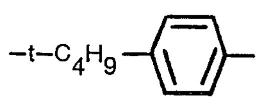 | $CF_3SO_3$ | 840 |

TABLE II

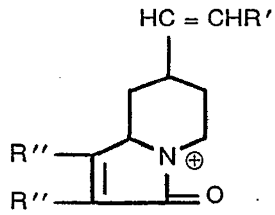 $X^{\ominus}$

| Compound | R′ | R″ | $X^{\theta}$ | λmax (nm) |
|---|---|---|---|---|
| 2–1 | 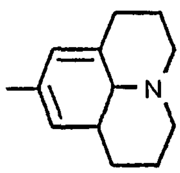 | 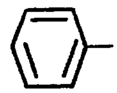 | $BF_4$ | 788 |
| 2–2 | 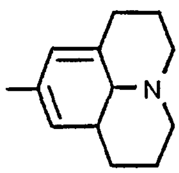 | 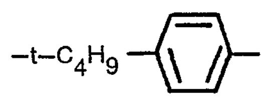 | $BF_4$ | 790 |
| 2–3 | 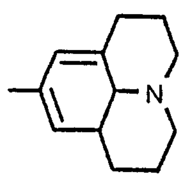 | 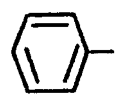 | $CF_3SO_3$ | 788 |
| 2–4 | 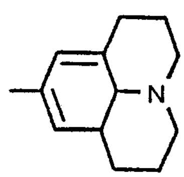 | 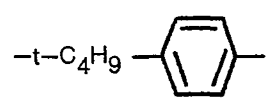 | $CF_3SO_3$ | 790 |

A further class of dyes according to the invention is represented by the structural formula:

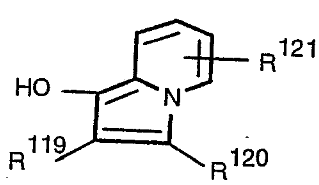 (XXII)

wherein

$R^{119}$ and $R^{120}$ are individually aryl containing 6 to 20 carbon atoms, such as phenyl and naphthyl; or, alkyl containing 1 to 18 carbon atoms, such as methyl, ethyl, propyl, decyl and lauryl;

$R^{121}$ is cyano, carboxy, formyl, acyl containing 2 to 18 carbon atoms, such as acetyl, propionyl and lauroyl; carboalkoxy containing 2 to 18 carbon atoms, such as carbomethoxy, carboethoxy and carbobutoxy; or aminocarbonyl containing 1 to 18 carbon atoms, such as unsubstituted aminocarbonyl, methylaminocarbonyl and dimethylaminocarbonyl which enables the compound to be a dye.

The compounds in this class are shown in the enol form, rather than the keto form. Examples of compounds within this class are as follows:

7-cyano-2,3-diphenyl-1-indolizinol

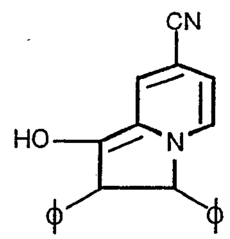

(405)

7-formyl-2,3-di-(4-methoxyphenyl)-1-indolizinol

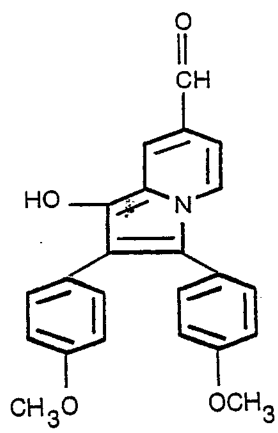

(423)

6-aminocarbonyl-2,3-diphenyl-1-indolizinol

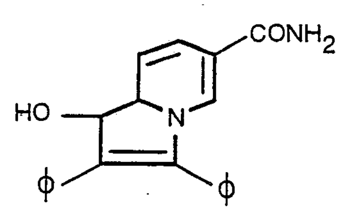

8-carboethoxy-2,3-diphenyl-1-indolizinol

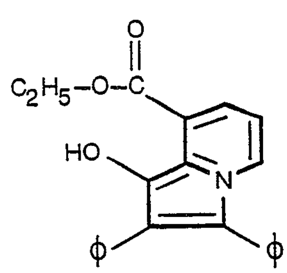

(490)

7-carboxy-2,3-diphenyl-1-indolizinol

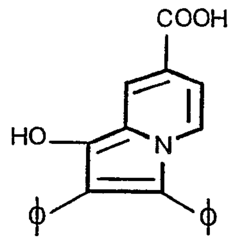

(420)

Optimum methods for preparation of dyes according to the invention will vary, depending upon the desired dye, the starting material, the cyclopropenone, the color-forming coupler, the pyridine compound, solvents present, reaction temperature, concentration of reactants and catalyst present. The cyclopropenone and pyridine compounds are mixed in about stoichiometric concentrations. However, it is often useful to mix the reactants with an excess of the pyridine compound to provide better yields or different isomers.

The reactants for forming a dye according to the invention can be mixed in a suitable reaction medium. For example, the cyclopropenone and pyridine compounds are mixed in an appropriate reaction medium, such as an organic solvent or medium that forms a coatable composition, for subsequent utilization of the dye which is formed.

A reaction medium which comprises a solvent for the reactants is most useful. A useful solvent includes, for example, pyridine, chlorinated hydrocarbons, such as methylene chloride and chlorobenzene, toluene, dioxane, and tetrahydrofuran. Pyridine and some pyridine related solvents, such as 4-picoline, are especially useful in producing isomers. The reactants are mixed at room temperature (about 19°C) and then heated to within the range of about 50 to about 150°C. The optimum reaction temperature will be influenced by the choice of solvent, the particular reactants, the desired dye, and other described factors.

When a dye according to the invention is formed by the reaction of a cyclopropenone with a pyridine compound and suitable color-forming compound, such as a color-forming coupler, it is preferred that the reaction be carried out in chemical association with an appropriate oxidant, such as elemental iodine, oxygen, copper bromide, copper chloride, copper acetate, benzoyl peroxide or copper acetyacetonate. The concentration of oxidant will vary, depending upon the particular reactants, processing conditions, desired dye, and reaction medium. An oxidant is especially useful in the reaction of a cyclopropenone with a pyridine compound and an active methylene coupler.

In preparing an oxoindolizine dye by the reaction of pyridine compound with a cyclopropenone compound, the condensation is generally carried out in a solvent. The concentration of reactants is generally about stoichiometric. However, an excess of pyridine or picoline is often useful. The reaction temperature is generally within the range of 0°C to 95°C. The reaction is preferably carried out in chemical association with an oxidant, such as copper ions or air.

An especially useful method according to the invention comprises preparation of a dye represented by the structure (XXI) comprising reacting a compound represented by the structure

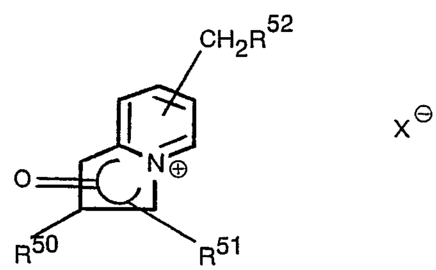

(XII)

wherein

$X^{\ominus}$, $R^{50}$, $R^{51}$ and $R^{52}$ are as defined above,

with an aldehyde or ketone represented by the formula

$$R^{122}—\overset{O}{\overset{\|}{C}}—R^{123} \qquad (XXIV)$$

wherein

$R^{122}$ and $R^{123}$ are individually hydrogen or substituents that do not adversely affect the oxoindolizinium

dye, such as alkyl containing 1 to 20 carbon atoms, for example, methyl, ethyl, propyl, butyl, decyl and lauryl; aryl containing 6 to 20 carbon atoms, such as phenyl, tolyl, and naphthyl; or a heterocyclic group, such as pyridyl and julolidyl; and at least one of $R^{122}$ and $R^{123}$ is a monovalent group which completes a chromophore as defined.

Such compounds include, for example, pyrylium, flavylium, dimethylamino benzaldehyde and cinnimaldehyde compounds. These reactants (XII) and (XXIV) are reacted in about equimolar proportions in a suitable solvent, such as acetic anhydride, with or without a catalyst, such as piperidine or sodium acetate, at a temperature within the range of about 20°C to about 140°C. The resulting dye crystallizes from the medium or is precipitated by addition of a non-solvent, such as water, ethyl ether or cyclohexane. An example of such a method according to the invention is a method of preparing a dye represented by the formula:

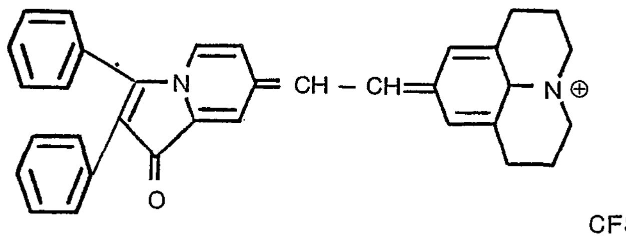

$CFSO_3^{\ominus}$

comprising the step:

(1) reacting a compound represented by the formula:

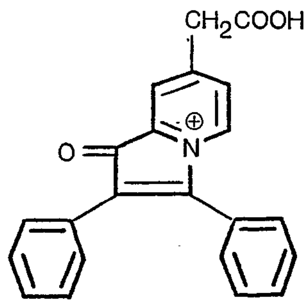

$CF_3SO_3^{\ominus}$

with a compound represented by the formula:

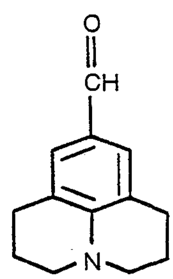

Another method of preparing dyes according to the invention comprises reacting an indolizinol represented by the formula:

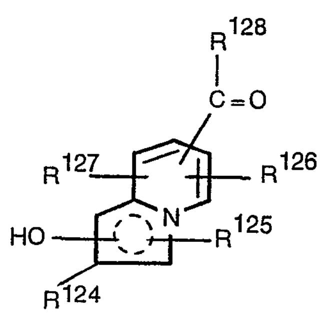

(XXV)

wherein

$R^{124}$ and $R^{125}$ are individually aryl containing 6 to 14 carbon atoms, such as phenyl, xylyl, methoxy-

phenyl and naphthyl; or, alkyl containing 1 to 20 carbon atoms, such as methyl, ethyl, propyl, decyl and lauryl;

$R^{126}$ is hydrogen, cyano, carboxy, formyl, acyl containing 2 to 18 carbon atoms, such as acetyl, propionyl, and lauroyl; carboalkoxy containing 2 to 10 carbon atoms, such as carbomethoxy, carboethoxy and carbobutoxy; or aminocarbonyl containing 1 to 18 carbon atoms, such as unsubstituted aminocarbonyl, methylaminocarbonyl and dimethylaminocarbonyl; and alkyl containing 1 to 18 carbon atoms, such as methyl, ethoxy, propyl, butyl, decyl and lauryl;

$R^{127}$ is hydrogen or alkyl containing 1 to 4 carbon atoms, such as methyl, ethyl, propyl and butyl;

$R^{128}$ is alkyl containing 1 to 18 carbon atoms, such as methyl, ethyl, propyl, butyl, decyl and lauryl; or aryl containing 6 to 14 carbon atoms, such as phenyl, tolyl, xylyl and naphthyl;

with an active methylene coupler, such as represented by formula (IX). The indolizinol represented by formula (XXV) and the active methylene coupler are reacted in about equimolar proportions in a suitable solvent, such as acetic anhydride, preferably with a catalyst, such as piperidine or sodium acetate, at a temperature within the range of 20°C to 140°C. The resulting dye crystallizes from the reaction medium and is preferably precipitated by the addition of a non-solvent, such as water, ethyl ether or cyclohexane.

Many oxoindolizine and oxoindolizinium dyes within Structures I and II are useful in imaging, such as in photothermographic imaging or in laser recording and reading applications. Especially useful dyes according to the invention are compounds that are image dyes or, alternatively, are capable of forming image dyes. Selection of an optimum indolizinone or indolizinium dye will depend upon such factors as the desired use, processing conditions, desired image, particular components with the dye, exposure means to form an image, and stability of the dye.

The following examples are included for a further understanding of the invention.

### Example 1

Preparation of 7,7-(1,2-(1,2-ethane-(E)-diylidene)*bis*-1,2-di-(4-*tert*-butyl-phenyl)-3(7H)-indolizinone

A solution (10 percent by weight) of 2,3-di(4-tertiarybutylphenyl) cyclopropenone, in 4-picoline (pyridine compound), was prepared containing a trace of cupric acetate (catalyst). The solution was sparged with a stream of air to provide agitation and excess oxygen. The solution was heated on a steam bath to 80°C to 95°C for 15 minutes. A pasty cyan-colored slurry resulted. The resulting mixture was filtered to remove excess picoline, and the colored solids washed with acetone. The solids were dried under vacuum to remove the acetone-washed solvent. A 25 percent yield of the desired dye was obtained based on the cyclopropenone starting material. The dye had a maximum absorption at 695 nm in chloroform solution. The structure was confirmed by mass spectroscopy, nuclear magnetic resonance, infrared spectral analysis and x-ray diffraction.

### Example 2

Preparation of 7-(4-Pyridyl)-2,3-di-(4-methoxyphenyl)indolizinol, Benzyl Bromide Salt

Equimolar amounts of benzyl bromide and 4,4′-di-pyridine were dissolved in N,N-dimethylformamide to form approximately a 10 percent by weight solution. The solution was heated for 10 minutes on a steam bath at 95°C to form the quaternary salt of bipyridine. The reaction mixture was cooled slightly, and an equimolar amount of 2,3-di-(4-methoxyphenyl) cyclopropenone was added to the solution. The reaction mixture was heated for 15 minutes and quenched in excess cold water. A solution of 48 percent hydrobromic acid was added to the water-N,N-dimethylformamide solution to precipitate the desired dye product. The precipitated dye was removed by filtration and dried under vacuum. The dye had a maximum absorption density at 535 nm in chloroform solution. The desired dye structure was confirmed by mass spectroscopy, nuclear magnetic resonance and infrared spectral analysis.

### Example 3

Preparation of 7-Dibenzoylmethylidene-2,3-di(4-methoxyphenyl)-1(7H)-indolizinone

A 10 percent solution of 2,3-di(4-methoxyphenyl) cyclopropenone in pyridine was refluxed under nitrogen for 15 minutes. The resulting solution was cooled slightly, and an equivalent amount of dibenzoylmethane based on the cyclopropenone was added to the green solution. The reaction mixture was refluxed for 60 minutes. The resulting reaction mixture was again cooled, and four equivalents of iodine dissolved in a small amount of pyridine was added to the reaction mixture. The mixture was further heated at 90°C on a steam bath for 15 minutes. The bright blue solution was quenched by pouring it into cold excess dilute hydrochloric acid. The desired dye precipitated and was removed from the solution by filtering. A 95 percent yield of the desired dye was obtained based on the starting cyclopropenone. The dye was chromatographed on silica gel to provide a purified product. The maximum absorption of the dye was at 605 nm in chloroform solution. The structure of the dye was confirmed by mass spectroscopy, nuclear magnetic resonance and infrared analysis.

### Example 4

Preparation of 7-Formyl-2,3-di(4-methoxyphenyl)-1-indolizinol

Equivalent amounts of 4-formylpyridine and 2,3-di(4-methoxyphenyl) cyclopropenone were dissolved in sufficient para-dioxane to form approximately a 10 percent solution. The mixture was refluxed at 102°C

under nitrogen for 2 hours. Sufficient water was then added to the reaction mixture to bring it to the cloud point at 80°C. The reaction mixture was then cooled to room temperature, and the product allowed to crystallize. The crystals were collected by filtration, and washed with a small amount of water. The dried crystals were the desired dye. The dye was produced in a 95 percent yield based on the amount of cyclopropenone. The yellow dye had a maximum absorption of 435 nm in chloroform solution. The structure of the dye was confirmed by mass spectroscopy, nuclear magnetic resonance and infrared analysis.

Examples 5—8

Other yellow dyes were prepared by a modification of the procedure described in Example 4. The modification consisted of substituting the particular pyridine needed to obtain the desired dye for the 4-formyl pyridine described in Example 4. Structures were confirmed by mass spectrometry, nuclear magnetic resonance and elemental analysis. Examples of the yellow dyes prepared are as follows:

EXAMPLE 5:

7-carboxyl-2,3-diphenyl-1-indolizinol

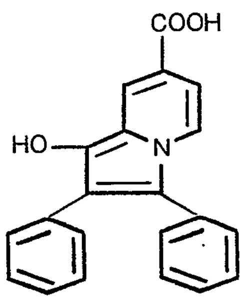

λ max 430

EXAMPLE 6:

7-carbomethoxy-2,3-di(4-tert-butylphenyl)-1-indolizinol

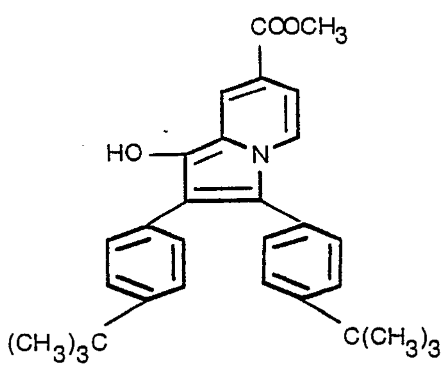

λ max 425

EXAMPLE 7:

7-aminocarbonyl-2,3-diphenyl-1-indolizinol

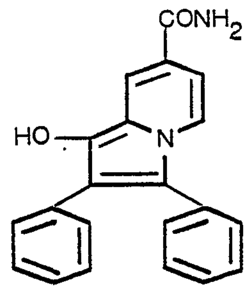

λ max 405

EXAMPLE 8:

7-cyano-2,3-diphenyl-1-indolizinol

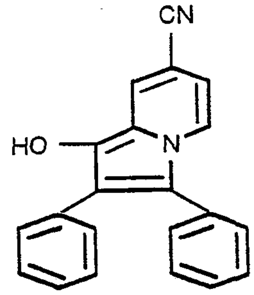

λ max 410

Example 9

Preparation of 1,2-di-(4-tert-butylphenyl)-7-[4-(4-dimethylaminophenyl)-1-(1,3-butadienyl)]-3-indolizinonium trifluoromethanesulfonate

Equivalent amounts of 4-dimethylaminocinnamaldehyde and 1,2-di(tert-butylphenyl-7-methyl-3-indolizinonyl trifluoromethane sulfonate were dissolved in acetic anhydride to form approximately a 10 percent solution. The reaction mixture was heated at 70—90°C for five minutes, cooled to room temperature and diluted with diethyl ether and the resulting product collected by filtration. The crude product was recrystallized from acetone to furnish the desired dye.

Example 10

Preparation of 7-(4-dimethyl-aminophenyl)-2,3-diphenyl-1-indolizinonium fluoborate

A 10% solution of 1,2-diphenyl-1-indolizinonium triiodide in dimethylaniline was warmed at 70—90°C for 10 minutes. The resulting solution was cooled and diluted with diethyl ether and the resulting solid redissolved in acetone. The desired dye was precipitated by the addition of dilute fluoboric acid to the solution.

Example 11

Preparation of 7-diethylamino-2,3-diphenyl-1-indolizinonium fluoborate

A 10% solution of 2,3-diphenyl-1-indolizinonium triiodide in pyridine was treated with two equivalents of anhydrous diethyl amine and heated at 70—90°C for 15 minutes. The reaction mixture was cooled and poured into diethyl ether and filtered to furnish the crude dye. The dye was washed thoroughly with water to remove soluble salts to furnish purified product.

Examples 12—14

Use of Dyes in Optical Disc for Laser Writing and Reading

Oxoindolizine and oxoindolizinium dyes for use in an optical disc were selected to provide the desired characteristics for laser writing and reading including among other characteristics, the desired solubility, absorption and stability characteristics.

In each of the examples an optical disc for laser writing and reading was prepared by coating, on a support designed for an optical disc, a layer of an amorphous composition comprising a binder, such as cellulose nitrate, and an oxoindolizine or oxoindolizinium dye having an absorption at a wavelength at which the laser was tuned, such as a wavelength in the range of about 300 to about 1000 nanometers. Optical discs were prepared by techniques described in, for example, "Disc-Storage Technology" by Robert M. White, *Scientific American*, August 1980, beginning at page 138, and *Research Disclosure*, November, 1978, Item No. 17522.

The dyes of Examples 12, 13 and 14 were individually incorporated in a coating composition containing cellulose nitrate (binder) and cyclohexanone (solvent). The resulting compositions were coated on optical disc supports containing a reflective metal layer, such as aluminum. The resulting optical discs were imagewise exposed to a laser emitting at 800 nanometers pulsed at 10 MHz and a 50% duty cycle in a 30 KHz bandwidth to provide an image on each optical disc. Reading from the exposed optical discs was by monitoring the feedback from the same laser. The following dyes were tested in the video discs:

| Example No. | Dye |
|---|---|
| 12 | 2,3-diphenyl-7-[2-(9-julolidinyl)ethenyl]-1-oxo-1H-indolizinium trifluoromethanesulfonate |
| 13 | 2,3-bis(4-t-butylphenyl)-7-[2-(9-julolidinyl)-ethenyl]-1-oxo-1H-indolizinium trifluoromethanesulfonate |
| 14 | 1,2-bis(4-t-butylphenyl)-7-[2-(9-julolidinyl)-ethenyl]-3-oxo-3H-indolizinium trifluoromethanesulfonate |

An image was recorded and read on each of the optical discs. The recording power at the discs was 12 mW in each case.

Example 15

Production of a Red Dye in a Coating

A solution was prepared containing 525 mg of poly(ethylene-co-1,4-cyclohexylenedimethylene-1-methyl-2,4-benzenedisulfonamide) (binder), 400 mg of 1-methyl-4-(4-pyridyl)pyridinium-para-toluenesulfonate (pyridine compound) and 9.980 g of 2-methoxyethanol (solvent). The polysulfonamide binder and quaternary salt (pyridine compound) were dissolved in the 2-methoxyethanol by gentle agitation at room temperature (19°C). A clear lacquer solution resulted which was coated on a poly(ethylene terephthalate) film support at a wet coating thickness of 0.125 mm. The coating was dried by heating the material to about 24°C for 30 minutes in a stream of air.

A second solution was prepared by dissolving 525 g of poly(styrene-co-butadiene) (KRO—3, which is a trade name of and available from Phillips Petroleum Company, U.S.A.), in 9.98 g of toluene with 40 mg of 1-phenyl-2-(para-methoxy-phenyl)cyclopropenone (photosensitive cyclopropenone compound). Solution was produced by stirring at 22°C for several hours. A clear lacquer solution resulted which was coated directly over the first layer containing the pyridine compound. A wet coating thickness of 0.125 mm was applied. The resulting composite two-layer element was dried by warming to 45°C for 30 minutes. The resulting element was exposed to a 250 watt mercury lamp for 20 seconds at a distance of 7.6 cm. The desired dye was produced by heating the element, after exposure, to 150°C for 3 seconds on a heated aluminum block. A brilliant red dye was formed which had a maximum absorption at 535 nm.

Example 16

Production of a Blue Dye in a Coating

A coating solution was prepared by dissolving 0.500 g of the polysulfonamide binder as described in Example 15 and 500 mg of 4-azastyryl-1-methyl-pyridinium *para*-toluenesulfonate (pyridine compound) in 10 g of 2-methoxyethanol (solvent). Solution was produced by stirring at room temperature (19°C). A clear lacquer solution resulted which was coated on a poly(ethylene terephthalate) film support by means of a doctor blade to produce a wet coating thickness of 0.125 mm. The resulting coating was dried by heating the coating to about 24°C for 30 minutes in a steam of air.

A second solution was prepared by dissolving 25 mg of phenylanisyl cyclopropenone and 0.50 g of poly(styrene-co-butadiene) resin in 10.0 g of toluene. A clear solution which resulted upon stirring the mixture for 3 hours at room temperature (19°C) was coated directly over the first layer containing the pyridine compound. A wet coating thickness of 0.125 mm was applied by means of a doctor blade. The

composite two-layer element was dried by warming the resulting coating to about 24°C for 30 minutes in a stream of air. A brilliant clear transparent film was obtained.

The resulting element was imagewise exposed and then heated as described in Example 15. A blue dye was formed which had a maximum absorption at 575 nm.

Example 17

Production of a Green Dye in a Coating

A coating solution was prepared by dissolving 0.50 g of poly(styrene-co-butadiene) resin and 125 mg of 4,4'-dipyridylethylene (pyridine compound) in 10.0 g of toluene (solvent). A clear solution which resulted upon stirring at room temperature (19°C) was coated on a poly(ethylene terephthalate) film support containing a gelatin subbing layer at a wet coating thickness of 0.125 mm. The resulting coating was dried by heating to 24°C for 30 minutes. A second layer was coated over the layer containing the pyridine compound. This second layer was prepared from a coating solution produced by dissolving 0.50 g of poly(vinyl alcohol) in 9.50 g of water. The composition containing the poly(vinyl alcohol) was coated at a wet coating thickness of 0.125 mm over the first layer. The resulting composite film was dried by heating to 24°C for 30 minutes. A top layer was prepared by coating a solution containing 125 mg of photosensitive phenylanisyl cyclopropenone and 0.50 g of poly(styrene-co-butadiene) dissolved in 10.0 g of toluene. The top layer was coated at a wet coating thickness of 0.125 mm. The resulting composite film was dried for 30 minutes at 24°C in an air stream. The composite film was imagewise exposed for 40 seconds and then heated as described in Example 15. A dye was produced which had a maximum absorption in the infrared region of the electromagnetic spectrum at 815 nm.

Example 18

One Layer Element

A coating solution was prepared by dissolving .50 g of poly(styrene-co-butadiene) resin, 40 mg of o,p-dianisylcyclopropenone (photosensitive cyclopropenone), and 40 mg of 1,2-bis(4-pyridyl)ethylene (pyridine compound) in 10.0 g of toluene. The solution was coated on a poly(ethylene terephthalate) film support at a wet coating thickness of 0.125 mm. The coating was dried by standing at 24°C for two hours. The resulting element was exposed to a 250 watt mercury lamp for 20 seconds at a distance of 7.6 cm through a mask to produce a developable image in the photographic element. The desired dye was produced by heating the element, after exposure, to 150°C for 10 seconds on a heated aluminum block. An infrared dye was formed in the film with a maximum absorbtion at 830 nm.

## Claims

1. A method of preparing an oxoindolizine or an oxoindolizinium dye compound which comprises reacting (A) a pyridine compound having hydrogen atoms on the carbon atom ortho to the heterocyclic nitrogen atom with (B) a disubstituted cyclopropenone compound, and, if substituents in the pyridine compound are not such that the reaction product is the desired dye compound, reacting the said reaction product with a color-forming compound to give the desired dye compound.

2. A method according to Claim 1 characterized in that said dye compound has the formula:

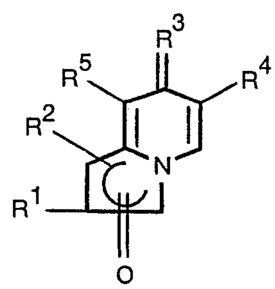

wherein

$R^1$ and $R^2$ are individually alkyl containing 1 to 18 carbon atoms; aryl containing 6 to 20 carbon atoms; or polystyryl having appended indolizine or indolizinium groups or combinations thereof;

$R^3$ is a divalent group which, with the indolizinone nucleus, completes an organic chromophore;

$R^4$ is hydrogen; alkyl containing 1 to 18 carbon atoms; cyano; acyl containing 2 to 20 carbon atoms; carboalkoxy containing 2 to 18 carbon atoms; aminocarbonyl containing from 1 to 18 carbon atoms; acyloxy containing 2 to 18 carbon atoms; bromine or chlorine; and

$R^5$ is hydrogen; chlorine; bromine or alkyl containing 1 to 18 carbon atoms; and said pyridine compound has the formula:

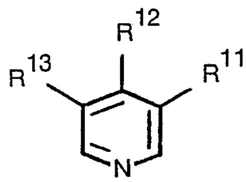

and said cyclopropenone compound is represented by the formula

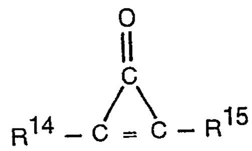

wherein

$R^{11}$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine;

$R^{12}$ is hydrogen; alkyl containing 1 to 18 carbon atoms; cyano; acyl containing 2 to 20 carbon atoms; benzyl; or pyridyl;

$R^{13}$ is hydrogen; chlorine; bromine or alkyl containing 1 to 18 carbon atoms; and

$R^{14}$ and $R^{15}$ are individually aryl containing 6 to 20 carbon atoms; aralkenyl containing 6 to 20 carbon atoms; alkyl containing 1 to 18 carbon atoms; or $R^{14}$ and $R^{15}$ together represent the carbon atoms necessary to complete a 7- or 8-member cyclic structure.

3. A method according to Claim 1 characterized in that said dye compound has the formula:

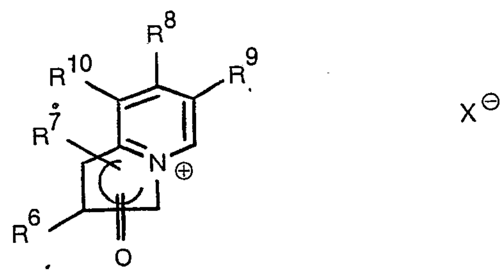

wherein

$X^{\ominus}$ is an anion;

$R^6$ and $R^7$ are individually alkyl containing 1 to 18 carbon atoms; aryl containing 6 to 20 carbon atoms; or polystyryl having appended indolizine or indolizinium groups or combinations thereof;

$R^8$ is a monovalent group which, with the indolizine nucleus, completes an organic chromophore;

$R^9$ is hydrogen; alkyl containing 1 to 18 carbon atoms; cyano, acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine; or chlorine; and

$R^{10}$ is hydrogen; chlorine; bromine; or, alkyl containing 1 to 18 carbon atoms; and said pyridine compound has the formula:

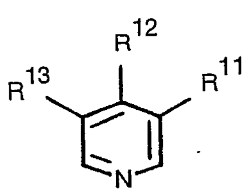

and said cyclopropenone compound is represented by the formula

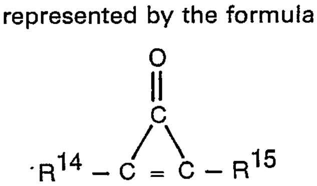

wherein:

$R^{11}$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine;

$R^{12}$ is hydrogen; alkyl containing 1 to 18 carbon atoms; cyano; acyl containing 2 to 20 carbon atoms; benzyl; or pyridyl;

$R^{13}$ is hydrogen; chlorine; bromine or alkyl containing 1 to 18 carbon atoms; and

$R^{14}$ and $R^{15}$ are individually aryl containing 6 to 20 carbon atoms; aralkenyl containing 6 to 20 carbon atoms; alkyl containing 1 to 18 carbon atoms; or $R^{14}$ and $R^{15}$ together represent the carbon atoms necessary to complete a 7- or 8-member cyclic structure, the anion $X^{\ominus}$ being provided by an oxidant or color-forming compound (if used) or otherwise.

4. A dye compound having the formula:

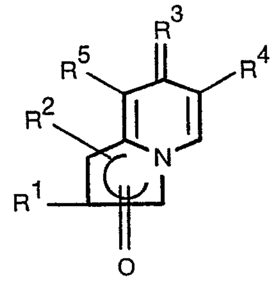

wherein

$R^1$ and $R^2$ are individually alkyl containing 1 to 18 carbon atoms; aryl containing 6 to 20 carbon atoms; or polystyryl having appended indolizine or indolizinium groups or combinations thereof:

$R^3$ is a divalent group which, with the indolizinone nucleus, completes an organic chromophore;

$R^4$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine; and

$R^5$ is hydrogen; chlorine; bromine or alkyl containing 1 to 18 carbon atoms.

5. A dye compound having the formula:

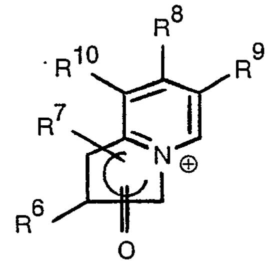

$X^{\ominus}$

wherein

$X^{\ominus}$ is an anion;

$R^6$ and $R^7$ are individually alkyl containing 1 to 18 carbon atoms; aryl containing 6 to 20 carbon atoms; or polystyryl having appended indolizine or indolizinium groups or combinations thereof;

$R^8$ is a monovalent group which, with the indolizinium nucleus, completes an organic chromophore;

$R^9$ is hydrogen; alkyl containing 1 to 18 carbon atoms; cyano, acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine or chlorine; and

$R^{10}$ is hydrogen; chlorine; bromine; or alkyl containing 1 to 18 carbon atoms.

6. A dye compound having the formula:

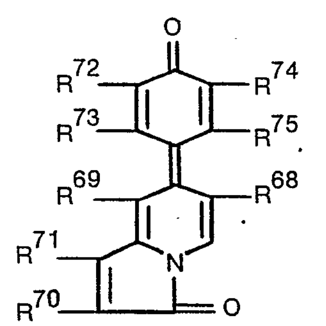

or

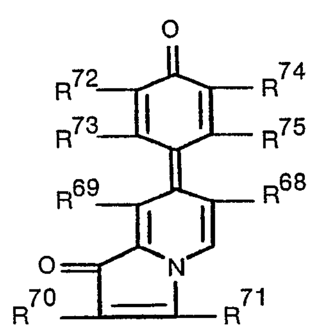

wherein:

$R^{68}$ is hydrogen; alkyl containing 1 to 18 carbon atoms; cyano; acyl containing 2 to 20 carbon atoms; carboalkoxy containing 2 to 18 carbon atoms; aminocarbonyl containing 1 to 18 carbon atoms; acyloxy containing 2 to 18 carbon atoms; bromine; or chlorine;

$R^{69}$ is hydrogen; chlorine; bromine; or alkyl containing 1 to 18 carbon atoms;

$R^{70}$ and $R^{71}$ are individually alkyl containing 1 to 18, preferably 1 to 10 carbon atoms; or aryl containing 6 to 20 carbon atoms;

$R^{72}$ and $R^{73}$ are individually hydrogen; alkyl containing 1 to 22 carbon atoms, aryl containing 6 to 20 carbon atoms; amino; carboxamido; sulfonamido; sulfamyl; carbamyl; halogen; alkoxy containing 1 to 18 carbon atoms; or $R^{72}$ and $R^{73}$ taken together represent the atoms necessary to complete a benzo group; and

$R^{74}$ and $R^{75}$ are individually hydrogen; hydroxy; alkyl containing 1 to 22 carbon atoms; aryl containing 6 to 20 carbon atoms; amino; carboxamido; sulfonamido; sulfamyl; carbamyl; halogen; or alkoxy containing 1 to 18 carbon atoms.

**Patentansprüche**

1. Verfahren zur Herstellung einer Oxoindolizin- oder Oxoindoliziniumfarbstoffverbindung, dadurch gekennzeichnet, daß man (A) eine Pyridinverbindung mit Wasserstoffatomen an dem Kohlenstoffatom in ortho-Stellung zum heterocyclischen Stickstoffatom mit (B) einer disubstituierten Cyclopropenonverbindung umsetzt, und daß man, falls die Substituenten der Pyridinverbindung nicht derart sind, daß das Reaktionsprodukt die gewünschte Farbstoffverbindung ist, das Reaktionsprodukt zur Bildung der gewünschten Farbstoffverbindung mit einer farbbildenden Verbindung umsetzt.

2. Verfahren nach Anspruch 1, dadurch gekennzeichnet, daß die Farbstoffverbindung der folgenden Formel entspricht:

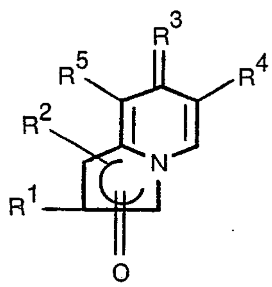

in der bedeuten:

$R^1$ und $R^2$ einzeln Alkylgruppen mit 1 bis 18 C—Atomen; Arylgruppen mit 6 bis 20 C—Atomen oder Polystyrylgruppen mit hieran befestigten Indolizin- oder Indoliziniumgruppen oder Kombinationen hiervon;

$R^3$ eine divalente Gruppe, die mit dem Indolizinonkern ein organisches Chromophor vervollständigt;

$R^4$ ein Wasserstoffatom, eine Alkylgruppe mit 1 bis 18 C—Atomen, eine Cyanogruppe; eine Acylgruppe mit 2 bis 20 C—Atomen; eine Carboalkoxygruppe mit 2 bis 18 C—Atomen, eine Aminocarbonylgruppe mit 1 bis 18 C—Atomen; eine Acyloxygruppe mit 2 bis 18 C—Atomen; eine Brom- oder ein Chloratom und

$R^5$ ein Wasserstoff-, Chlor- oder Bromatom oder eine Alkylgruppe mit 1 bis 18 C—Atomen,

und daß die Pyridinverbindung der folgenden Formel entspricht:

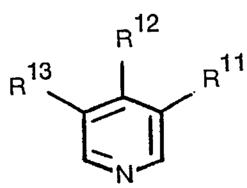

und daß die Cyclopropenonverbindung der folgenden Formel entspricht:

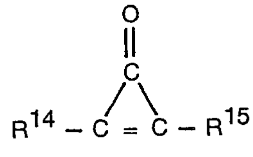

(IV)

worin bedeuten:

$R^{11}$ ein Wasserstoffatom; eine Alkylgruppe mit 1 bis 18 C—Atomen; eine Cyanogruppe; eine Acylgruppe mit 2 bis 20 C—Atomen; eine Carboalkoxygruppe mit 2 bis 18 C—Atomen; eine Aminocarbonylgruppe mit 1 bis 18 C—Atomen; eine Acyloxygruppe mit 2 bis 18 C—Atomen; ein Brom- oder Chloratom;

$R^{12}$ ein Wasserstoffatom; eine Alkylgruppe mit 1 bis 18 C—Atomen; eine Cyanogruppe; eine Acylgruppe mit 2 bis 20 C—Atomen; eine Benzyl- oder Pyridylgruppe;

$R^{13}$ ein Wasserstoff-, Chlor- oder Bromatom oder eine Alkylgruppe mit 1 bis 18 C—Atomen und

$R^{14}$ und $R^{15}$ einzeln Arylgruppen mit 6 bis 20 C—Atomen; Aralkenylgruppen mit 6 bis 20 C—Atomen; Alkylgruppen mit 1 bis 18 C—Atomen; oder $R^{14}$ und $R^{15}$ gemeinsam die Kohlenstoffatome, die zur Vervollständigung einer 7- oder 8- gliedrigen cyclischen Struktur erforderlich sind.

3. Verfahren nach Anspruch 1, dadurch gekennzeichnet, daß die Farbstoffverbindung der folgenden Formel entspricht:

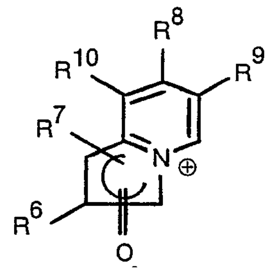

$X^{\ominus}$

in der bedeuten:

$X^{\ominus}$ ein Anion;

$R^6$ und $R^7$ einzeln Alkylgruppen mit 1 bis 18 C—Atomen; Arylgruppen mit 6 bis 20 C—Atomen oder Polystyrylgruppen mit hieran befestigten Indolizin- oder Indoliziniumgruppen oder Kombinationen hiervon;

$R^8$ eine monovalente Gruppe, die mit dem Indoliziniumkern ein organisches Chromophor vervollständigt;

$R^9$ ein Wasserstoffatom; eine Alkylgruppe mit 1 bis 18 C—Atomen; eine Cyanogruppe; eine Acylgruppe mit 2 bis 20 C—Atomen; eine Carboalkoxygruppe mit 2 bis 18 C—Atomen; eine Aminocarbonylgruppe mit 1 bis 18 C—Atomen, eine Acyloxygruppe mit 2 bis 18 C—Atomen oder ein Brom- oder Chloratom und

$R^{10}$ ein Wasserstoff-, Chlor- oder Bromatom oder eine Alkylgruppe mit 1 bis 18 C—Atomen;

daß die Pyridinverbindung der folgenden Formel entspricht:

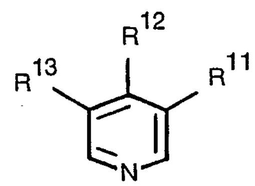

und daß die Cyclopropenonverbindung durch die folgende Formel wiedergegeben wird:

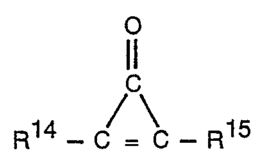

(IV)

wobei in den Formeln bedeuten:

$R^{11}$ ein Wasserstoffatom; eine Alkylgruppe mit 1 bis 18 C—Atomen; eine Cyanogruppe, eine Acylgruppe mit 2 bis 20 C—Atomen, eine Carboalkoxygruppe mit 2 bis 18 C—Atomen; eine Aminocarbonylgruppe mit 1 bis 18 C—Atomen; eine Acyloxygruppe mit 2 bis 18 C—Atomen; ein Brom- oder Chloratom;

$R^{12}$ ein Wasserstoffatom; eine Alkylgruppe mit 1 bis 18 C—Atomen; eine Cyanogruppe, eine Acylgruppe mit 2 bis 20 C—Atomen; eine Benzyl- oder Pyridylgruppe;

$R^{13}$ ein Wasserstoff-, Chlor oder Bromatom oder eine Alkylgruppe mit 1 bis 18 C—Atomen und

$R^{14}$ und $R^{15}$ einzeln Arylgruppen mit 6 bis 20 C—Atomen; Aralkenylgruppen mit 6 bis 20 C—Atomen; Alkylgruppen mit 1 bis 18 C—Atomen; oder $R^{14}$ und $R^{15}$ gemeinsam die Kohlenstoffatome, die zur Vervollständigung einer 7- oder 8-gliedrigen cyclischen Struktur erforderlich sind,

und das Anion$^{\ominus}$ durch ein Oxidationsmittel oder eine farbbildende Verbindung (sofern verwendet) oder in anderer Weise geliefert wird.

4. Farbstoffverbindung der folgenden Formel:

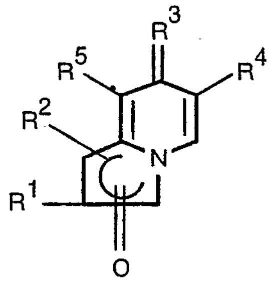

in der bedeuten:

$R^1$ und $R^2$ einzeln Alkylgruppen mit 1 bis 18 C—Atomen, Arylgruppen mit 6 bis 20 C—Atomen oder Polystyrylgruppen mit hieran befestigten Indolizin- oder Indoliziniumgruppen oder Kombinationen hiervon;

$R^3$ eine divalente Gruppe, die mit dem Indolizi onkern ein organisches Chromophor vervollständigt;

$R^4$ ein Wasserstoffatom, eine Alkylgruppe mit 1 bis 18 C—Atomen, eine Cyanogruppe; eine Acylgruppe mit 2 bis 20 C—Atomen; eine Carboalkoxygruppe mit 2 bis 18 C—Atomen; eine Aminocarbonylgruppe mit 1 bis 18 C—Atomen, eine Acyloxygruppe mit 2 bis 18 C—Atomen; ein Brom- oder ein Chloratom und

$R^5$ ein Wasserstoff-, Chlor- oder Bromatom oder eine Alkylgruppe mit 1 bis 18 C—Atomen.

5. Farbstoffverbindung der folgenden Formel:

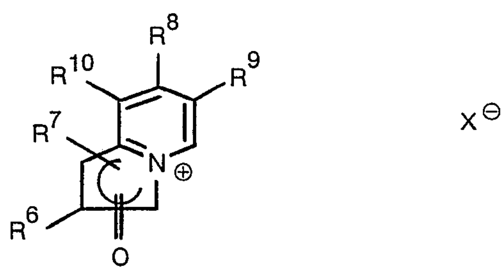

in der bedeuten:

$X^{\ominus}$ ein Anion;

$R^6$ und $R^7$ einzeln Alkylgruppen mit 1 bis 18 C—Atomen; Arylgruppen mit 6 bis 20 C—Atomen oder Polystyrylgruppen mit hieran befestigten Indolizin- oder Indoliziniumgruppen oder Kombinationen hiervon;

$R^8$ eine monovalente Gruppe, die mit dem Indoliziniumkern eine organisches Chromophor vervollständigt;

$R^9$ ein Wasserstoffatom; eine Alkylgruppe mit 1 bis 18 C—Atomen; eine Cyanogruppe; eine Acylgruppe mit 2 bis 20 C—Atomen; eine Carboalkoxygruppe mit 2 bis 18 C—Atomen; eine Aminocarbonylgruppe mit 1 bis 18 C—Atomen; eine Acyloxygruppe mit 2 bis 18 C—Atomen oder ein Brom- oder Chloratom und

$R^{10}$ ein Wasserstoff-, Chlor- oder Bromatom oder eine Alkylgruppe mit 1 bis 18 C—Atomen.

6. Frbstoffverbindung einer der folgenden Formeln:

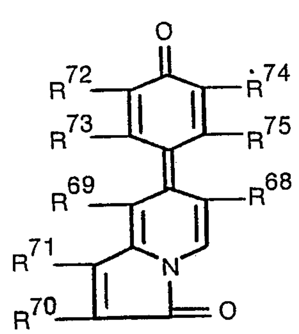

oder

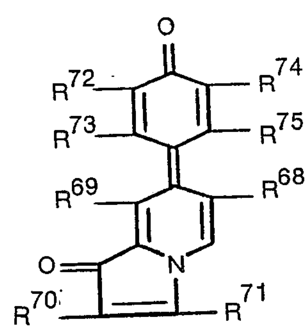

worin bedeuten:

$R^{68}$ ein Wasserstoffatom; eine Alkylgruppe mit 1 bis 18 C—Atomen; eine Cyanogruppe; eine Acylgruppe mit 2 bis 20 C—Atomen; eine Carboalkoxygruppe mit 2 bis 18 C—Atomen; eine Aminocarbonylgruppe mit 1 bis 18 C—Atomen; eine Acyloxygruppe mit 2 bis 18 C—Atomen; ein Brom- oder ein Chloratom;

$R^{69}$ ein Wasserstoff-, Chlor- oder Bromatom oder eine Alkylgruppe mit 1 bis 18 C—Atomen;

$R^{70}$ und $R^{71}$ einzeln Alkylgruppen mit 1 bis 18, verzugsweise 1 bis 19 C—Atomen oder Arylgruppen mit 6 bis 20 C—Atomen;

$R^{72}$ und $R^{73}$ einzeln Wasserstoffatome; Alkylgruppen mit 1 bis 22 C—Atomen; Arylgruppen mit 6 bis 20 C—Atomen; Amino-, Carboxamido-, Sulfonamido-, Sulfamyl- oder Carbamylgruppen, Halogenatome; Alkoxygruppen mit 1 bis 18 C—Atomen oder $R^{72}$ und $R^{73}$ gemeinsam die zur Vervollständigung einer Benzogruppe erforderlichen Atome und

$R^{74}$ und $R^{75}$ einzeln Wasserstoffatome; Hydroxygruppen; Alkylgruppen mit 1 bis 22 C—Atomen; Arylgruppen mit 6 bis 20 C—Atomen; Amino-, Carboxamido-, Sulfonamido-, Sulfamyl- oder Carbamylgruppen; Halogenatome oder Alkoxygruppen mit 1 bis 18 C—Atomen.

**Revendications**

1. Procédé de préparation d'un colorant d'oxoindolizine ou d'oxoindolizinium qui consiste à faire réagir (A) une pyridine comprenant des atomes d'hydrogène sur l'atome de carbone situé en ortho relativement à l'atome d'azote hétérocyclique avec (B) une cyclopropénone disubstituée et, si les substituants de la pyridine ne sont pas tels que le produit de la réaction soit le colorant désiré, à faire réagir le dit produit de réaction avec un dérivé chromogène afin d'obtenir le colorant désiré.

2. Procédé conforme à la revendication 1, caractérisé en ce que le dit colorant présence la formule:

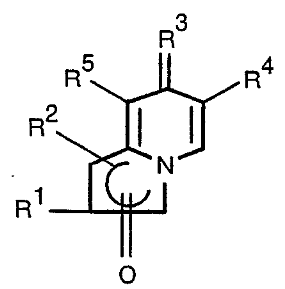

dans laquelle:

$R^1$ et $R^2$ sont chacun alkyle contenant de 1 à 18 atomes de carbone; aryle contenant de 6 à 20 atomes de carbone; ou polystyryle auquel sont attachés des groupes latéraux indolizine ou indolizinium ou des associations de tels groupes;

$R^3$ est un groupe divalent qui, avec le noyau indolizinone, complète un chromophore organique;

$R^4$ est un atome d'hydrogène; alkyle contenant de 1 à 18 atomes de carbone; cyano; acyle contenant de 2 à 20 atomes de carbone, carbalkoxy contenant de 2 à 18 atomes de carbone; aminocarbonyle contenant de 2 à 18 atomes de carbone; acyloxy contenant de 2 à 18 atomes de carbone; un atome de brome ou de chlore; et

$R^5$ est un atome d'hydrogène, de chlore ou de brome ou alkyle contenant de 1 à 18 atomes de carbone; et le dit dérivé de pyridine présente la formule:

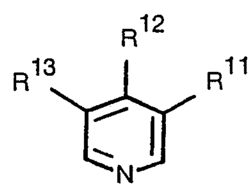

et le dit dérivé de cyclopropénone est représenté par la formule:

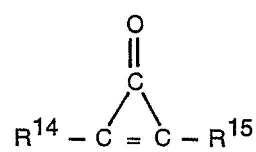

(IV)

dans laquelle

$R^{11}$ est un atome d'hydrogène; alkyle contenant de 1 à 18 atomes de carbone; cyano; acyle contenant de 2 à 20 atomes de carbone; carbalkoxy contenant de 2 à 18 atomes de carbone; aminocarbonyle contenant de 1 à 18 atomes de carbone; acyloxy contenant de 2 à 18 atomes de carbone; un atome de brome ou de chlore.

$R^{12}$ est un atome d'hydrogène; alkyle contenant de 1 à 18 atomes de carbone; cyano; acyle contenant de 2 à 20 atomes de carbone; benzyle; ou pyridyle;

$R^{13}$ est un atome d'hydrogène; de chlore; de brome ou alkyle contenant de 1 à 18 atomes de carbone; et

$R^{14}$ et $R^{15}$ représentent chacun aryle contenant de 6 à 20 atomes de carbone; aralkényle contenant de 6 à 20 atomes de carbone; alkyle contenant de 1 à 18 atomes de carbone; ou bien $R^{14}$ et $R^{15}$ représentent ensemble les atomes de carbone nécessaires pour compléter une structure cyclique à 7 ou 8 atomes.

3. Procédé conforme à la revendication 1, caractérisé en ce que le dit colorant présente la formule:

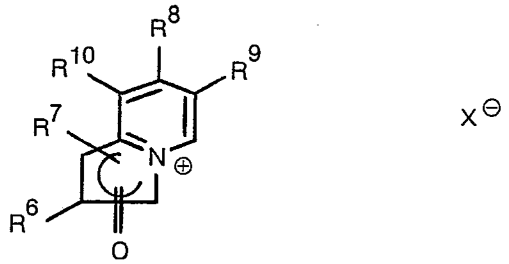

dans laquelle

$X^-$ est un anion;

$R^6$ et $R^7$ représentent chacun alkyle contenant de 1 à 18 atomes de carbone; aryle contenant de 6 à 20 atomes de carbone; ou polystyryle auquel sont attachés des groupes latéraux indolizine ou indolizinium ou des associations de tels groupes;

$R^8$ est un groupe monovalent qui, avec le noyau indolizinium, complète un chromophore organique;

$R^9$ est un atome d'hydrogène; alkyle contenant de 1 à 18 atomes de carbone; cyano; acyle contenant de 2 à 20 atomes de carbone; carbalkoxy contenant de 2 à 18 atomes de carbone; aminocarbonyle contenant de 1 à 18 atomes de carbone; acyloxy contenant de 2 à 18 atomes de carbone; un atome de brome; ou de chlore; et

$R^{10}$ est un atome d'hydrogène; de chlore; ou de brome ou alkyle contenant de 1 à 18 atomes de carbone; et le dit dérivé de pyridine présente la formule:

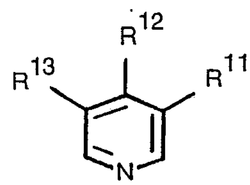

et le dit dérivé de cyclopropénone est représenté par la formule

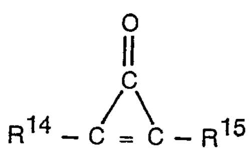

(IV)

dans laquelle

$R^{11}$ est un atome d'hydrogène; alkyle contenant de 1 à 18 atomes de carbone; cyano; acyle contenant de 2 à 20 atomes de carbone; carbalkoxy contenant de 2 à 18 atomes de carbone; aminocarbonyle contenant de 1 à 18 atomes de carbone; acyloxy contenant de 2 à 18 atomes de carbone; un atome de brome; ou de chlore;

$R^{12}$ est un atome d'hydrogène; alkyle contenant de 1 à 18 atomes de carbone; cyano; acyle contenant de 2 à 20 atomes de carbone; benzyle ou pyridyle;

$R^{13}$ est un atome d'hydrogène; de chlore; ou de brome ou alkyle contenant de 1 à 18 atomes de carbone; et

$R^{14}$ et $R^{15}$ représentent chacun aryle contenant de 6 à 20 atomes de carbone; aralkényle contenant de 6 à 20 atomes de carbone; alkyle contenant de 1 à 18 atomes de carbone; ou bien $R^{14}$ et $R^{15}$ représentent ensemble les atomes de carbone nécessaires pour compléter une structure cyclique à 7 ou 8 atomes, l'anion $X^{\ominus}$ étant fourni par un oxydant ou un dérivé chromogène (si on en utilise) ou autrement.

4. Colorant présent la formule:

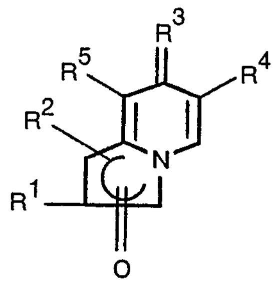

dans laquelle

$R^1$ et $R^2$ sont chacun alkyle contenant de 1 à 18 atomes de carbone; aryle contenant de 6 à 20 atomes de carbone ou polystyryle auquel sont attachés des groupes latéraux indolizine ou indolizinium ou des associations de tels groupes;

$R^3$ est un groupe divalent qui, avec le noyau indolizinone, complète un chromophore organique;

$R^4$ est un atome d'hydrogène, alkyle contenant de 1 à 18 atomes de carbone; cyano; acyle contenant de 2 à 20 atomes de carbone; carbalkoxy contenant de 2 à 18 atomes de carbone; aminocarbonyle contenant de 1 à 18 atomes de carbone; acyloxy contenant de 2 à 18 atomes de carbone; un atome de brome; ou de chlore; et

$R^5$ est un atome d'hydrogène, de chlore; de brome ou alkyle contenant de 1 à 18 atomes de carbone.

5. Colorant présentant la formule:

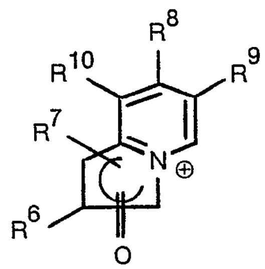

dans laquelle

$X^{\ominus}$ est un anion;

$R^6$ et $R^7$ représentent chacun alkyle contenant de 1 à 18 atomes de carbone; aryle contenant de 6 à 20 atomes de carbone; ou polystyryle auquel sont attachés des groupes latéraux indolizine ou indolizinium ou des associations de tels groupes;

$R^8$ est un groupe monovalent qui, avec le noyau indolizinium, complète un chromophore organique;

$R^9$ est un atome d'hydrogène; alkyle contenant de 1 à 18 atomes de carbone; cyano; acyle contenant de 2 à 20 atomes de carbone; carbalkoxy contenant de 2 à 18 atomes de carbone; aminocarbonyle contenant de 1 à 18 atomes de carbone; acyloxy contenant de 2 à 18 atomes de carbone; un atome de brome; ou de chlore; et

$R^{10}$ est un atome d'hydrogène, de chlore ou de brome ou un groupe alkyle contenant de 1 à 18 atomes de carbone.

6. Colorant présentant les formules:

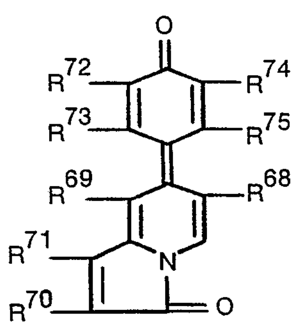

ou

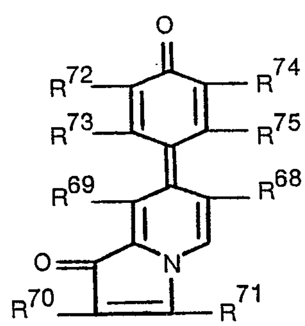

dans lesquelles

$R^{68}$ est un atome d'hydrogène; alkyle contenant de 1 à 18 atomes de carbone; cyano; acyle contenant de 2 à 20 atomes de carbone; carbalkoxy contenant de 2 à 18 atomes de carbone; aminocarbonyle contenant de 1 à 18 atomes de carbone; acyloxy contenant de 2 à 18 atomes de carbone; un atome de brome ou de chlore;

$R^{69}$ est un atome d'hydrogène, de chlore ou de brome ou alkyle contenant de 1 à 18 atomes de carbone;

$R^{70}$ et $R^{71}$ représentent chacun alkyle contenant de 1 à 18, de préférence de 1 à 10 atomes de carbone; ou aryle contenant de 6 à 20 atomes de carbone;

$R^{72}$ et $R^{73}$ représentent chacun un atome d'hydrogène; alkyle contenant de 1 à 22 atomes de carbone, aryle contenant de 6 à 20 atomes de carbone; amino, carboxamido, sulfonamido, sulfamyle ou carbamyle; un atome d'halogène; un groupe alkoxy contenant de 1 à 18 atomes de carbone; ou bien $R^{72}$ et $R^{73}$ représentent ensemble les atomes de carbone nécessaires pour compléter un groupe benzo; et

$R^{74}$ et $R^{75}$ représentent chacun un atomes d'hydrogène; un groupe hydroxy; alkyle contenant de 1 à 22 atomes de carbone; aryle contenant de 6 à 20 atomes de carbone; amino, carboxamido, sulfonamido, sulfamyle ou carbamyle; un atome d'halogène ou alkoxy contenant de 1 à 18 atomes de carbone.